# EUROPEAN PATENT APPLICATION

(11) **EP 2 397 544 A1**
(43) Date of publication of application: **21.12.2011**
(21) Application number: 10741220.7
(22) Date of filing: 09.02.2010
(51) Int. Cl.: C12N 15/09, C07K 1/04, C07K 1/113, C07K 16/00

(54) **IgG-Fc FRAGMENT AND PROCESS FOR PRODUCING SAME**

(30) Priority: 10.02.2009 JP 2009029180
(71) Applicant: Otsuka Chemical Co., Ltd., Osaka-shi, Osaka 540-0021 (JP)
(72) Inventor: KAJIHARA, Yasuhiro, Toyonaka-shi Osaka 560-0054 (JP); FUKAE, Kazuhiro, Tokushima-shi Tokushima 771-0193 (JP)
(74) Representative: Wasner, Marita
(86) International application number: PCT/JP2010/051858
(87) International publication number: WO 2010/092943

(57) **Abstract**

Disclosed is a full-length IgG-Fc fragment having a substantially homogeneous sugar chain added thereto. Also disclosed is a process for producing the full-length IgG-Fc fragment. Specifically disclosed is an IgG-Fc fragment having a sugar chain added thereto, in which the sugar chain is added to the same position as that in a naturally occurring IgG-Fc fragment, any one amino acid residue selected from 1^{st} to 30^{th} amino acid residues from the sugar chain-added amino acid residue on the N-terminal side of the sugar chain-added amino acid residue is substituted by a Cys residue and at least one Met residue is substituted by an amino acid residue other than a Met residue. Also specifically disclosed is a process for producing the IgG-Fc fragment.

## Description

### Field of the Invention

The present invention relates to a novel human IgG-Fc fragment and a process for producing the same.

### Background of the Invention

In recent years, various antibody medicines that specifically bind to different target molecules have been developed. Antibodies are proteins having a Y-shaped basic structure. The top half of the Y-shape is the Fab region that binds to an antigen, composed of 2 light chains and 2 heavy chains. The bottom half of the Y-shape is referred to as the Fc region.

A portion close to the tip of the Fab region is a variable region that differs depending on the antibody. The variable region of the heavy chain is referred to as the VH region, and the variable region of the light chain is referred to as the VL region. Fab and Fc regions outside of the variable region are regions with relatively small alteration, generally referred to as the constant region. The constant region of the light chain is referred to as the CL region and the constant region of the heavy chain is referred to as the CH region. A portion within CH corresponding to the Fab region is referred to as the CH1 domain, and portions corresponding to the Fc region are referred to as CH2 and CH3 domains in this order from the N-terminal side. CH1 and CH2 domains are bound by a hinge region, and the heavy chains on either side are connected by a disulfide bond at the hinge region.

An antibody against a particular antigen can be obtained by e.g. immunizing a mouse with such antigen. However, antibodies produced by mice have high immunogenicity in human, and administration to human beings is limited. In addition, the half-life of mouse antibody is relatively short in a human body, and it is likely that sufficient effect is not obtained.

To solve this problem, chimeric antibodies in which the constant region of a mouse antibody is replaced with the constant region of a human antibody have been developed. A chimeric antibody is expected to be capable of binding to an antigen with the same specificity as a mouse antibody, as well as have low immunogenicity. As a result, human constant regions for use in chimeric antibodies or processes for producing Fc regions have been researched, and in particular, among the immunoglobulins classified into 5 types of IgG, IgA, IgM, IgD, and IgE, research on IgG, which is the antibody that account for 70-75% of human immunoglobulins and present at the greatest amount in plasma, has been promoted. Most of the currently commercially available antibody medicines are also IgG. IgG shows antigen destruction activity called antibody-dependent cellular cytotoxicity (ADCC), and in particular, subclasses IgG1 and IgG3 are known to have strong ADCC activity.

The Fc fragment of IgG is a homodimer consisting of a covalent bond at the hinge region and a non-covalent bond between the CH3 domains, and an N-type sugar chain is bound to Asn at residue 297 of the CH2 domain (position 69 in SEQ ID NO: 1). According to X-ray crystallography, this sugar chain is inwardly oriented and forms hydrogen bonds at multiple sites with the protein surface of the CH2 domain. This sugar chain is suggested to be essential for structural formation and maintenance of Fc fragment (see, e.g. Sondermann, P. et al. Nature Vol. 406, 2000, pp. 267-).

In the process of IgG destroying the antigen by ADCC, the variable region of IgG first binds to a particular protein present on a target cell. Next, the Fc fragment binds to an Fc receptor on effector cells such as an NK cell, which causes a protein called perforin to be released from the effector cell. The perforin forms holes in target cells, which in turn causes the target cell captured by IgG to be destroyed.

It has been found that the structure of the sugar chain bound to IgG1 is involved in the expression of ADCC activity, and there have been many researches until now regarding Fc fragments having a sugar chain added thereto. For example, it has been reported that by introducing the glycosyltransferase N-acetylglucosamine transferase (GNTIII) gene into a CHO cell, an anti-neuroblastoma antibody having a biantennary sugar chain with GlcNAc as the non-reducing terminal was prepared from this CHO cell. It was shown by this research that ADCC activity on neuroblastoma is enhanced by sugar chain addition (see, e.g. Umana, P. et al. Nature Biotechnology Vol. 17, 1999, pp. 176-).

In addition, crystallography of a complex between the IgG1-Fc fragment and FcγRIII suggests that in Fc fragment and Fc receptor binding, N-linked sugar chain bound to Asn297 of the Fc fragment interacts with the Fc fragment protein to stabilize its structure (see, e.g. Sondermann, P. et al. Nature Vol. 406, 2000, pp. 267-). An Fc fragment having a sugar chain bound thereto is also shown to be thermodynamically as well as biologically stable compared to an Fc fragment not having a sugar chain bound thereto (see, e.g. Mimura, Y. et al. Molecular Immunology 37(2000) 697-706).

It is also known that the structure of the sugar chain may affect antibody activity. For example, the expression of human IgG1 in which a fucose bound to position 6 of GlcNAc on the reducing terminal of the sugar chain is removed has been reported, and ADCC activity via FcγRIII binding of the antibody was shown to be enhanced by 40-50 folds (see, e.g. Shields, R.L. et al. The Journal of Biological Chemistry Vol. 277, 2002, pp. 26733-26740). Meanwhile, it has also been confirmed that when a sugar chain bound to Asn297 is sialylated, the cellular cytotoxicity of IgG in vivo decreases (see, e.g. Kaneko, Y. et al. Science Vol. 313 (2006) 670-), and the anti-inflammatory activity of IgG in animal models increases (see, e.g. Anthony, R.M. Science Vol. 320 (2008) 373-) etc.

An example of expressing the IgG1-Fc fragment in yeast, then modifying the sugar chain structure by in vitro enzymatic reaction, and synthesizing an IgG1-Fc fragment having a sugar chain derivative has also been reported (see, e.g. Wei et al. Biochemistry Vol.47 (2008) 10294-10304). However, in this process, since the sugar chain attaches to Asn in the consensus sequence of the N-linked sugar chain (Asn-X-Thr or Asn-X-Ser; wherein X is an amino acid other than proline), when the sugar chain is to be added to a position different from that in a naturally occurring IgG₁-Fc fragment, the corresponding consensus sequence must be introduced when expressing the IgG1-Fc peptide.

As described, IgGs having various sugar chains added thereto have been generated until now and their functions investigated. However, since they utilize cell cultures such as CHO cells or human 293T cells and yeasts for expression, the structure of the sugar chains bound to Asn297 are glycoforms, in other words, they have slightly different structures from each other. When antibodies are to be used for pharmaceuticals, the added sugar chain that affects cellular cytotoxicity must be as homogeneous as possible. IgG with homogeneous sugar chain structure is also necessary to investigate the sugar chain structure involved in cellular cytotoxicity.

The present inventors have up to now developed a process for obtaining an amino acid having a homogenous sugar chain added thereto, and by a solid-phase synthesis employing this, found a process for synthesizing sugar chain-added peptides having sugar chains with extremely homogeneous sugar chain structure accurately added at a desired position (see, e.g. International Publication WO 2004/005330). This process is not suitable for synthesizing long peptides, but by combining this process with an expression system, i.e., by dividing the peptide, and then synthesizing each with solid-phase synthesis or expressing each with an expression system, followed by linking them with a ligation process, longer peptides having a sugar chain added thereto can be obtained. In particular, for peptides not having a sugar chain added thereto, a homogeneous peptide can be economically obtained in large quantities by expression with an expression system.

The present inventors utilized this process to synthesize a part of an IgG-Fc fragment having a sugar chain added thereto. In this process, position 293 (Glu is substituted by Cys) through Lys at position 320 of the IgG-Fc fragment (positions 65 to 92 of SEQ ID NO: 1) was synthesized by solid-phase synthesis, and Asn having a sugar chain added thereto was employed for adding Asn at position 297. Meanwhile, Cys at its C-terminal position 321 through Ser at position 444 (positions 93 to 216 of SEQ ID NO: 1) was expressed by an E. coli expression system. However, Cys321-Ser444 peptide expressed with an E. coli expression system could not be stably obtained, and full-length IgG-Fc fragment could not be obtained by further linking the peptides. In order to employ IgG-Fc fragments in chimeric antibodies, the peptide portion of the IgG-Fc fragment having a homogenous sugar chain added thereto needs to be made longer to be extended to a length that is at least the minimum for allowing binding with the Fc receptor.

### Related art

Patent Document 1
   International Publication WO 2004/005330

Non-Patent Document 1
   Sondermann, P. et al. Nature Vol. 406, 2000, pp. 267-Non-Patent Document 2
   Umana, P. et al. Nature Biotechnology Vol. 17, 1999, pp. 176-
Non-Patent Document 3
   Mimura, Y. et al. Molecular Immunology 37(2000) 697-706 Non-Patent Document 4
   Shields, R.L. et al. The Journal of Biological Chemistry Vol. 277, 2002, pp. 26733-26740
Non-Patent Document 5
   Kaneko, Y. et al. Science Vol. 313 (2006) 670-Non-Patent Document 6
   Anthony, R.M. Science Vol. 320 (2008) 373-Non-Patent Document 7
   Wei et al. Biochemistry Vol.47 (2008) 10294-10304

### Summary of the Invention

### Problems to be Solved by the Invention

Accordingly, the objective of the present invention is to provide a full-length IgG-Fc fragment having a substantially homogeneous sugar chain added thereto, as well as a process for producing the same.

### Means for Solving the Problems

The present inventors have performed repeated research to solve the above problems, and it was found that after the peptide of interest was expressed as a fusion protein with a purification tag using an E. coli expression system, by adding a strong acid instead of the conventionally used formic acid as well as adding particular solvents such as acetonitrile in the step of cleaving the purification tag, the peptide of interest can be very stably produced. Further, it was found that by ligation using the peptide of interest, a peptide having a sugar chain added thereto that is longer than conventional one is obtained, thereby completing the present invention.
Accordingly, the present invention relates to:
<1> an IgG-Fc fragment having a sugar chain added thereto, in which the sugar chain is added at the same position as that in a naturally occurring IgG-Fc fragment, and among the amino acids at positions 1-30 from the sugar chain-added amino acid on the N-terminal side of the sugar chain-added amino acid: (i) any one is substituted by Cys; (ii) any one amino acid that is not Ser in the naturally occurring form is substituted by Ser; (iii) any one amino acid that is not Thr in the naturally occurring form is substituted by Thr; or (iv) any one amino acid that is not Ala in the naturally occurring form is substituted by Ala, and at least one Met is substituted by an amino acid other than Met;
<2> the IgG-Fc fragment according to above <1>, wherein said IgG-Fc fragment is an IgG1-Fc fragment in which a sugar chain is added to Asn at position 69 of the amino acid sequence shown in SEQ ID NO: 1, and among the amino acids at positions 59-68: (i) any one is substituted by Cys; (ii) any one is substituted by Ser; (iii) any one amino acid that is not Thr in the naturally occurring form is substituted by Thr; or (iv) any one amino acid that is not Ala in the naturally occurring form is substituted by Ala;
<3> the IgG-Fc fragment according to above <2>, wherein Glu at position 65 of the amino acid sequence shown in SEQ ID NO: 1 is substituted by Cys;
<4> the IgG-Fc fragment according to above <2> or <3>, wherein Met at positions 130 and 200 of the amino acid sequence shown in SEQ ID NO: 1 are substituted by Leu;
<5> the IgG-Fc fragment according to any one of above <1> to <4>, wherein said sugar chain is a sugar chain represented by: 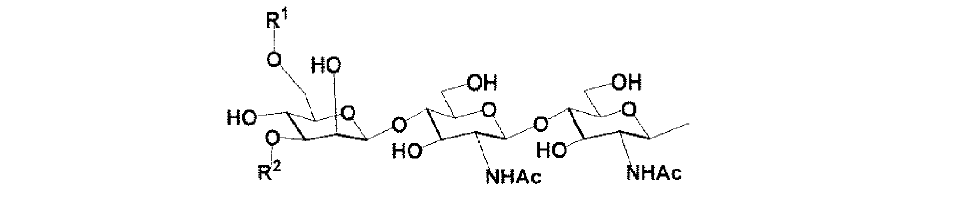 [wherein R¹ and R², identical or different, are 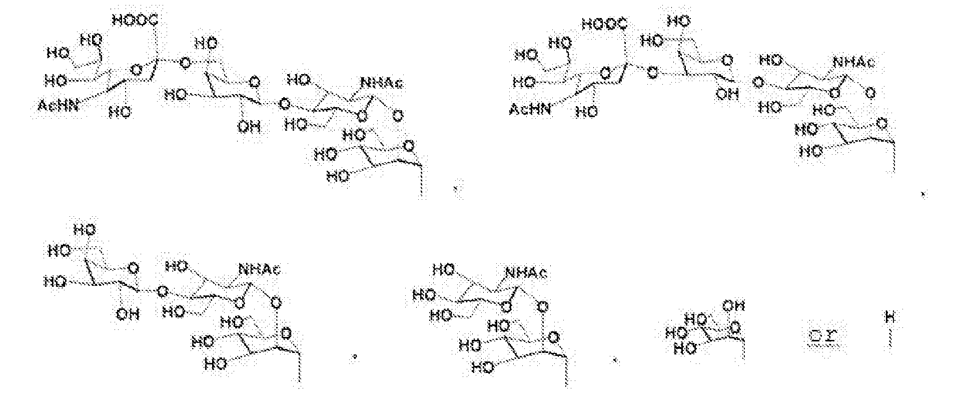 and Ac represents an acetyl group];
<6> a process for producing an IgG-Fc fragment having a substantially homogeneous sugar chain added thereto, comprising a step of synthesizing by solid-phase synthesis using Asn having a substantially homogenous sugar chain added thereto, a partial peptide of said IgG-Fc fragment which is a peptide having a sugar chain added thereto comprising a sugar chain-added amino acid having 3 to 50 amino acid residues; a step of expressing at least one of a partial peptide on the N-terminal side of said peptide having a sugar chain added thereto within said IgG-Fc fragment and a partial peptide on the C-terminal side of said peptide having a sugar chain added thereto within said IgG-Fc fragment by an expression system, and synthesizing the remainder by solid-phase synthesis; and a step of linking said peptide having a sugar chain added thereto, said N-terminal partial peptide, and said C-terminal partial peptide by ligation; wherein the step of expressing said partial peptide by an expression system comprises: a step of expressing said partial peptide as a fusion protein with a purification tag via Met, and purifying the fusion protein by utilizing the purification tag; and a step of cleaving said partial peptide and said purification tag by degrading Met in the presence of a strong acid and a water-miscible solvent;
<7> the process according to above <6>, wherein said strong acid is selected from the group consisting of trifluoroacetic acid, hydrogen fluoride and methanesulfonic acid;
<8> the process according to above <6> or <7>, wherein the N-terminal amino acid of said peptide having a sugar chain added thereto is substituted with Cys or a threonine derivative shown in the following formula (1) for synthesis; 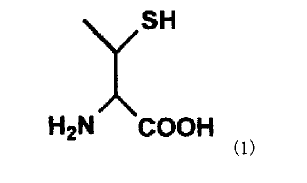
<9> the process according to any one of above <6> to <8>, wherein the step of expressing said partial peptide by an expression system is such that the Met contained in the partial peptide is substituted with an amino acid other than Met;
<10> the process according to any one of above <6> to <9>, wherein said IgG-Fc fragment is an IgG1-Fc fragment, and wherein when said N-terminal partial peptide is synthesized by solid-phase synthesis, the step of synthesizing the N-terminal partial peptide comprises: a step of synthesizing each peptide of said N-terminal partial peptide divided between Thr at position 32 and Cys at position 33 of the amino acid sequence shown in SEQ ID NO: 1 by solid-phase synthesis; and a step of linking the synthesized peptides by ligation;
<11> the process according to above <10>, wherein when said C-terminal partial peptide is synthesized by solid-phase synthesis, the step of synthesizing the C-terminal partial peptide comprises: a step of synthesizing each peptide of said C-terminal partial peptide divided between Thr at position 138 and Cys at position 139 and/or between Ser at position 196 and Cys at position 197 of the amino acid sequence shown in SEQ ID NO: 1 by solid-phase synthesis; and a step of linking the synthesized peptides by ligation;
<12> the process according to any one of above <10> or <11>, wherein said peptide having a sugar chain added thereto is positions 65-92 of the amino acid sequence shown in SEQ ID NO: 1, said N-terminal partial peptide is positions 1-64, and said C-terminal partial peptide is positions 93-216, wherein said peptide having a sugar chain added thereto is synthesized with solid-phase synthesis by substituting Glu at position 65 with Cys, wherein said N-terminal partial peptide is divided into positions 1-32 and 33-64 and each synthesized with solid-phase synthesis, and
   wherein said C-terminal partial peptide is expressed by an expression system such that Met at positions 130 and 200 are substituted with Leu;
<13> the process according to any one of above <6> to <12>, further comprising a step of folding the IgG-Fc fragment after the linking step by said ligation;
<14> the process according to any one of above <6> to <13>, wherein said expression system is an E. coli expression system;
<15> a chimeric antibody comprising an IgG-Fc fragment according to any one of above <1> to <5>, or an IgG-Fc fragment produced by the process for producing the IgG-Fc fragment according to any one of above <6> to <14>;
<16> a method for designing a process for producing an IgG-Fc fragment having a homogenous sugar chain added thereto, wherein said IgG-Fc fragment is produced by dividing it into a peptide having a sugar chain added thereto comprising a sugar chain-added amino acid and one or more other peptides, wherein said peptide having a sugar chain added thereto has any amino acid at positions 1-30 from the sugar chain-added amino acid on the N-terminal side of said sugar chain-added amino acid as the N-terminus, and has an amino acid that flanks on the N-terminal side of the closest Cys, Ser, Thr or Ala of the C-terminal side of the sugar chain-added amino acid as the C-terminus, wherein the peptide having a sugar chain added thereto is synthesized by solid-phase synthesis employing an amino acid having a homogenous sugar chain added thereto, wherein the other peptides are either expressed by an expression system or synthesized by solid-phase synthesis, and wherein said peptide having a sugar chain added thereto and said peptides are linked by ligation;
<17> the process according to above <16>, wherein in the solid-phase synthesis of said peptide having a sugar chain added thereto, the N-terminal amino acid is substituted with Cys or a Thr derivative shown in the following formula (1) for synthesis; 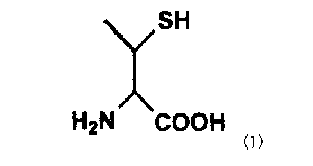
<18> the process according to above <16> or <17>, wherein the other peptides are divided into two or more partial peptides at the N-terminal side of at least one amino acid selected from the group consisting of Cys, Ser, Thr and Ala contained in the peptide, and each of the partial peptides are either expressed by an expression system or synthesized by solid-phase synthesis, and
   wherein said peptide having a sugar chain added thereto and said partial peptides are linked by ligation; and
<19> the process according to any one of above <16> to <18>, wherein when the other peptides are expressed by an expression system, the peptide is expressed as a fusion protein with a purification tag via Met, purified by binding the purification tag to the particular substance, said peptide and said purification tag is cleaved by degrading Met in the presence of a strong acid and a water-miscible solvent, and Met contained in said peptide is altered to an amino acid other than Met for expression.

### Advantages of the Invention

According to the present invention, a full-length IgG-Fc fragment having a homogenous sugar chain added thereto is obtained, and this can be used to perform accurate research of sugar chain structure-dependent activity of an IgG-Fc fragment including cellular cytotoxicity, and in turn antibody activity. In addition, by generating a chimeric antibody using an IgG-Fc fragment having a homogenous sugar chain added thereto, an antibody of homogenous quality with no lot-to-lot difference can be obtained which can also be used as pharmaceuticals.

### Brief Description of the Drawings

Figure 1 is an outline drawing showing the synthetic route for the human IgG1-Fc fragment of the present invention.
Figure 2 shows the amino acid sequence of the human IgG1-Fc fragment and fragments A-D.
Figure 3 is a schematic diagram showing the structure of the plasmid vector pET-16b employed for the expression of fragment D.
Figure 4 is an outline drawing showing a process for producing the expression vector for fragment D.
Figure 5 is a calibration curve showing the relationship between lysozyme concentration and absorbance created using lysozyme to determine fragment D concentration.
Figure 6 shows the result of gel filtration chromatography of the reaction solution of ligation between fragments C and D.
Figure 7 shows the results of analysis by SDS-PAGE after ligation of fragments C and D. Lane 1 shows fragment C, lane 2 shows His tag + fragment D (16 kDa), and lane 3 shows the reaction solution. M is the molecular weight marker.
Figure 8 shows the result of analysis by SDS-PAGE after PNGase F treatment to determine the compound obtained after ligation of fragments C and D. Lane 1 shows the ligation product, lane 2 shows PNGaseF, lane 3 shows fragment C, lane 4 shows the reaction solution, and M shows the molecular weight marker.
Figure 9 shows the result of analysis by SDS-PAGE of the reaction solution after ligation of fragment A + B and fragment C + D. Lane 1 shows fragment A + B, lane 2 shows fragment C + D, lane 3 shows the reaction solution, and M shows the molecular weight marker.

### Description of Embodiments

The IgG-Fc fragment according to the present invention is a peptide comprising the CH2 and CH3 domains of IgG immunoglobulin. IgG comprises any derived from any mammals such as human, monkeys, mice, dogs, cows, and horses, although those derived from human is preferred when used for a chimeric antibody. IgG also has 4 subclasses IgG1 to IgG4. The IgG-Fc fragment of the present invention may be of any subclass, but IgG1 having strong cellular cytotoxicity is preferred.

The IgG1-Fc fragment according to the present invention has the amino acid sequence shown in SEQ ID NO: 1. Further, the IgG1-Fc fragment according to the present invention comprises a peptide having one or several amino acids deleted, substituted, or added from the amino acid sequence shown in SEQ ID NO: 1; a peptide having one or several amino acids conservatively substituted from the amino acid sequence shown in SEQ ID NO: 1; and an IgG-Fc variant, and for example comprises an IgG1-Fc fragment derivative having the amino acid sequence shown in SEQ ID NO: 2, provided that it exerts the effects of the present invention described below.

Accordingly, when referring to position X in SEQ ID NO: 1 of an IgG-Fc fragment herein below, in addition to position X of a naturally occurring IgG-Fc fragment having SEQ ID NO: 1, the amino acid at a position corresponding to position X of SEQ ID NO: 1 in a peptide having one or several amino acids are deleted, substituted, or added from the amino acid sequence shown in SEQ ID NO: 1; a peptide having one or several amino acids conservatively substituted from the amino acid sequence shown in SEQ ID NO: 1; and an IgG-Fc variant is also included.

Moreover, an IgG1-Fc fragment having the amino acid sequence shown in SEQ ID NO: 2 comprises Glu at position 65 substituted by Cys, Gln at position 128 substituted by Asp, Met at position 130 substituted by Leu, and Met at position 200 substituted by Leu, compared to a naturally occurring IgG1-Fc fragment having the amino acid sequence shown in SEQ ID NO: 1.

As used herein, an "amino acid" is used as its most extended meaning, and includes natural amino acids as well as non-natural amino acids such as amino acid variants and derivatives. Those skilled in the art will recognize in consideration of this extended meaning that examples of amino acids herein include natural proteinous L-amino acids; D-amino acids; chemically modified amino acids such as amino acid variants and derivatives; natural non-proteinous amino acids such as norleucine, β-alanine, and ornithine; and chemically synthesized compounds having properties characteristic of amino acids well known in the art. Examples of non-natural amino acids include α-methylamino acids (such as α-methylalanine), D-amino acids, histidine-like amino acids (such as 2-amino-histidine, β-hydroxy-histidine, homohistidine, α-fluoromethyl-histidine and α-methyl-histidine), amino acids having excess methylene on the side chain ("homo" amino acids), and amino acids in which the carboxylic group amino acid in the side chain is substituted with a sulfonate group (such as cysteic acid). In a preferred aspect, the amino acids contained in the compound of the present invention consist only of natural amino acids. Further, amino acids are generally described herein using the three-letter representation.

As used herein, when "one or several amino acids are deleted, substituted, or added from an amino acid," the number of amino acids substituted etc. is not particularly limited, provided that IgG-Fc fragment activity is retained, but is from 1 to 9, preferably from 1 to 5, and more preferably from about 1 to 3, or within 20%, preferably within 10% of the total length. The substituted or added amino acid may be a natural amino acid, a non-natural amino acid or an amino acid analogue, preferably a natural amino acid.

As used herein, "one or several amino acids are conservatively substituted from an amino acid" refers to an amino acid substitution in which hydrophilic and/or hydrophobic index between the original amino acid and the substituted amino acid are similar, and when comparing before and after such substitution, obvious reduction or elimination of IgG-Fc fragment activity does not occur.

As used herein, an "IgG-Fc variant" is a compound in which the IgG-Fc fragment is naturally or artificially modified, and examples of such modification include alkylation, acylation (e.g. acetylation), amidation, carboxylation, ester formation, disulfide bond formation, sugar chain addition, lipidation, phosphorylation, hydroxylation, and binding of labeling component of/to one or more amino acid residues of the IgG-Fc fragment.

The IgG-Fc fragment according to the present invention has a sugar chain added at the same position as that in a naturally occurring IgG-Fc fragment. The same position as that in a naturally occurring IgG-Fc fragment is, for example, in the case of an IgG1-Fc fragment, Asn at position 69 in the amino acid sequence shown in SEQ ID NO: 1.
Moreover, the IgG-Fc fragment according to the present invention comprises those further having one or more sugar chains added at positions other than the position same as that in a naturally occurring IgG-Fc fragment.

As used herein, a "sugar chain" refers to a compound consisting of one or more unit sugars (monosaccharide and/or a derivative thereof) arranged in a row. When two or more unit sugars are arranged in a row, each unit sugar is bound by dehydration condensation by a glycoside bond in between. Examples of such sugar chains include, but are not limited to, monosaccharides and polysaccharides contained in living organisms (glucose, galactose, mannose, fucose, xylose, N-acetylglucosamine, N-acetylgalactosamine, sialic acid and complexes and derivative thereof), as well as a wide range of sugar chains degraded or derivatized from complex biomolecules such as degraded polysaccharides, glycoproteins, proteoglycans, glycosaminoglycans, and glycolipids. Sugar chains may be linear or branched.

In addition, "sugar chain" as used herein includes sugar chain derivatives, and examples of sugar chain derivative include, but are not limited to, a sugar chain in which the sugar constituting the sugar chain is a sugar having a carboxyl group (e.g., aldonic acid in which the C-1 position is oxidized to carboxylic acid (e.g., D-glucose oxidized to D-gluconic acid) and uronic acid in which the terminal C atom has become a carboxylic acid (D-glucose oxidized to D-glucuronic acid)), a sugar having an amino group or amino group derivative (e.g., acetylated amino group) (e.g., N-acetyl-D-glucosamine, N-acetyl-D-galactosamine), a sugar having both amino and carboxyl groups (e.g., N-acetylneuraminic acid (sialic acid) and N-acetylmuramic acid), deoxylated sugar (e.g., 2-deoxy-D-ribose), a sulfated sugar comprising a sulfate group, and a phosphorylated sugar comprising a phosphate group etc.

A preferred sugar chain in the present invention is for example a sugar chain that increases cellular cytotoxicity when attached to an IgG-Fc fragment.

Considering the fact that the IgG-Fc fragment having a sugar chain added thereto of the present invention will be administered to a living organism, the sugar chain in the IgG-Fc fragment having a sugar chain added thereto of the present invention is for example an N-linked or O-linked sugar chain, which is a sugar chain that is present in vivo as a complex carbohydrate (such as glycopeptide (or glycoprotein), proteoglycan, and glycolipid), preferably a sugar chain that is bound to a peptide (or protein) in vivo as a glycopeptide (or glycoprotein).

The sugar chain used in the present invention is an N-linked sugar chain. Examples of N-linked sugar chains can include high-mannose, complex, and hybrid types, and complex type is particularly preferred.

In an aspect of the present invention, the sugar chain in the IgG-Fc fragment having a sugar chain added thereto of the present invention is preferably a sugar chain consisting of 4 or more, e.g., 5 or more, 7 or more, and in particular 9 or more sugars.

In a preferred aspect of the present invention, the sugar chain in the IgG-Fc fragment having a sugar chain added thereto is a sugar chain consisting of from 5 to 11, from 9 to 11, or 9 sugars.

In a preferred aspect of the present invention, the sugar chain in the IgG-Fc fragment having a sugar chain added thereto of the present invention is a sugar chain selected from the group consisting of a disialo sugar chain, a monosialo sugar chain, an asialo sugar chain, a diGlcNAc sugar chain and a dimannose sugar chain, more preferably an asialosugar chain.

Preferred sugar chains used in the present invention include, for example, sugar chains etc. represented by the following general formula: 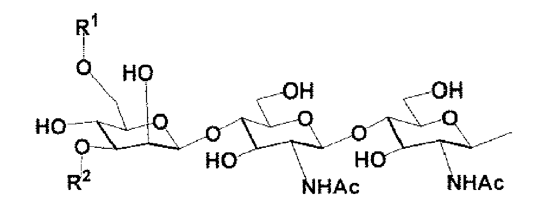 [wherein R¹ and R², identical or different, are 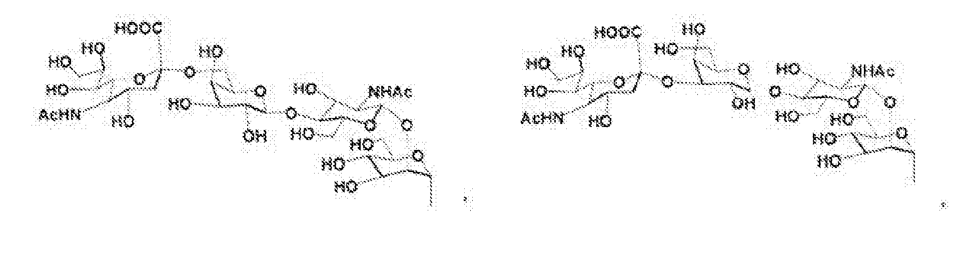 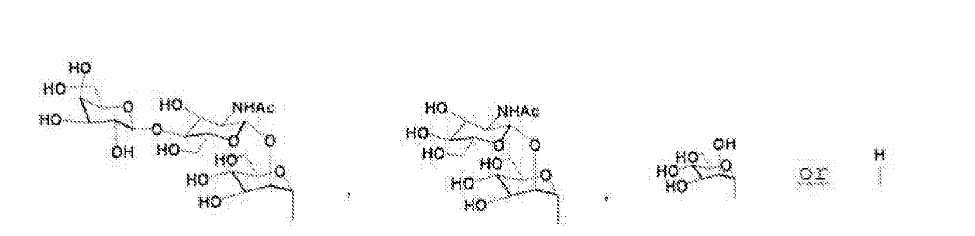 and Ac represents an acetyl group].

The preferred sugar chain in the present invention can include, for example, a sugar chain having the same structure (sugar chains in which the type of component sugar and their binding format are the same) as sugar chains that exist as glycoproteins bound to proteins in a human body (e.g., a sugar chain described in "FEBS LETTERS Vol.50, No. 3, Feb. 1975"), or a sugar chain which have one or more sugars deleted from its non-reducing terminal, examples of which are described in Tables 1-4 below.

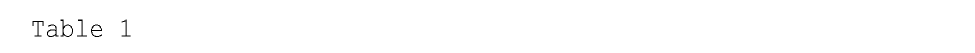

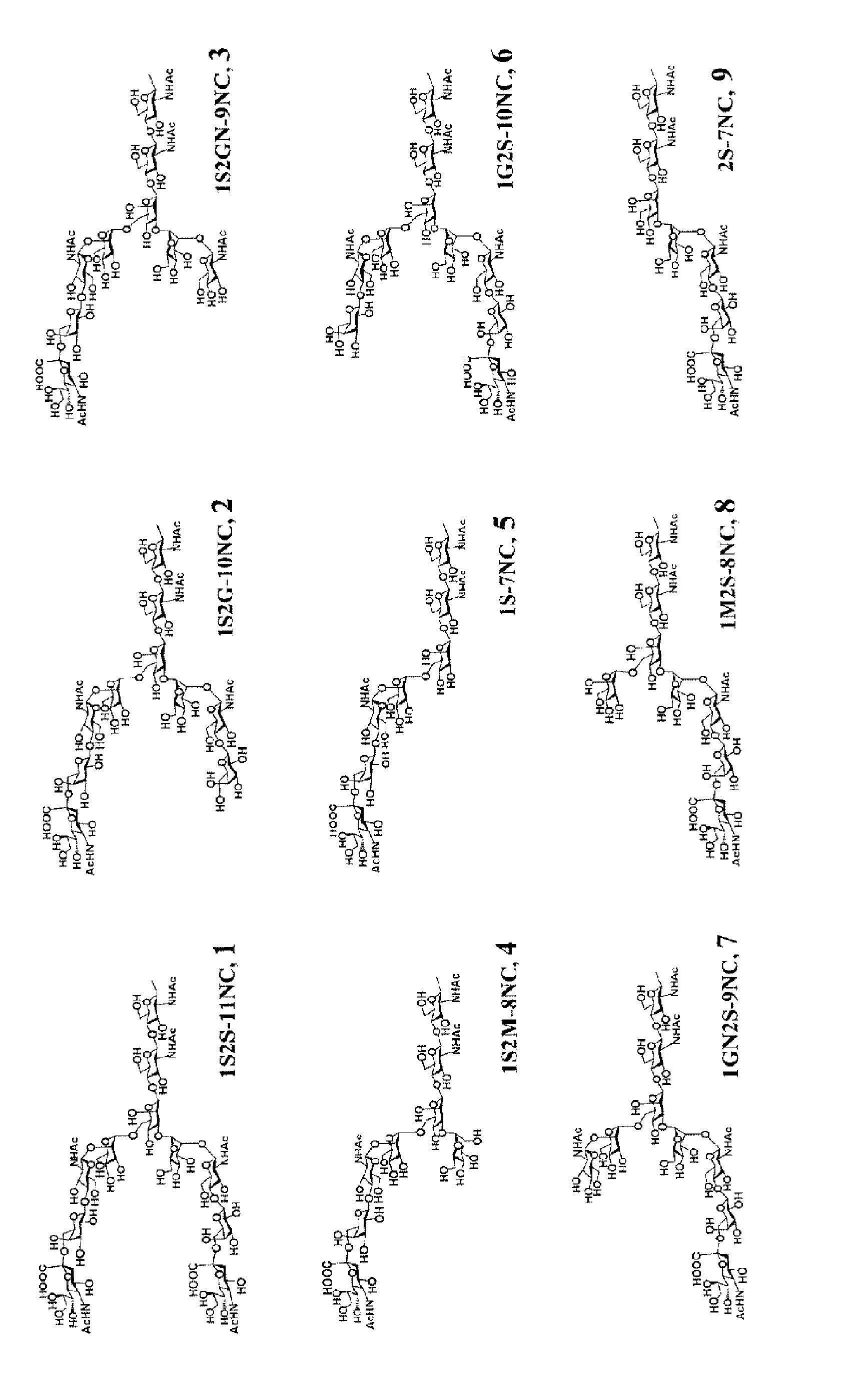

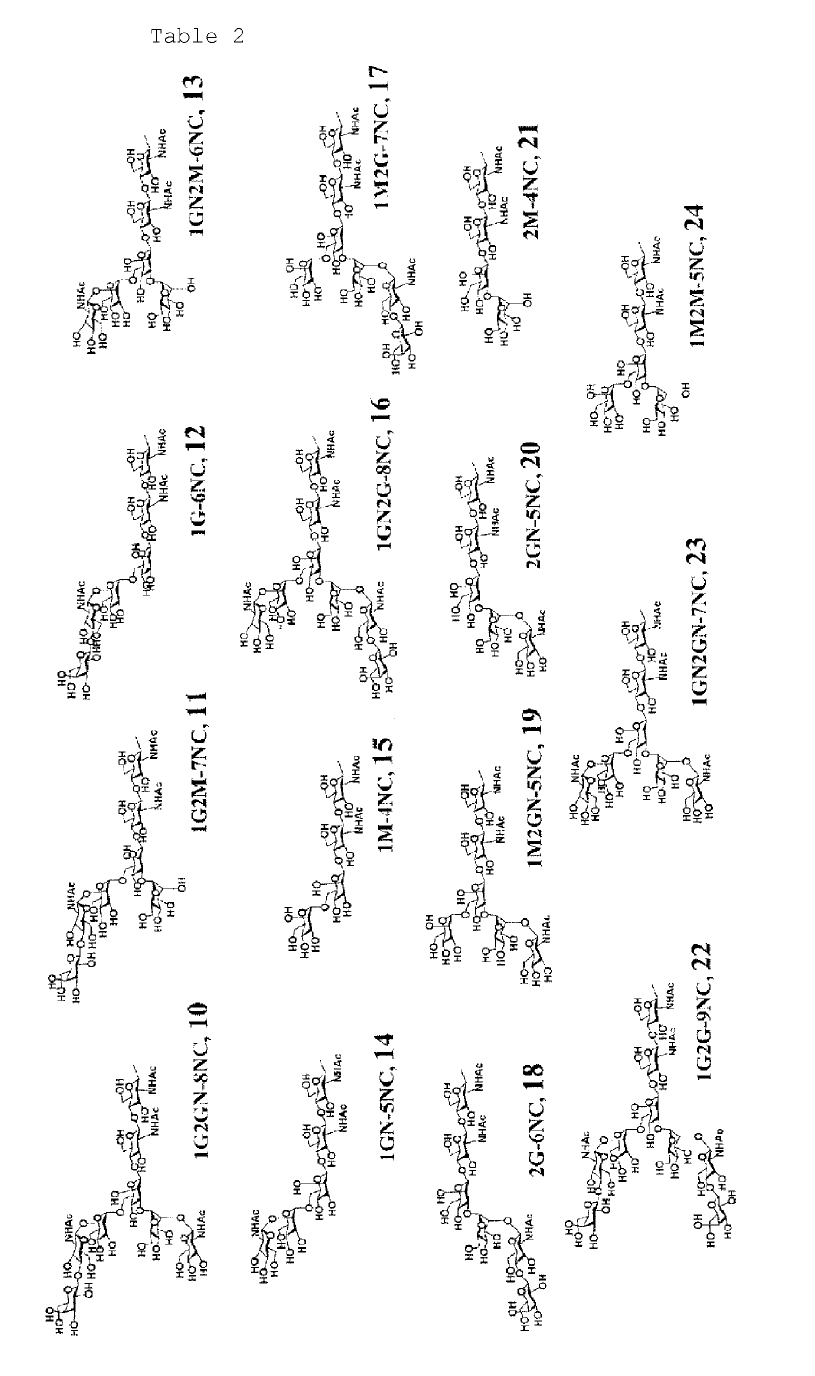

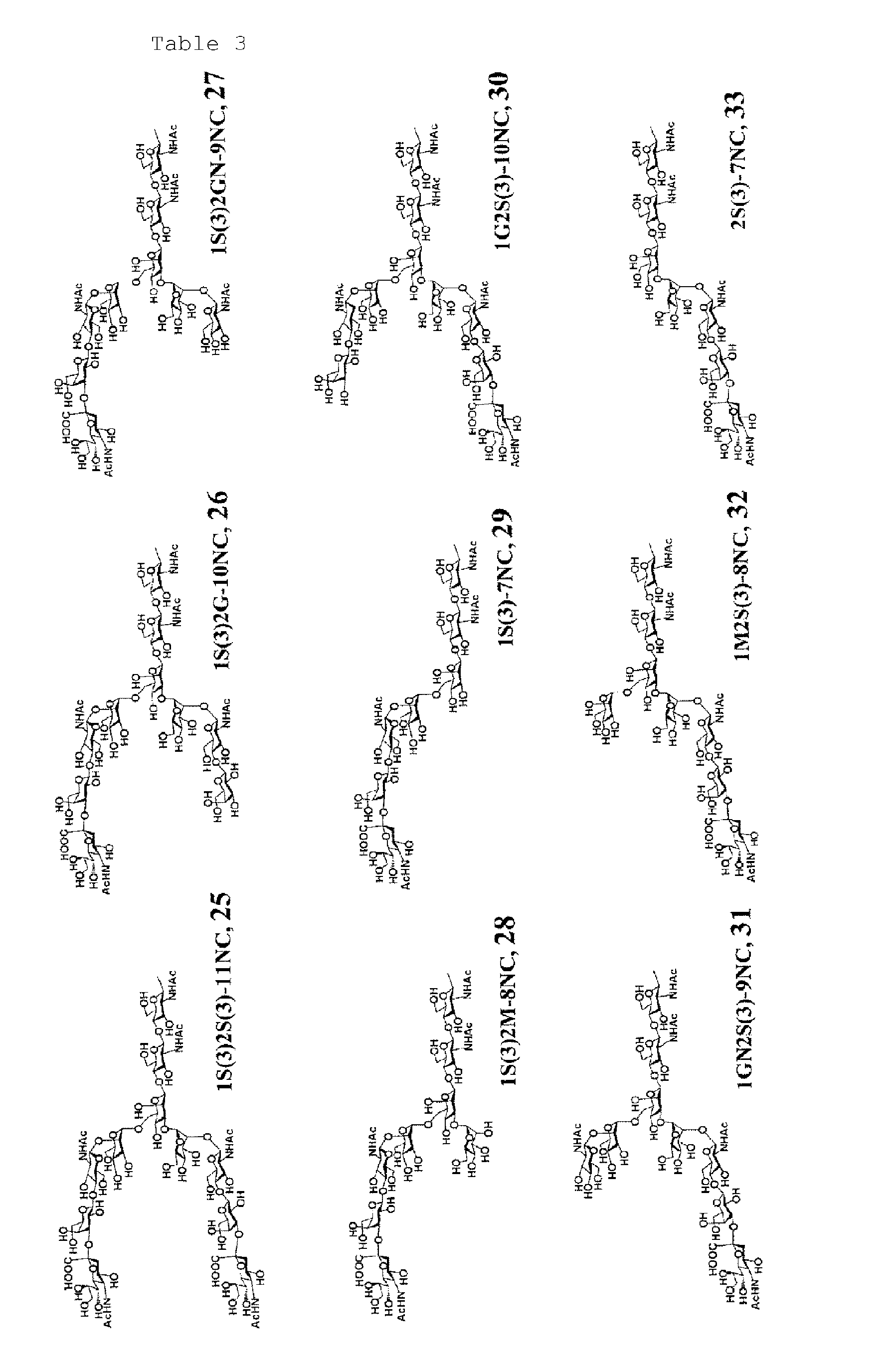

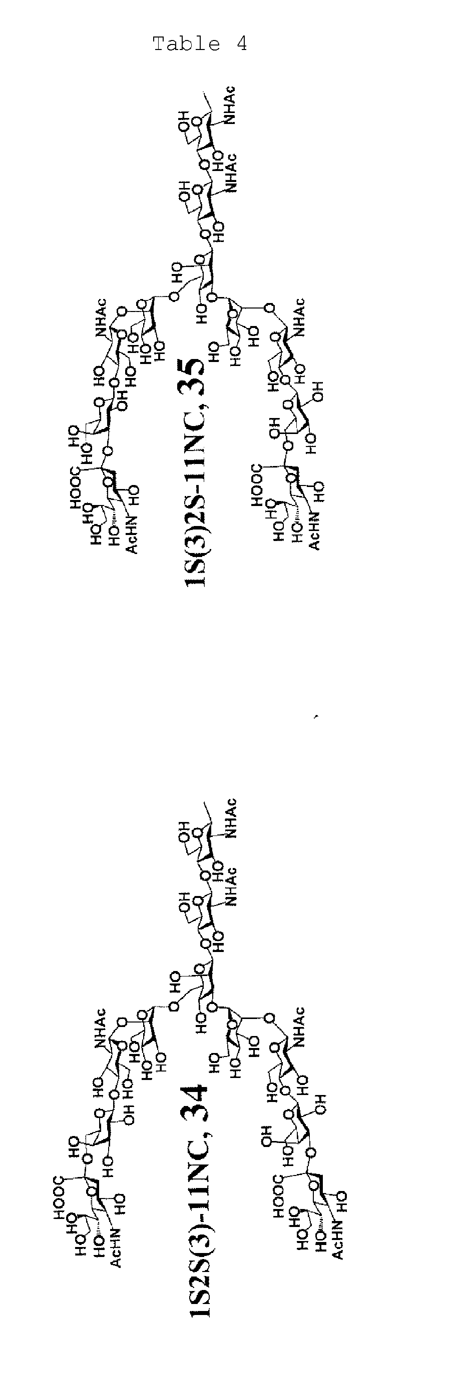

As used herein, a "sugar chain-added amino acid" refers to an amino acid having a sugar chain bound thereto. The sugar chain and the amino acid may be bound via a linker. The binding site of the sugar chain and the amino acid is not particularly limited, but it is preferred that the amino acid is bound to the reducing terminal of the sugar chain.
The type of amino acid the sugar chain binds is not particularly limited, and it is any amino acid of the IgG-Fc fragment described above, preferably Asn or Ser, more preferably Asn.

When the sugar chain and the amino acid bind via a linker, the type of linker is not particularly limited, and examples can include -NH-(CO)-(CH₂)ₐ-CH₂- (wherein a is an integer, and although it is not limited, provided that the linker function of interest is not inhibited, a preferably represents an integer from 0-4), C₁₋₁₀ polymethylene, -CH₂-R-(wherein R is a group produced by one hydrogen atom detached from a group selected from the group consisting of alkyl, substituted alkyl, alkenyl, substituted alkenyl, alkynyl, substituted alkynyl, aryl, substituted aryl, carbocyclic group, substituted carbocyclic group, heterocyclic group, and substituted heterocyclic group).

Moreover, the IgG-Fc fragment having a sugar chain added thereto according to the present invention may be produced by any production process, for example, it may be produced by solid-phase synthesis using a sugar chain-added amino acid as the raw material, or it may be produced by binding a sugar chain having no amino acid bound thereto directly or via a linker to an amino acid on a peptide. A sugar chain having no amino acid bound thereto may be prepared by for example solid-phase synthesis, liquid-phase synthesis, and expression by an expression system. In addition, as long as the final structure match, those produced by extending the sugar chain by further adding a sugar or a sugar chain onto the sugar chain of an IgG-Fc fragment having a sugar chain added thereto, and those produced by a combination process of solid-phase synthesis and expression by an expression system described below etc. are included in the IgG-Fc fragment having a sugar chain added thereto of the present invention.

In a preferred aspect of the present invention, the structure of the sugar chain added to the IgG-Fc fragment is substantially homogenous. As used herein, the structure of the sugar chain is substantially homogenous means that when comparing IgG-Fc fragments having a sugar chain added thereto, the site of sugar chain addition, the type of each sugar constituting the sugar chain sugar chain, the binding order, and the binding format between sugars are identical, and at least 90% or more, preferably 95% or more, and more preferably 99% or more of the structure of the sugar chain is homogenous. A glycopeptide having a homogenous sugar chain is of a constant quality, and particularly preferred for fields such as production of pharmaceuticals or assays. The proportion of the homogeneous sugar chain can be measured, for example, by methods using HPLC, capillary electrophoresis, NMR, and mass spectrometry. The production of a sugar chain having homogeneous sugar chain structure is described, e.g., in International Publication WO 02/04471, WO 03/008431, WO 2004/058984, WO 2004/058824, WO 2004/070046, and WO 2007/011055, the disclosures of which are incorporated herein by reference in their entirety.

The IgG-Fc fragment having a sugar chain added thereto according to the present invention have, among the amino acids in a position that is 1-30 amino acids from the sugar chain-added amino acid on the N-terminal side of the sugar chain-added amino acid: (i) any one substituted by Cys; (ii) any one amino acid that is not Ser in the naturally occurring form substituted by Ser; (iii) any one amino acid that is not Thr in the naturally occurring form substituted by Thr; or (iv) any one amino acid that is not Ala in the naturally occurring form substituted by Ala. Preferably, the position is 1-20 amino acids from the sugar chain-added amino acid, and further preferably 1-10 amino acids. For example, in the case of an IgG1-Fc fragment, preferably, among the amino acids at positions 59-68 in the amino acid sequence shown in SEQ ID NO: 1: (i) any one is substituted by Cys; or (ii) any one is substituted by Ser; or (iii) any one amino acid that is not Thr in the naturally occurring form (amino acid other than at position 61) is substituted by Thr; or (iv) any one amino acid that is not Ala in the naturally occurring form (amino acid other than at position 59) is substituted by Ala, and most preferably, Glu at position 65 is substituted by Cys.
A peptide having Cys at the N-terminus is preferably used in the ligation. As described below, the IgG-Fc fragment having a sugar chain added thereto according to the present invention can be obtained by for example synthesizing a peptide having a sugar chain added thereto comprising a sugar chain-added amino acid by solid-phase synthesis, and then ligating this with other portions of the peptide. When synthesizing a peptide having a sugar chain added thereto, it is preferred that its N-terminus is in the range of 1-30 amino acids from the N-terminal side of the sugar chain-added amino acid. In this way, if the N-terminus of the peptide having a sugar chain added thereto was made to be Cys, a substitution to Cys in the region of 1-30 amino acids from the N-terminal side of the sugar chain-added amino acid will occur after ligation (corresponding to (i)).
As described below, Cys can be exchanged to Ser or Ala after ligation (corresponding to (ii) or (iv)). Meantime, the N-terminus of a peptide having a sugar chain added thereto can also be made to be a threonine derivative (1) described below and still favorably carry out the ligation reaction, and this can be converted to Thr after ligation (corresponding to (iii)).
According to the process for producing the IgG-Fc fragment having a sugar chain added thereto according to the present invention described below, since substitution by Cys, Ser, Ala, or Thr can be inserted into the region of 1-30 amino acids from the N-terminal side of the sugar chain-added amino acid as shown, a variant having Cys, Ser, Ala or Thr at a position different from that in a naturally occurring form can also be synthesized, and the IgG-Fc fragment having a sugar chain added thereto thus obtained is also encompassed in the present invention.

Further, the IgG-Fc fragment having a sugar chain added thereto according to the present invention have at least one Met substituted with an amino acid other than Met. As used herein, an "amino acid other than Met" is not particularly limited, but e.g. a neutral amino acid is preferred, and Leu etc. having a structure relatively similar to Met is preferably employed. For example, in the case of an IgG1-Fc fragment, it is preferred that Met at positions 130 and 200 in the amino acid sequence shown in SEQ ID NO: 1 are substituted by Leu.

In a preferred aspect of the present invention, an IgG-Fc fragment having a sugar chain added thereto has a predetermined conformation. A predetermined conformation means, for example, to have a similar conformation as that in a naturally occurring IgG-Fc fragment. In the case of an IgG1-Fc fragment, disulfide bonds are formed between Cys at position 33 and Cys at position 93, as well as between Cys at position 139 and Cys at position 197 of the amino acid sequence shown in SEQ ID NO: 1. Whether or not the IgG-Fc fragment has a predetermined conformation can be confirmed by for example disulfide mapping, evaluation of binding to an antibody specific to a conformational epitope, and X-ray crystallography.

### (Process for producing an IgG-Fc fragment)

The process for producing an IgG-Fc fragment having a substantially homogeneous sugar chain added thereto according to the present invention will be described next. (Solid-phase synthesis of a peptide having a sugar chain added thereto)
The process for producing an IgG-Fc fragment having a substantially homogeneous sugar chain added thereto according to the present invention comprises, first, a step of synthesizing by solid-phase synthesis using Asn having a substantially homogenous sugar chain added thereto, a partial peptide of the IgG-Fc fragment which is a peptide having a sugar chain added thereto comprising a sugar chain-added amino acid having 3 to 50 amino acid residues.

The solid-phase synthesis can be performed by a well-known method or a method based thereon. An example is described in International Publication WO 2004/005330, the disclosure of which is incorporated herein by reference in its entirety.

Specifically, first, (1) the hydroxyl group of a resin having a hydroxyl group and the carboxyl group of an amino acid in which the amino group nitrogen is protected with a fat-soluble protecting group are subjected to an esterification reaction. In this case, since the amino group nitrogen of the amino acid is protected with a fat-soluble protecting group, self-condensation between amino acids is prevented, resulting in a reaction of the hydroxyl group of the resin and the carboxyl group of the amino acid to cause esterification.
Next, (2) the fat-soluble protecting group of the ester obtained above is detached to form a free amino group;
(3) this free amino group and the carboxyl group of any amino acid in which the amino group nitrogen is protected with a fat-soluble protecting group are subjected to an amidation reaction;
(4) the above fat-soluble protecting group is detached to form a free amino group; and
(5) above steps (3) and (4) are repeated once or more, thereby providing a peptide which is any number of any amino acids linked together, has a resin bound to one terminus, and has a free amino group on the other terminus.
(6) Next, the carboxyl group of the asparagine portion of a sugar chain asparagine (asparagine having a sugar chain added thereto) in which the amino group nitrogen is protected with a fat-soluble protecting group and the above free amino group are subjected to an amidation reaction;
(7) further the above fat-soluble protecting group is detached to form a free amino group;
(8) this free amino group and the carboxyl group of any amino acid in which the amino group nitrogen is protected with a fat-soluble protecting group are subjected to an amidation reaction;
(9) above steps (7) and (8) are repeated once or more; and
(10) the above fat-soluble protecting group is detached to form a free amino group, thereby providing a glycopeptide which is any number of any amino acids linked together, has a resin bound to one terminus, has a free amino group on the other terminus, and has a sugar chain asparagine in the middle.
(11) Finally, the resin is cleaved with an acid to allow the production of a glycopeptide having a sugar chain asparagine at any position on the peptide chain.

Further, by appropriately adding the above step (6) of subjecting the carboxyl group of the asparagine portion of a sugar chain asparagine in which the amino group nitrogen is protected with a fat-soluble protecting group and the above free amino group to an amidation reaction, a glycopeptide having at least two or more sugar chain asparagines at any position on the peptide chain can be produced. In addition, by employing a different sugar chain asparagine, a glycopeptide having two or more sugar chain asparagines at any position on the peptide chain can also be produced.

Moreover, this sugar chain asparagine can also be introduced at the tip of the peptide chain.

A resin having a hydroxyl group may usually be a resin having a hydroxyl group used in solid-phase synthesis, and for example, Amino-PEGA resin (available from Merck & Co., Inc.), Wang resin (available from Merck & Co., Inc.), and HMPA-PEGA resin (available from Merck & Co., Inc.) can be used. Considering the fact that thioesterification is performed after solid-phase synthesis, HMPB-PEGA resin is preferred.

Any amino acid can be used as the amino acid, and examples can include the natural amino acids serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cystein (Cys), methionine (Met), phenylalanine (Phe), tryptophan (Trp), and proline (Pro).

Examples of fat-soluble protecting groups can include carbonate- and amide-based protecting groups such as 9-fluorenylmethoxycarbonyl (Fmoc) group, t-butyloxycarbonyl (Boc) group, a benzyl group, an allyl group, an allyloxycarbonyl group, and an acetyl group. For introducing a fat-soluble protecting group, for example when introducing an Fmoc group, introduction can be carried out by adding 9-fluorenylmethyl-N-succinimidylcarbonate and sodium bicarbonate for reaction. The reaction may be carried out at 0-50°C, preferably at room temperature for about 1-5 hours.

For amino acids protected with a fat-soluble protecting group, the above amino acids can be produced with the above method. Commercially available products may also be used. Examples can include Fmoc-Ser, Fmoc-Asn, Fmoc-Val, Fmoc-Leu, Fmoc-Ile, Fmoc-Ala, Fmoc-Tyr, Fmoc-Gly, Fmoc-Lys, Fmoc-Arg, Fmoc-His, Fmoc-Asp, Fmoc-Glu, Fmoc-Gln, Fmoc-Thr, Fmoc-Cys, Fmoc-Met, Fmoc-Phe, Fmoc-Trp, and Fmoc-Pro.

For example, well-known dehydration condensation agents such as 1-mesitylenesulfonyl-3-nitro-1,2,4-triazole (MSNT), dicyclohexylcarbodiimide (DCC), and diisopropylcarbodiimide (DIPCDI) can be used as esterification catalysts. The proportion for use between the amino acid and the dehydration condensation agent is 1 part by weight of the former to usually 1-10 parts by weight, preferably 2-5 parts by weight of the latter.

It is preferred that the esterification reaction is carried out for example by placing a resin in a solid phase column, washing this resin with a solvent, and then adding the amino acid solution. Examples of washing solvents can include dimethylformamide (DMF), 2-propanol, and methylene chloride. Examples of solvents for dissolving the amino acid can include dimethylsulfoxide (DMSO), DMF, and methylene chloride. The esterification reaction may be carried out at 0-50°C, preferably at room temperature for about 10 minutes to 30 hours, preferably about 15 minutes to 24 hours.

It is also preferred to cap the unreacted hydroxyl groups on the solid phase by acetylation using acetic anhydride etc.

Detachment of the fat-soluble protecting group can be carried out by for example treatment with a base. Examples of bases can include piperidine and morpholine. It is preferred to perform this treatment in the presence of a solvent. Examples of solvents can include DMSO, DMF, and methanol.

It is preferred that the amidation reaction between the free amino group and the carboxyl group of any amino acid in which the amino group nitrogen is protected with a fat-soluble protecting group is carried out in the presence of an activating agent and an solvent.

Examples of activating agents can include dicyclohexylcarbodiimide (DCC), 1-ethyl-3-(3-dimethylaminopropyl)carbodiimide hydrochloride (WSC/HCl), diphenylphosphorylazide (DPPA), carbonyldiimidazole (CDI), diethylcyanophosphonate (DEPC), diisopropylcarbodiimide (DIPCI), benzotriazol-1-yloxy-trispirodinophosphonium hexafluorophosphate (BOP), 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), 2-(1H-benzotriazol-1-yl)-1,1,3,3-tetramethyluronium hexafluorophosphate (HBTU), 0-(7-azabenzotriazol-1-yl)-1,1,3,3-tetramethyluronium hexafluorophosphonate (HATU), O-benzotriazol-1-yl-1,1,3,3-tetramethyluronium tetrafluoroborate (TBTU), and 3,4-dihydro-3-hydrodi-4-oxa-1,2,3-benzotriazine (Dhbt).

It is preferred that the amount of the activating agent used is 1-20 equivalents, preferably 1-10 equivalents, and further preferably 1-5 equivalents to one equivalent of any amino acid in which the amino group nitrogen is protected with a fat-soluble protecting group.

Examples of solvents can include DMSO, DMF, and methylene chloride. The reaction may be carried out at 0-50°C, preferably at room temperature for about 10-30 hours, preferably about 15 minutes to 24 hours. Detachment of the fat-soluble protecting group can be performed as above.

It is preferred that an acid is used to cleave the peptide chain from the resin. Examples of acids can include trifluoroacetic acid (TFA) and hydrogen fluoride (HF).

By appropriately adding above step (6) of subjecting the carboxyl group of the asparagine portion of a sugar chain asparagine in which the amino group nitrogen is protected with a fat-soluble protecting group to an amidation reaction and step (7) of detaching the above fat-soluble protecting group to form a free amino group, a glycopeptide having at least two or more sugar chain asparagines at any position on the peptide chain can be produced.

Further, by performing above step (6) of subjecting the carboxyl group of the asparagine portion of a sugar chain asparagine in which the amino group nitrogen is protected with a fat-soluble protecting group to an amidation reaction and step (7) of detaching the above fat-soluble protecting group to form a free amino group in the final step, a glycopeptide having at least one or more sugar chain asparagines on the peptide chain can be produced.

Moreover, by (1) subjecting the hydroxyl group of a resin having a hydroxyl group and the carboxyl group of the asparagine portion of a sugar chain asparagine in which the amino group nitrogen is protected with a fat-soluble protecting group to an esterification reaction instead of or in addition to above step (6), a glycopeptide having a sugar chain asparagine at the tip can be produced.

In this way, among the IgG-Fc fragments, a peptide having a sugar chain added thereto can be obtained.
Moreover, as described above, Asn having a substantially homogenous sugar chain added thereto used in the above solid-phase synthesis is described, e.g., in International Publication WO 02/04471, WO 03/008431, WO 2004/058984, WO 2004/058824, WO 2004/070046, and WO 2007/011055, the disclosures of which are incorporated herein by reference in their entirety.

It is preferred that the above peptide having a sugar chain added thereto have Cys as its N-terminal amino acid. By having this amino acid as the N-terminus, it can be favorably applied to ligation such as NCL described below. Moreover, the Cys residue can be converted to Ala or Ser after ligation. Accordingly, if the N-terminus of a peptide having a sugar chain added thereto is made to be positions 39 or 59 of the amino acid sequence shown in SEQ ID NO: 1, by converting the Cys residue to Ala or Ser after ligation, it can be made to be the same amino acid sequence as the naturally occurring form. A method for converting the Cys residue to Ala or Ser will be described below.

In addition, the N-terminal amino acid of the peptide having a sugar chain added thereto may be a threonine derivative shown by the following formula (1) (hereinafter referred to as "threonine derivative (1)"). A peptide having a sugar chain added thereto having a threonine derivative (1) at the N-terminus can also be favorably applied to ligation. Moreover, the threonine derivative (1) can be converted to Thr after ligation. Accordingly, if the N-terminus of a peptide having a sugar chain added thereto is made to be e.g. position 61 of the amino acid sequence shown in SEQ ID NO: 1, by converting the threonine derivative (1) to Thr after ligation, it can be made to be the same amino acid sequence as the naturally occurring form. A method for converting the threonine derivative (1) to Thr will be described below.

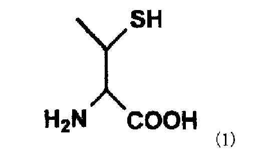

In addition, since the peptide having a sugar chain added thereto will be synthesized by solid-phase synthesis, it is preferably 50 amino acids or less, and further preferably 40 amino acids or less. The peptide having a sugar chain added thereto is preferably 3 amino acids or more, further preferably 6 amino acids or more.
Further, when synthesis is by solid-phase synthesis, since it is not desirable to attach so many amino acids after binding the sugar chain-added amino acid, the N-terminus of the peptide having a sugar chain added thereto is preferably a position within 30-1 amino acids, more preferably a position within 20-1 amino acids, further preferably within 10-1 amino acids from the N-terminal side of the sugar chain-added amino acid.

Moreover, it is preferred that the C-terminus of the above peptide having a sugar chain added thereto is an amino acid that flanks on the N-terminal side of the closest Cys, Ser, Thr or Ala on the C-terminal side of the peptide having a sugar chain added thereto. As a result, the C-terminal partial peptide of a peptide having a sugar chain added thereto will have Cys, Ser, Thr or Ala at the N-terminus.
The C-terminal partial peptide can be directly subjected to ligation if its N-terminus is Cys, and the amino acid sequence after ligation will be the same as that in a naturally occurring form. If the N-terminus of the C-terminal partial peptide is Ser or Ala, by making its N-terminus Cys, and converting Cys to Ser or Ala after ligation upon production of the C-terminal partial peptide, an amino acid sequence that is the same as the naturally occurring form can be obtained. If the N-terminus of the C-terminal partial peptide is Thr, by making its N-terminus a threonine derivative (1), and converting the threonine derivative (1) to Thr after ligation upon production of the C-terminal partial peptide, an amino acid sequence that is the same as the naturally occurring form can be obtained.

The process for producing the IgG-Fc fragment according to the present invention also comprises the step of expressing at least one of a partial peptide on the N-terminal side of the peptide having a sugar chain added thereto (N-terminal partial peptide) and a partial peptide on the C-terminal side of the peptide having a sugar chain added thereto (C-terminal partial peptide) of an IgG-Fc fragment as described above by an expression system, and synthesizing the remainder by solid-phase synthesis.
Since a method for expression by an expression system allows for the production of a polypeptide at a lower cost and with greater efficiency than solid-phase synthesis if an efficient expression system therefor can be constructed, it is particularly preferably used for the production of a relatively long partial peptide not comprising any sugar chain. On the other hand, solid-phase synthesis allows for the production of a polypeptide having an accurate amino acid sequence, and also prevents excess sugar chains to be added thereto by post-translational modification. Those skilled in the art can appropriately decide whether the N-terminal and C-terminal partial peptides should be expressed by an expression system or synthesized by solid-phase synthesis depending on the length or characteristic of each.

Moreover, the N-terminal and C-terminal partial peptides can be further divided depending on its length, each of these expressed by an expression system or synthesized by solid-phase synthesis, and then subjected to ligation. Upon further division, by designing the division so that its N-terminus is Cys, Ser, Thr or Ala, they can be favorably applied to subsequent ligation. The Cys residue can be converted to Ser or Ala after ligation. Accordingly, Ser or Ala can also be employed as the N-terminal amino acid of the partial peptide.

Moreover, a method for converting a Cys residue to Ser is described, e.g., in International Publication WO 2009/17154, the disclosure of which is incorporated herein by reference in its entirety. More specifically, examples include a method comprising:
(a) a step of converting said - SH group into an -SMe group by reacting the -SH group of the Cys residue with a methylating agent;
(b) a step of producing a reaction intermediate by reacting the -SMe group obtained in step (a) with a cyanidation agent; and
(c) a step of converting the reaction intermediate obtained in step (b) to a Ser residue under a condition more basic than step (b).
A method for converting Cys residue to Ala is also described, e.g., in Wan et al., Angew. Chem. Int. Ed., 2007, 45, 9248-9252, the disclosure of which is incorporated herein by reference in its entirety.

Further, a peptide in which the N-terminus is a threonine derivative (1) can also be favorably applied to ligation.
This threonine derivative (1) can be converted to Thr after ligation. Accordingly, Thr can also be employed as the N-terminal amino acid of the partial peptide. A method for converting the Thr derivative to Thr is described, e.g., in International Publication WO 2009/17154, the disclosure of which is incorporated herein by reference in its entirety. More specifically, examples include a method comprising:
(a) a step of converting said -SH group into an -SMe group by reacting the -SH group of the threonine derivative (1) residue in the peptide with a methylating agent;
(b) a step of producing a reaction intermediate by reacting the -SMe group obtained in step (a) with a cyanidation agent; and
(c) a step of converting the reaction intermediate obtained in step (b) to a peptide comprising a threonine residue under a condition more basic than step (b).

### (Expression by an expression system)

The expression system used herein is an expression system which employs microorganism such as bacteria or animal and plant cells as the host cell. A method for preparing a polypeptide chain by an expression system is well-known to those skilled in the art, and typically comprises a step of preparing a nucleic acid molecule encoding the partial peptide to be expressed; a step of introducing the prepared nucleic acid molecule into the host cell of the expression system; a step of culturing and proliferating the transformant obtained by introduction to express the desired partial peptide; and a step of purifying the produced polypeptide chain as necessary.

Any method known in the corresponding technical field can be used as the method for preparing a nucleic acid molecule encoding the polypeptide chain to be expressed. Examples include a method of generating a cDNA from the mRNA of the cell expressing the polypeptide chain of interest by e.g. RT-PCR, and performing nucleic acid amplification such as PCR using a primer suitable for the template. The nucleic acid molecule obtained can be cloned into various vectors and stored. Specific vectors compatible with a predetermined host are well-known to those skilled in the art, many of which are commercially available.

Moreover, in the present invention, to facilitate the recovery of the expressed polypeptide, a purification tag composed of a polypeptide that binds to a particular substance is expressed as a fusion protein with the polypeptide of interest. The purification tag is not particularly limited as long as it serves its function, and examples include a His tag, a GST tag, an S tag, and a T7 tag. In addition, in the present invention, the terminus of these tags which binds to the polypeptide of interest is made so that it comprises Met. Therefore, a nucleic acid molecule encoding the protein of interest is linked to a nucleic acid molecule encoding Met and a purification tag and cloned into a vector.

The expressed fusion protein of the purification tag with the polypeptide of interest can be purified by binding the purification tag with the above particular substance. If the purification tag binds to the polypeptide of interest via Met, the purification tag and the polypeptide of interest can be cleaved for example by degrading Met with CNBr.

When performing this cleaving step, it is preferred to add a strong acid and a water-miscible solvent such as acetonitrile, thereby allowing for improvement of the production rate of the polypeptide of interest. Moreover, since Met contained in the polypeptide of interest will also be degraded when cleaving the purification tag, it is preferred to design the nucleic acid molecule encoding the protein of interest so that Met contained in the polypeptide expressed by the expression system is substituted with an another predetermined amino acid (e.g. including but not limited to Leu).

Any method known in the corresponding technical field can be used as the method for introducing the prepared nucleic acid molecule into the microorganism or host cell of the expression system. Examples include numerous well-known techniques which encompass a method for integrating the gene into a virus vector and infecting a mesenchymal cell with the vector for introduction, as well as direct delivery of gene by calcium phosphate transfection, DEAE-dextran transfection, protoplast fusion, electroporation, liposome fusion, transfection by polybrene, and laser microperforation of cell membrane. Those skilled in the art can also use any technique other than those stated above for the present invention, which allows for integration of said gene into a cell genome and suitable introduction into a cell in a way to allow expression of the gene.
Any microorganism or cells such as yeasts, animal cells, insect cells, and plant cells can be used for the preparation of the expression system, although bacteria, in particular E. coli is preferred in terms of production efficiency or ease of handling. In an aspect of the production process of the present invention, since sugar chains having identical structure are synthetically bound, an expression system in which no sugar chain is added to the polypeptide chain, e.g. a bacterial expression system is preferred.

For the medium for culturing a transformant obtained by using prokaryotes such as E. coli or eukaryotes such as yeast as the host, either natural or synthetic media may be used, as long as it contains a carbon source, a nitrogen source, and inorganic salts etc. that may be assimilated by the organism, and culturing of transformant is efficiently carried out. The carbon source may be any that may be assimilated by the organism, and glucose, fructose, sucrose, molasses containing these, carbohydrates such as starch or starch hydrolysate, organic acids such as acetic acid and propionic acid, and alcohols such as ethanol and propanol can be employed. For the nitrogen source, ammonia, an ammonium salt of inorganic or organic acids such as ammonium chloride, ammonium sulfate, ammonium acetate, and ammonium phosphate, other nitrogen-containing compounds, as well as peptone, meat extract, yeast extract, corn steep liquor, casein hydrolysate, soybean cake, and soybean cake hydrolysate, and various zymocytes and digests thereof etc. can be employed. For inorganic salts, monopotassium phosphate, dipotassium phosphate, magnesium phosphate, magnesium sulfate, sodium chloride, ferrous sulfate, manganese sulfate, copper sulfate, and calcium carbonate etc. can be employed.

The culturing of a transformant using prokaryotes such as E. coli or eukaryotes such as yeast as the host is usually carried out under aerobic conditions such as by shaking culture or deep aeration stirring culture. The culture temperature may be 15-40°C, and culture time is usually 16 hours to 7 days. The pH during culture is maintained at 3.0-9.0. The adjustment of pH is performed using an inorganic or organic acid, an alkali solution, urea, calcium carbonate, and ammonia etc. Antibiotics such as ampicillin or tetracyclin may also be added to the media as necessary. When cultivating a microorganism transformed with a recombinant vector using an inducible promoter as the promoter, an inducer may be added to the media as necessary. For example, when cultivating a microorganism transformed with a recombinant vector using a lac promoter, isopropyl-β-D-thiogalactopyranoside etc. may be added to the media, and when cultivating a microorganism transformed with a recombinant vector using a trp promoter, indoleacrylic acid etc. may be added to the media.

The transformant in cultivation can express the polypeptide naturally or by induction, and produce and accumulate this in the culture. Methods for expressing the polypeptide other than direct expression which can be carried out are e.g. production by secretion and expression of fusion protein. Polypeptide production methods include a method by production within the host cell, a method by secretion outside the host cell, or a method by production on the host extracellular membrane, and the method can be selected by altering the host cell used or the structure of the polypeptide to be produced.

For a polypeptide produced by a transformant into which a gene encoding the polypeptide is introduced, for example when the polypeptide is expressed in a dissolved state within the cell, after the culturing is complete, cells are recovered by centrifugation, suspended in a water-based buffer, and then cells are homogenized with e.g. an ultrasound homogenizer, a french press, Manton-Gaulin homogenizer, or Dynomill to obtain a cell-free extract. From the supernatant obtained by centrifuging the cell-free extract, an ordinary method for enzyme isolation and purification, i.e., methods such as solvent extraction, salt precipitation by e.g. ammonium sulfate, desalting, precipitation by an organic solvent, diethylaminoethyl (DEAE)-Sepharose, anion exchange chromatography using a resin such as Diaion® HPA, cation exchange chromatography using a resin such as Sepharose FF, hydrophobic chromatography using a resin such as butylSepharose and phenylSepharose, gel filtration using molecular sieve, affinity chromatography, chromatofocusing, electrophoresis such as isoelectric electrophoresis are used alone or in combination, and a purified preparation of the polypeptide can be obtained.

The above cell-free extract comprising the polypeptide of interest can be subjected to a step of inactivating or removing the organism-derived materials before or after the above purification step, preferably after the purification step. This step comprises, but is not limited to, any method known to those skilled in the art such as heat treatment, filtration, organic solvent treatment, purification using a column etc., phototreatment using a psoralen derivative etc., and ozone treatment. Heat treatment, for example, can be performed under a temperature and duration condition of 60-65°C for 10-144 hours that is used in treatment of fibrinogen and albumin formulation etc.

If the polypeptide is expressed by forming an insoluble form within cells, cell are homogenized after recovery, and centrifuged to recover the insoluble form of the polypeptide as a precipitated fraction. The insoluble form of the recovered polypeptide is dissolved with a protein denaturant. After returning the polypeptide to its normal conformation by dilution or dialysis of the dissolved solution, isolation and purification similar to the above can provide a purified preparation of the polypeptide.

If the polypeptide is secreted outside of the cell, the polypeptide or a derivative thereof can be recovered from the culture supernatant. In other words, the culture is treated by a method such as centrifugation to obtain the soluble fraction, and a purified preparation of the polypeptide can be obtained from the soluble fraction by isolation and purification similar to the above.

As described above, in the method of the present invention, the polypeptide of interest and a purification tag is fused via Met and expressed. After purifying this fusion protein by binding it to a substance the purification tag has affinity to, treatment with CNBr etc. in the presence of a strong acid and a water-miscible solvent will degrade Met and the polypeptide of interest is cleaved from the purification tag.

As used herein, a strong acid refers to an acid that does not contain weak acids such as formic acid and acetic acid. Formic acid may formylate the polypeptide chain and acetic acid may acetylate the polypeptide chain, which may decrease the production rate of the protein of interest. In the present invention, strong acids include acids weaker than acids generally referred to as a strong acid, provided that it does not cause such formylation. Examples of preferred strong acids can include, but are not limited to, trifluoroacetic acid (TFA), hydrogen fluoride, and methanesulfonic acid.
The strong acid is preferably added at a concentration of 0.1%-50%, more preferably 1%-20%, and further preferably 1%-5%.

Further, in the present invention, the water-miscible solvent is not particularly limited as long as it is a solvent that is water-miscible, and examples can include acetonitrile, trifluoroethanol, dimethylformamide, dimethylsulfoxide (DMSO), and methylene chloride, among which acetonitrile, trifluoroethanol, and dimethylformamide are preferred.
The solvent is preferably added at a concentration of 1%-70%, more preferably 20%-60%, and further preferably, 35%-50%.
In this way, by cleaving in the presence of a strong acid and a water-miscible solvent, it is possible to increase the production rate of the polypeptide of interest without causing formylation etc. of the polypeptide of interest.

In the production process according to the present invention, portions other than the peptide having a sugar chain added thereto and the partial peptide to be expressed by an expression system from an IgG-Fc fragment can be appropriately accurately synthesized by solid-phase synthesis. The solid-phase synthesis eliminates the use of a sugar chain-added amino acid, and can be carried out by following the synthesis by solid-phase synthesis of a sugar chain peptide as described above.

### (Linking step by ligation)

Next, in the process for producing the IgG-Fc fragment according to the present invention, a peptide having a sugar chain added thereto, a partial peptide expressed by an expression system, and a partial peptide synthesized by solid-phase synthesis are linked by ligation.
Moreover, examples of "ligation" as used herein include native chemical ligation (NCL) and kinetically controlled ligation (KCL). NCL is described, e.g., in International Publication WO 96/34878, the disclosure of which is incorporated herein by reference in its entirety. KCL is a reaction kinetics-controlled NCL reported by Kent (Kent et al., Angew. Chem. Int. Ed., 2006, 45, 3985-3988).

Linking by ligation can be performed in any of between peptide-peptide, between peptide-glycopeptide, and between glycopeptide-glycopeptide.
A peptide (or glycopeptide) having an α-carboxythioester portion at the C-terminus used in the ligation can be produced by a method well-known to those skilled in the art as described in International Publication WO 96/34878.

For example, as described in the Examples below, a protected peptide (or glycopeptide) in which the amino acid side chain and the N-terminal amino group are protected is obtained by solid-phase synthesis, the carboxyl group on the C-terminal side thereof is condensed with benzylthio in liquid-phase using PyBOP (Benzotriazole-1-yl-oxy-tris-pyrrolidino-phosphonium hexafluorophosphate)/DIPEA as the condensation agent, and then 95% TFA solution is used to deprotect the amino acid chain to obtain a peptide (or glycopeptide) having an α-carboxythioester at the C-terminus.

Ligation can be carried out using a method well-known to such as those described in International Publication WO 96/34878, as well as by referring to the description in the following Examples. For example, a first peptide having an α-carboxythioester portion represented by -C(=O)-SR at the C-terminus and a second peptide having an amino acid residue having an -SH group at the N-terminus are prepared by referring to International Publication WO 96/34878. Moreover, in the first peptide, R is not particularly limited as long as it does not inhibit thiol exchange reaction and becomes a leaving group in the nucleophilic substitution reaction to the carbonyl carbon, and preferably selected from e.g. a benzyl such as benzylmercaptan, thiophenol, an aryl such as 4-(carboxymethyl)-thiophenol, and an alkyl such as 2-mercaptoethanesulfonate and 3-mercaptopropionic acid amide. The -SH group at the N-terminal of the second peptide may also be protected by a protecting group as desired, but this protecting group is deprotected at a desired time point before the ligation reaction below, and the second peptide having a -SH group at the N-terminus reacts with the first peptide. If the protecting group is one which is naturally deprotected under a condition in which ligation occur, such as a disulfide group, the second peptide protected by the protecting group can be directly used in the ligation reaction below. The disulfide group is easily deprotected under the reaction condition of subsequent ligation.

These two peptides are mixed in a solution such as 100 mM phosphate buffer, in the presence of a catalytic thiol such as 4-mercaptophenylacetic acid, benzylmercaptan, thiophenol as necessary. Preferably, the proportion of 0.5-2 equivalents of the second peptide and about 5 equivalents of catalytic thiol for 1 equivalent of the first peptide are used for reaction. The reaction is desirably performed at a condition of pH about 6.5-7.5 at about 20-40°C for about 1-30 hours. The progress of reaction can be confirmed by a well-known method which is a combination of HPLC, MS etc.

A reducing agent such as Tris 2-carboxyethylphospine hydrochloride (TCEP) is added to this to suppress side reactions, and optionally purified to allow linking of the first peptide and the second peptide.

Moreover, in a peptide having a carboxythioester portion (-C=O-SR) at the C-terminus, if a peptide having a different R group exists, it is possible to manipulate the order of the ligation reaction (see e.g. Protein Science (2007), 16: 2056-2064), and this can be taken into consideration when performing multiple ligations. For example, if aryl, benzyl and alkyl groups exist as R, since the linking reaction generally proceeds in this order, when performing the ligation reaction between peptide a having - C=O-SR at the C-terminus (wherein R is an aryl group) with peptide b having -C=O-SR at the C-terminus (wherein R is a benzyl group) and a SH group at the N-terminus, ligation first occurs between the C-terminus of peptide a and the N-terminus of peptide b, and the obtained peptide a + b having -C=O-SR (wherein R is a benzyl group) at the C-terminus can be used for a further ligation reaction between another peptide c having a SH group at the N-terminus.

### (Folding step)

After ligating the full-length IgG-Fc fragment by ligation, the IgG-Fc fragment can be treated to have an appropriate conformation by performing a folding step.
Various well-known methods can be used for the folding step, for example it can be carried out by dialysis in a folding buffer. The folding buffer may comprise for example a compound having a guanidino group such as guanidine or a salt thereof, and the pH may be 6.0-9.0. Dialysis may be performed multiple times, and the composition or pH of the buffer for each dialysis treatment may be the same or different.
Folding of the polypeptide can be confirmed by any method for analyzing polypeptide conformation, and examples include, but are not limited to, disulfide mapping, evaluation of binding to an antibody specific to a conformational epitope, and X-ray crystallography.

### (In the case of an IgG1-Fc fragment)

The present invention also provides a process for producing an IgG1-Fc fragment. IgG1-Fc fragments include an IgG1-Fc fragment comprising the amino acid sequence shown in SEQ ID NO: 1 as described above, as well as a peptide having one or several amino acids deleted, substituted, or added from the amino acid sequence shown in SEQ ID NO: 1, a peptide having one or several amino acids conservatively substituted from the amino acid sequence shown in SEQ ID NO: 1, and an IgG-Fc variant.

When producing an IgG1-Fc fragment, the N-terminus of a peptide having a sugar chain added thereto is preferably at positions 39-68 of the amino acid sequence shown in SEQ ID NO: 1, more preferably at positions 49-68, and further preferably at positions 59-68, for example, it can be at position 65. It is also preferred that the C-terminus is up to the closest Cys at position 93 on the C-terminal side, i.e. up to Lys at position 92. This length will allow uneventful synthesis by solid-phase synthesis, and there is little effect on the sugar chain since after binding the sugar chain asparagine at position 69 in the solid-phase synthesis there remains only to attach 4 amino acids.

When the N-terminus of a peptide having a sugar chain added thereto is made to be position 65, if Glu at position 65 is substituted by Cys for synthesis, the peptide having a sugar chain added thereto can be favorably subjected to ligation.
The N-terminus of a peptide having a sugar chain added thereto can also be made to be e.g. Ser at position 39, Ala at position 59, or Thr at position 61. If the N-terminus is positions 39 or 59, by substituting the N-terminus to Cys for synthesis, and then converting this to Ser or Ala after ligation, an amino acid sequence that is the same as the naturally occurring form can be obtained. If the N-terminus is position 61, by substituting the N-terminus to a threonine derivative (1) for synthesis, and then converting this to Thr after ligation, an amino acid sequence that is the same as the naturally occurring form can be obtained.

Moreover, the C-terminus of a peptide having a sugar chain added thereto may be short of the closest Ser, Ala or Thr. In this way, by making the N-terminus of the C-terminal partial peptide Cys or a threonine derivative (1) as described above, and converting it to Ser, Ala or Thr respectively after ligation, an amino acid sequence that is the same as the naturally occurring form can be obtained.

When the peptide having a sugar chain added thereto is positions 65-92, the N-terminal partial peptide will be positions 1-64 and the C-terminal partial peptide will be positions 93-216. Each of these can be obtained by synthesis by solid-phase synthesis or expression by an expression system, but they may each be divided into smaller partial peptides and produced, and then linked by ligation. When dividing into smaller partial peptides, it is preferred to divide on the N-terminal side of Cys, Ser, Ala or Thr. In this way, the N-terminus of the partial peptide can be made to be Cys, Ser, Ala or Thr, and thus the partial peptides can be synthesized with Cys or threonine derivative (1) suitable for ligation as the N-terminus, and used directly in case of Cys and converted in case of Ser, Ala or Thr to make the binding site the same amino acid sequence as the naturally occurring form.

Among these, Cys is preferred because it does not require conversion. For example, the N-terminal partial peptide may be further divided between Thr at position 32 and Cys at position 33, and each of them synthesized by solid-phase synthesis. When divided this way, the partial peptide of positions 33-64 will also have Cys at the N-terminus, making it readily subjectable to ligation to make the ligation site also the same amino acid sequence as the naturally occurring IgG1-Fc fragment. Similarly, the C-terminal partial peptide may also be divided between Thr at position 138 and Cys at position 139 and/or between Ser at position 196 and Cys at position 197, and each synthesized by solid-phase synthesis. In this way, the N-terminus of each peptide will be Cys.

The C-terminal partial peptide may also be expressed in full-length by an expression system. According to this method, it is possible to efficiently produce a relatively long C-terminal partial peptide in a large amount and at a low cost. In this case, it is preferred that Met at positions 130 and 200 contained in the C-terminal partial peptide is designed to be expressed as substituted with Leu etc. to prevent degradation upon cleaving of the purification tag.

### (Chimeric antibody)

The chimeric antibody according to the present invention is a chimeric antibody comprising a human IgG-Fc fragment. It is preferred that the chimeric antibody will comprise as much human antibody portions as possible, as long as it binds specifically to the antigen.
The chimeric antibody of the present invention can be obtained, for example, by expressing the polypeptide portion other than IgG-Fc as a thioester using the intein method, and linking by ligation the IgG-Fc fragment having a sugar chain added thereto according to the present invention with an IgG-Fc fragment having a sugar chain added thereto produced by the process for producing an IgG-Fc fragment having a substantially homogeneous sugar chain added thereto according to the present invention. In particular, IgG-Fc fragment can be favorably subjected to ligation because its N-terminus is Cys.
The chimeric antibody according to the present invention is homogenous in the amino acid sequence, the sugar chain structure, the position the sugar chain is added to and conformation. There is no lot-to-lot difference, and is thus useful as pharmaceuticals or a research tool.

### (Method for designing the process of producing an IgG-Fc fragment)

The present invention also provides a method for designing a process for producing an IgG-Fc fragment having a homogenous sugar chain added thereto.
The designing method produces the IgG-Fc fragment divided into a peptide having a sugar chain added thereto comprising a sugar chain-added amino acid and one or more other peptides, wherein the peptide having a sugar chain added thereto has any amino acid at positions 1-30 from the sugar chain-added amino acid on the N-terminal side of said sugar chain-added amino acid as the N-terminus, and has an amino acid that flanks on the N-terminal side of the closest Cys, Ser, Thr or Ala of the C-terminal side of the sugar chain-added amino acid as the C-terminus, and wherein the peptide having a sugar chain added thereto is synthesized by solid-phase synthesis employing an amino acid having a homogenous sugar chain added thereto.

It is possible to favorably synthesize a peptide having a sugar chain added thereto by synthesizing it by solid-phase synthesis as a relatively short peptide having a sugar chain added thereto comprising a sugar chain-added amino acid. When using solid-phase synthesis, a peptide will be synthesized in the orientation of C- to N-terminus. Accordingly, by making the N-terminus of a peptide having a sugar chain added thereto in the range of positions 1-30, preferably positions 1-20, and more preferably positions 1-10 from sugar chain-added amino acid on the N-terminal side of the sugar chain-added amino acid, the number of amino acids to be attached after binding of the sugar chain-added amino acid can be limited and therefore suppress the effect of synthesis on the sugar chain. It is further preferred to have the N-terminus of a peptide having a sugar chain added thereto within the range of 5-1 amino acids from the N-terminal side of the sugar chain-added amino acids.

If Cys is present within a range of 1-30 amino acids from the N-terminal side of the sugar chain-added amino acid, the synthesized peptide having a sugar chain added thereto can be directly subjected to ligation by making Cys the N-terminus and will also have the same amino acid sequence as the naturally occurring form after ligation. If Cys is not present within a range of 1-30 amino acids from the N-terminal side of the sugar chain-added amino acid as with IgG1-Fc, the amino acid to be the N-terminus may be substituted with Cys for synthesis upon synthesizing the peptide having a sugar chain added thereto.
Cys can also be converted to Ala or Ser after ligation. Accordingly, if positions 39 or 59 in the amino acid sequence shown in SEQ ID NO: 1 are employed as the N-terminus of a peptide having a sugar chain added thereto, by substituting the N-terminus of a peptide having a sugar chain added thereto to Cys for synthesis, and then converting it to Ser or Ala after ligation, an amino acid sequence that is the same as the naturally occurring form can be obtained.
If the N-terminus of a peptide having a sugar chain added thereto is substituted with a threonine derivative (1), it can be converted to Thr after ligation, and thus it is also preferred to make the N-terminus of a peptide having a sugar chain added thereto to be position 61 in the amino acid sequence shown in SEQ ID NO: 1.

It is also preferred to make the C-terminus of a peptide having a sugar chain added thereto to be an amino acid that flanks on the N-terminal side of the closest Cys on C-terminal side of the sugar chain-added amino acid. As a result, the C-terminal partial peptide of a peptide having a sugar chain added thereto can have Cys at the N-terminus, thereby making it readily subjectable to the subsequent ligation step, and an amino acid sequence that is the same as the naturally occurring form at the ligation site can be obtained.

The C-terminus of a peptide having a sugar chain added thereto may also be an amino acid that flanks on the N-terminal side of the closest Ser, Ala or Thr on the C-terminal side of the sugar chain-added amino acid. In such a case, by synthesizing the N-terminus of the C-terminal partial peptide as Cys or a threonine derivative (1), and then converting it to Sel, Ala or Thr after ligation, an amino acid sequence that is the same as the naturally occurring form can be obtained.

In addition, in the designing method according to the present invention, peptides other than the peptide having a sugar chain added thereto are either expressed by an expression system or synthesized by solid-phase synthesis.
Those skilled in the art can appropriately determine whether to employ expression by an expression system or synthesis by solid-phase synthesis depending on the length or characteristic of the peptide. Further, if the polypeptide is long, it may be divided into two or more partial peptides on the N-terminal side of Cys, Ser, Ala or Thr contained in the polypeptide, and each of the partial peptides decided whether to be expressed by an expression system or synthesized by solid-phase synthesis. In this way, each of the partial peptides will be readily linked by ligation, and the amino acid sequence after ligation having an amino acid sequence that is the same as the naturally occurring form can be obtained.

In the designing method according to the present invention, when the other peptides are expressed by an expression system, the peptide is expressed as a fusion protein with a purification tag having affinity to a particular substance and comprising Met, purified by binding the purification tag to the particular substance, and the peptide and purification tag are cleaved by degrading Met in the presence of a strong acid and a water-miscible solvent.
By adding a strong acid and a water-miscible solvent, it is possible to improve the production rate of the polypeptide of interest without causing formylation etc. of the peptide of interest.
In doing so, Met contained in the peptide of interest is altered to an amino acid other than Met for expression. In this way, the peptide of interest is prevented from degrading when cleaving the purification tag.

Moreover, terms used herein are employed to describe particular embodiments, and not intended to limit the present invention.
As used herein, unless clearly recognized otherwise from the context, the terms "comprising," "containing," and "including" intend the presence of the stated items (such as members, steps, components, and numbers), and do not exclude the presence of other items (such as members, steps, components, and numbers).

Unless defined otherwise, all terms (including technical and scientific terms) used herein have meanings which are the same as that widely understood by those skilled in the art to which the present invention belongs. Unless specifically defined otherwise, the terms used herein should be construed as having a meaning that is consistent with that herein and in the related technical field, and should not be construed to be idealized or excessively formal meaning.

The embodiments of the present invention are sometimes described referring to the schematic diagrams, but note that in case of a schematic diagram, the expression used may be exaggerated to clarify the description.
Terms such as first and second are used to express various components, and it should be recognized that these components are not to be limited by these terms. These terms are used merely to distinguish one component from another component, for example, stating the first component as the second component, and similarly stating the second component as the first component is possible without departing from the scope of the present invention.

The present invention will now be described in detail referring to Examples below. However, the present invention can be embodied by various aspects, and is not to be construed as limited to the Examples described herein.

### Examples

A human IgG1-Fc fragment comprising the amino acid sequence shown in SEQ ID NO: 2 was synthesized as the IgG-Fc fragment having a sugar chain added thereto according to the present invention. This human IgG1-Fc fragment has Glu at position 65 of the amino acid sequence shown in SEQ ID NO: 1 substituted by Cys, and Met at positions 130 and 200 substituted by Leu in the peptide chain. Gln at position 128 is also substituted by Asp.

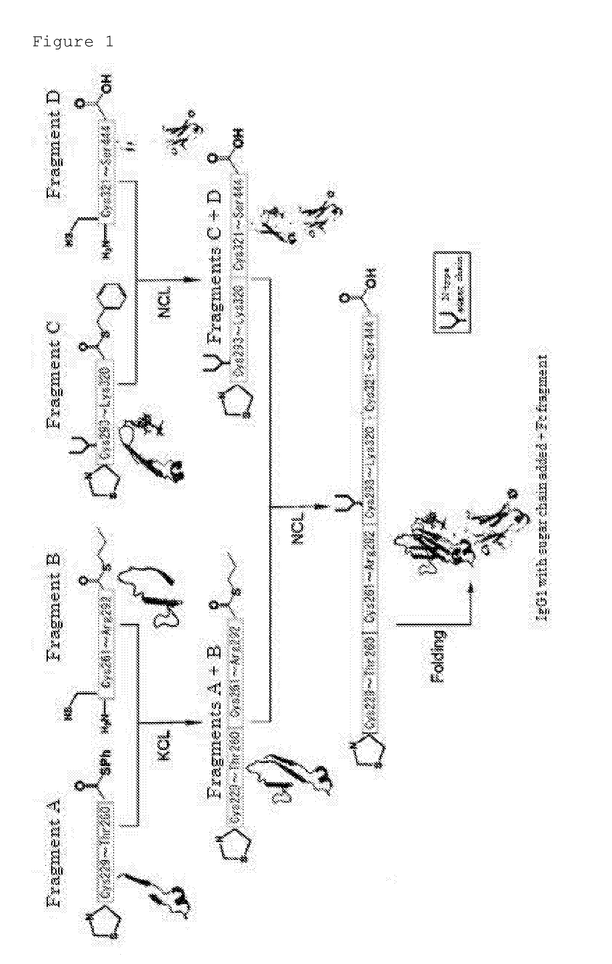

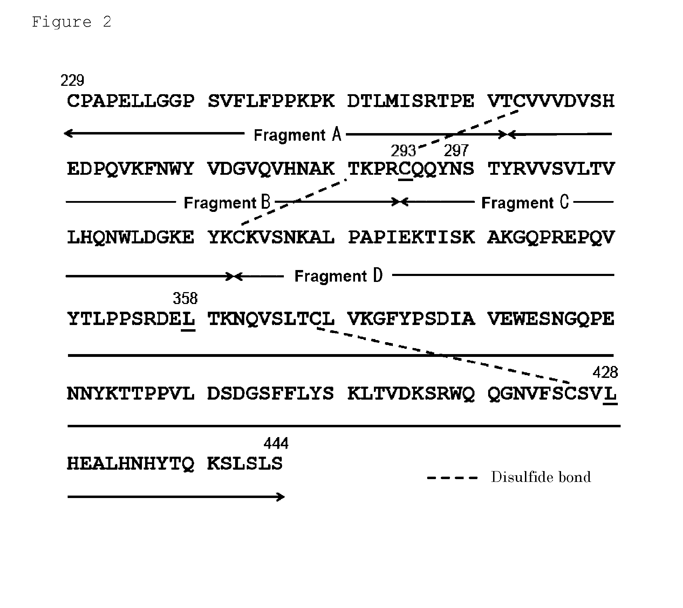

A brief overview of the synthesis is shown in Figure 1, and the amino acid sequence of the IgG1-Fc fragment is shown in Figure 2. Cys at position 1 to Thr at position 32 in SEQ ID NO: 2 was set as fragment A, Cys at position 33 to Arg at position 64 was set as fragment B, Cys at position 65 to Lys at position 92 was set as fragment C, and Cys at position 93 to Ser at position 216 was set as fragment D. Fragments A and B correspond to the N-terminal partial peptide, fragment C corresponds to the peptide having a sugar chain added thereto, and fragment D corresponds to the C-terminal partial peptide. Fragments A-C were chemically synthesized and fragment D was prepared by E. coli expression.

Next, fragments A and B were linked by KCL to generate fragment A + B, and fragments C and D were linked by NCL to generate fragment C + D, after which the two were linked by NCL.
Upon solid-phase synthesis of fragment C, the sugar chain was bound to Asn at position 69 of SEQ ID NO: 2 by employing an asialosugar chain-bound Asn shown below.

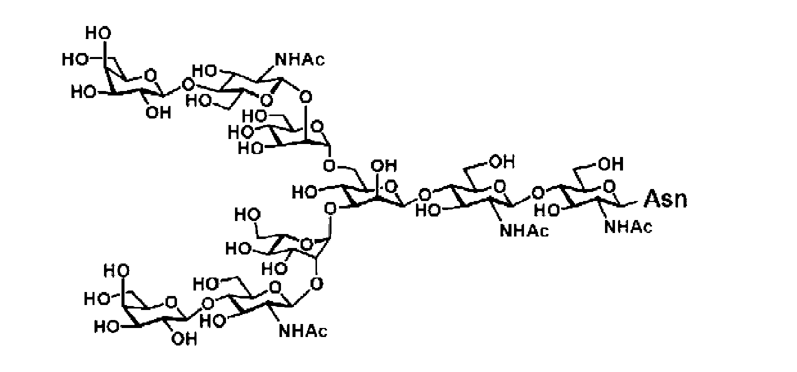

The experiment protocol will now be described in detail below.
In addition, the RP-HPLC analysis device used was from Waters Corporation, the UV detector used was Waters 486 from Waters and a photodiode array detector (Waters 2996), and the column used was Cadenza column (Imtakt Corp., 3 µm, 75 × 4.6 mm), Vydac C-18 (5 µm, 4.6 × 250 mm, 10 × 250 mm) and Superdex 75^{™} 10/300 GL. Esquire 3000 plus from Bruker Daltonics was used for ESI mass measurement.

### <Synthesis of peptide thioester using solid-phase synthesis by Fmoc>

### 1-1. Synthesis of fragment A protected peptide 1

Common solid-phase synthesis by Fmoc was used for extension of peptides. First, Amino PEGA resin (2 g, 100 µmol) was placed in a solid-phase synthesis tube, washed well with DCM (dichloromethane) and DMF (N,N-dimethylformamide), and then swelled well with DMF before use. Two and a half equivalents of HMPB (4-(4-hydroxymethyl-3-metoxyphenoxybutyric acid) (60.0 mg, 0.25 mmol), 2.5 equivalents of TBTU (80.2 mg, 0.25 mmol), and N-ethylmorpholine (31.6 µl, 0.25 mmol) were dissolved in DMF (2 ml), placed in a tube containing the resin, and stirred at room temperature for 2 hours. The resin was washed well with DMF and DCM to provide HMPB-PEGA resin.

This resin was used for solid-phase synthesis. Introduction of the amino acid to the solid phase is as follows.
Five equivalents of Fmoc-Thr (Bu^{t})-OH (198.8 mg, 0.5 mmol), 5 equivalents of MSNT (148 mg, 0.5 mmol), and 3.75 equivalents of N-methylimidazole (29.8 µl, 0.38 mmol) were dissolved in DCM (2 ml, 250 mM), placed in a solid-phase synthesis tube containing the resin, and stirred at room temperature for 2 hours. After stirring, the resin was washed with DCM and DMF. The Fmoc group was deprotected using 20% piperidine/DMF solution (1 ml) for 20 minutes. After washing with DMF, the reaction was confirmed by Kaiser Test, and subjected to condensation with the next amino acid. The process shown below was used for subsequent extension of the peptide chain to sequentially condense the amino acids.
Five equivalents of Fmoc-Val-COOH (169.7 mg, 0.5 mmol), 5 equivalents of HOBt (N-hydroxybenzotriazole)·H₂O (67.6 mg, 0.5 mmol), and 5 equivalents of DIPCDI (77 µl, 0.5 mmol) were mixed with 250 mM DMF solvent (2 ml), activated for 15 minutes, and then placed in a tube containing the resin, and stirred at room temperature for 1 hour. After stirring, the resin was washed with DCM and DMF. The Fmoc group was subsequently deprotected with a process similar to that for the first amino acid.

In this way, condensation and deprotection up to residue 32 were performed. The amino acids used were as follows:
Glu(Bu^{t}) (212.8 mg, 0.5 mmol), Pro (168.7 mg, 0.5 mmol), Thr (But) (198.8 mg, 0.5 mmol), Arg(Pbf) (324.4 mg, 0.5 mmol), Ser(Bu^{t}) (191.8 mg, 0.5 mmol), Ile (176.7 mg, 0.5 mmol), Met (185.8 mg, 0.5 mmol), Leu (176.7 mg, 0.5 mmol), Thr (But) (198.8 mg, 0.5 mmol), Asp(Bu^{t}) (205.8 mg, 0.5 mmol), Lys(Boc) (234.3 mg, 0.5 mmol), Pro (168.7 mg, 0.5 mmol), Lys(Boc) (234.3 mg, 0.5 mmol), Pro (168.7 mg, 0.5 mmol), Pro (168.7 mg, 0.5 mmol), Phe (193.7 mg, 0.5 mmol), Leu (176.7 mg, 0.5 mmol), Phe (193.7 mg, 0.5 mmol), Val (169.7 mg, 0.5 mmol), Ser(Bu^{t}) (191.8 mg, 0.5 mmol), Pro (168.7 mg, 0.5 mmol), Gly (148.7 mg, 0.5 mmol), Gly (148.7 mg, 0.5 mmol), Leu (176.7 mg, 0.5 mmol), Leu (176.7 mg, 0.5 mmol), Glu(Bu^{t}) (212.8 mg, 0.5 mmol), Pro (168.7 mg, 0.5 mmol), Ala (155.7 mg, 0.5 mmol), Pro (168.7 mg, 0.5 mmol), and Boc-L-thiazolidine-4-carboxylic acid (116.6 mg, 0.5 mmol)

Next, the resin of 50 µmol worth of peptide with completed condensation was washed well with DMF and DCM, 7 ml of AcOH:TFE = 1:1 solution was added to this resin, stirred for 24 hours, and the protected peptide was cleaved out from the resin. A recovery flask containing hexane was prepared, the solution containing the dissolved protected peptide was added dropwise to this, and the resin was washed with MeOH. This was concentrated under reduced pressure at room temperature, and subjected to azeotropy with acetic acid and benzene. Purification with HPLC yielded the target side chain-protected peptide 1.

### 1-2. Synthesis of fragment A peptide thioester 3

The protected peptide 1 (50 umol), MS4A (10 mg), and thiophenol (153 µl, 1.5 mmol) were stirred in DMF solvent (6.75 ml) under Ar flow at -20°C for 1.5 hours, PyBOP (130 mg, 0.25 mmol) and DIPEA (42.5 µl, 0.25 mmol) were added, and stirred for 3 hours. Then, diethyl ether was added to the reaction solution on ice, and the compound was allowed to precipitate. After filtration, the precipitate was recovered, 95% TFA aqueous solution comprising DTT was added, and stirred at room temperature for 2 hours. The reaction solution was concentrated under reduced pressure, and purification with HPLC yielded the target peptide thioester 3. HPLC analysis: Cadenza CD-18 (3 µm, 4.6 × 75 mm); developing solvent A: 0.1% TFA aqueous solution; B: 0.1% TFA acetonitrile:water = 90:10; gradient A:B = 95:5 → 25:75, 15 min.; flow rate: 1.0 ml/min
HPLC purification: Vydac C-18 (5 µm, 10 × 250 mm); gradient A:B = 70:30 → 30:70, 30 min.; flow rate: 4.0 ml/min
ESI-MS: m/z calcd for C₁₆₅H₂₅₇N₃₇O₄₃S₃: [M + 2H]²⁺ 1772.6, [M + 3H]³⁺ 1182.1, [M + 4H]⁴⁺ 886.8, found 1772.5, 1182.0, 886.8
The synthetic scheme from fragment A protected peptide 1 to fragment A peptide thioester 3 as described above is shown below.

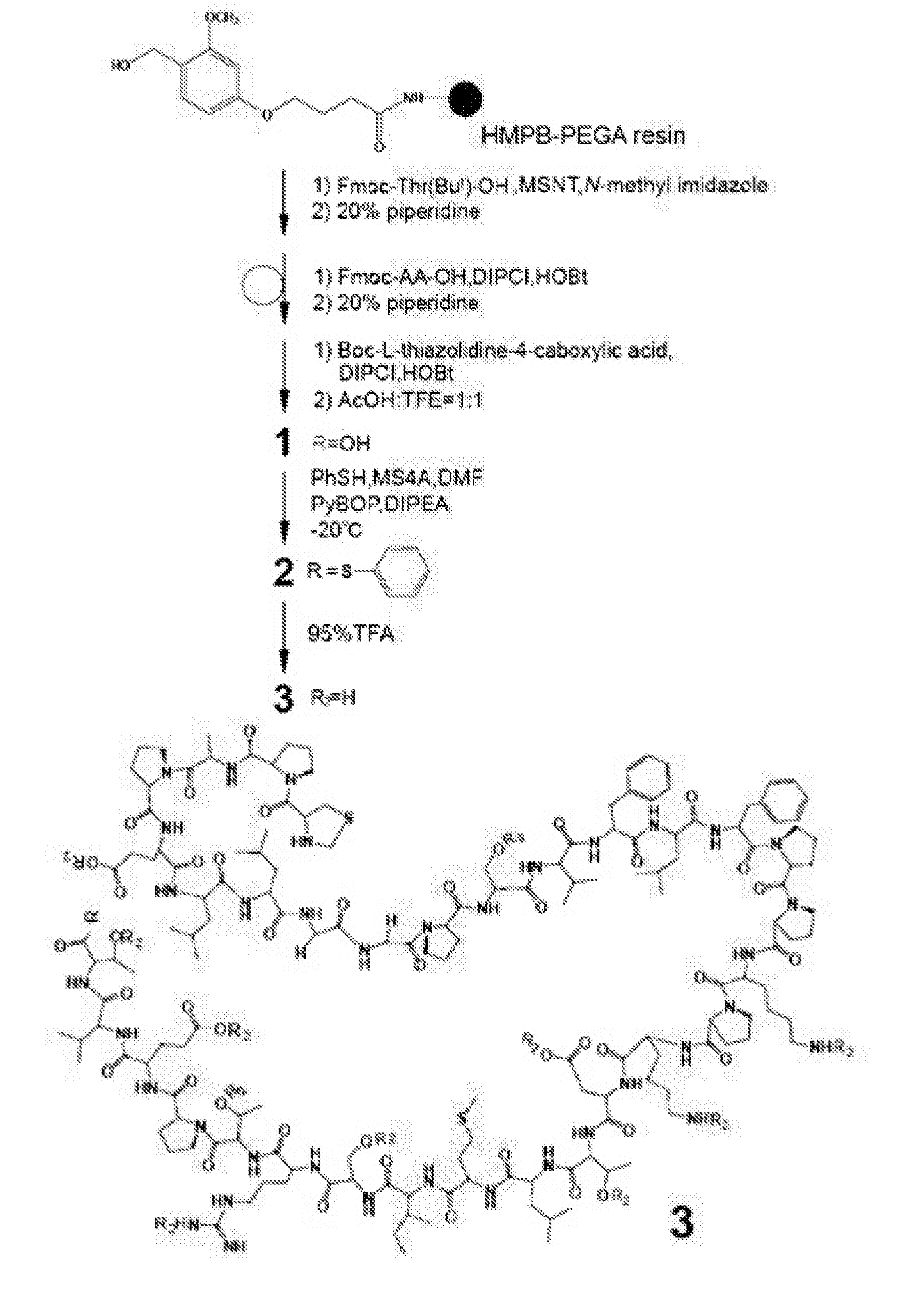

### 2-1. Synthesis of fragment B protected peptide 4

As with fragment A, the resin used was the HMPB-PEGA resin (0.1 mmol), and condensation of amino acids was carried out using Fmoc. Fmoc-Arg(Pbf)-OH (324.4 mg, 0.5 mmol), 5 equivalents of MSNT (1-(mesitylene-2-sulfonyl)-3-nitro-1H-1,2,4-triazole) (148 mg, 0.5 mmol), and 3.75 equivalents of N-methylimidazole (29.8 µl, 0.38 mmol) were dissolved in DCM (2 ml, 250 mM), placed in a solid-phase synthesis tube containing the resin, and stirred at room temperature for 2 hours. After stirring, the resin was washed with DCM and DMF. The Fmoc group was deprotected using 20% piperidine/DMF solution (1 ml) for 20 minutes. After washing with DMF, the reaction was confirmed by Kaiser Test, and subjected to condensation with the next amino acid. The process shown below was used for subsequent extension of the peptide chain to sequentially condense the amino acids.

Five equivalents of Fmoc-Pro-COOH (168.7 mg, 0.5 mmol), 5 equivalents of HOBt˙H₂O (67.6 mg, 0.5 mmol), and 5 equivalents of DIPCDI (77 µl, 0.5 mmol) were mixed with 250 mM DMF solvent (2 ml), activated for 15 minutes, and then placed in a tube containing the resin, and stirred at room temperature for 1 hour. After stirring, the resin was washed with DCM and DMF. The Fmoc group was subsequently deprotected with a process similar to that for the first amino acid.
In this way, condensation and deprotection up to residue 32 were performed. The amino acids used were as follows:
Lys(Boc) (234.3 mg, 0.5 mmol), Thr (But) (198.8 mg, 0.5 mmol), Lys(Boc) (234.3 mg, 0.5 mmol), Ala (155.7 mg, 0.5 mmol), Asn(Trt) (298.4 mg, 0.5 mmol), His(Trt) (309.9 mg, 0.5 mmol), Val (169.7 mg, 0.5 mmol), Gln (184.2 mg, 0.5 mmol), Val (169.7 mg, 0.5 mmol), Gly (148.7 mg, 0.5 mmol), Asp(Bu^{t}) (205.8 mg, 0.5 mmol), Val (169.7 mg, 0.5 mmol), Tyr (229.8 mg, 0.5 mmol), Trp(Boc) (263.3 mg, 0.5 mmol), Asn (177.2 mg, 0.5 mmol), Phe (193.7 mg, 0.5 mmol), Lys(Boc) (234.3 mg, 0.5 mmol), Val (169.7 mg, 0.5 mmol), Gln (184.2 mg, 0.5 mmol), Pro (168.7 mg, 0.5 mmol), Asp(Bu^{t}) (205.8 mg, 0.5 mmol), Glu(Bu^{t}) (212.8 mg, 0.5 mmol), His(Trt) (309.9 mg, 0.5 mmol), Ser(Bu^{t}) (191.8 mg, 0.5 mmol), Val (169.7 mg, 0.5 mmol), Asp(Bu^{t}) (205.8 mg, 0.5 mmol), Val (169.7 mg, 0.5 mmol), Val (169.7 mg, 0.5 mmol), Val (169.7 mg, 0.5 mmol), and Cys(Trt) (292.9 mg, 0.5 mmol)

After deprotection of the Fmoc group of Cys at residue 32, 10 equivalents (1 mmol) of di-tert-butyl dicarbonate to one equivalent of resin was mixed with 5 ml of DMF solvent, and stirred for 2 hours to Boc-protect the amino group of Cys. Moreover, free amino group was confirmed by Kaiser Test (+) after deprotection of Fmoc, Boc-lation was terminated by Kaiser Test (-).

Next, the resin of 50 µmol worth of peptide with completed condensation was washed well with DMF and DCM, 7 ml of AcOH:TFE = 1:1 solution was added to this resin, stirred for 24 hours, and the protected peptide was cleaved out from the resin. A recovery flask containing hexane was prepared, the solution containing the dissolved protected peptide was added dropwise to this, and the resin was washed with MeOH. This was concentrated under reduced pressure at room temperature, and subjected to azeotropy with acetic acid and benzene. Purification with HPLC yielded the target side chain-protected peptide 4.

### 2-2. Synthesis of fragment B peptide thioester 6

The protected peptide 4 (50 µmol), MS4A (10 mg), and 1-propanethiol (136 µl, 1.5 mmol) were stirred in DMF solvent (6.75 ml) under Ar flow at -20°C for 1.5 hours, PyBOP (Benzotriazole-1-yl-oxy-tris-pyrrolidino-phosphonium) (130 mg, 0.25 mmol) and DIPEA (42.5 µl, 0.25 mmol) were added, and stirred for 3 hours. Then, diethyl ether was added to the reaction solution on ice, and the compound was allowed to precipitate. After filtration, the precipitate was recovered, 95% TFA aqueous solution was added, and stirred at room temperature for 2 hours. The reaction solution was concentrated under reduced pressure, and purification with HPLC yielded the target peptide thioester 6.
HPLC analysis: Cadenza CD-18 (3 µm, 4.6 × 75 mm); developing solvent A: 0.1% TFA aqueous solution; B: 0.1% TFA acetonitrile:water = 90:10; gradient A:B = 95:5 → 25:75, 15 min.; flow rate: 1.0 ml/min
HPLC purification: Vydac C-18 (5 µm, 10 × 250 mm); gradient A:B = 75:25 → 55:45, 30 min.; flow rate: 4.0 ml/min
ESI-MS: m/z calcd for C₁₆₈H₂₅₉N₄₇O₄₇S₂: [M + 2H]²⁺ 1877.6, [M + 3H]³⁺ 1252.1, [M + 4H]⁴⁺ 939.3, [M + 5H]⁵⁺ 751.6, found 1878.3, 1252.6, 939.8, 752.0
The synthetic scheme from fragment B protected peptide 4 to fragment B peptide thioester 6 as described above is shown below.

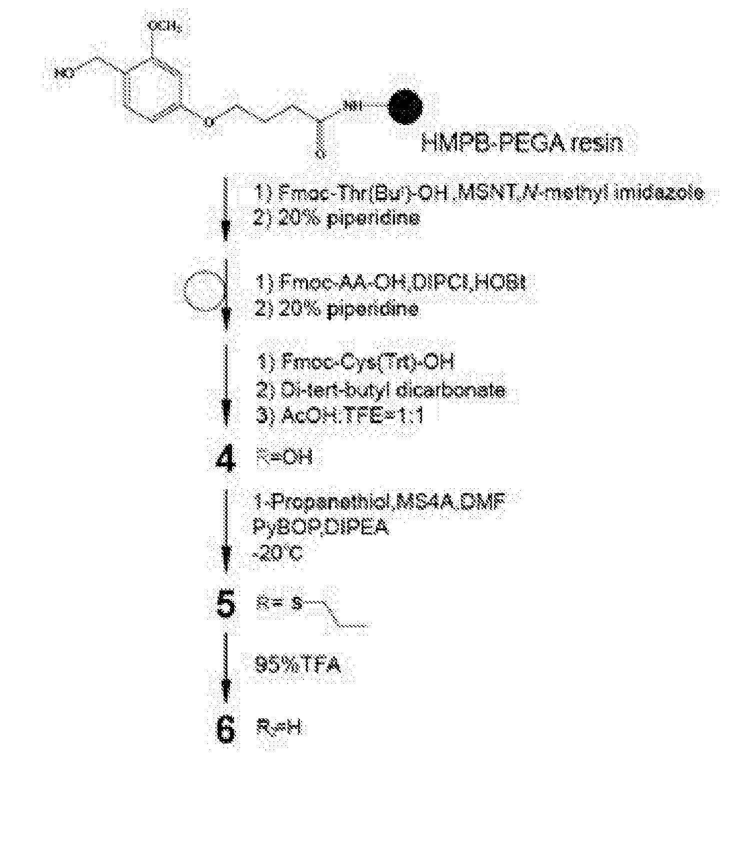

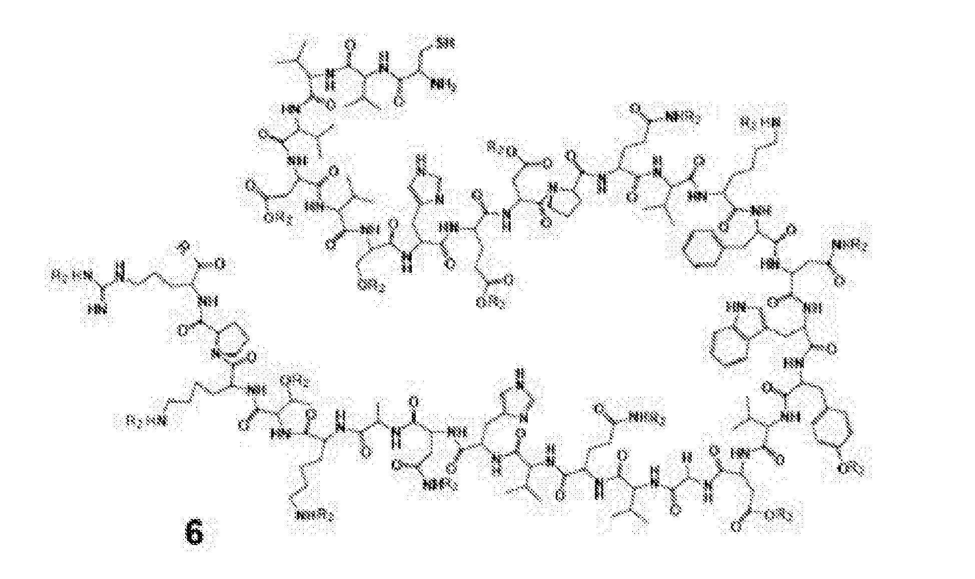

### 3-1. Synthesis of fragment C protected glycopeptide 8 having asialosugar chain 7

As with fragment A and fragment B, the resin used was the HMPB-PEGA resin (0.1 mmol), and condensation of amino acids was carried out using Fmoc. Fmoc-Lys(Boc)-OH (234 mg, 0.5 mmol), 5 equivalents of MSNT (148 mg, 0.5 mmol), and 3.75 equivalents of N-methylimidazole (29.8 µl, 0.38 mmol) were dissolved in DCM (2 ml, 250 mM), placed in a solid-phase synthesis tube containing the resin, and stirred at room temperature for 2 hours. After stirring, the resin was washed with DCM and DMF. The Fmoc group was deprotected using 20% piperidine/DMF solution (1 ml) for 20 minutes. After washing with DMF, the reaction was confirmed by Kaiser Test, and subjected to condensation with the next amino acid.
The process shown below was used for subsequent extension of the peptide chain to sequentially condense the amino acids.

Five equivalents of Fmoc-Tyr(Bu^{t})-OH (229.8 mg, 0.5 mmol), 5 equivalents of HOBt˙H₂O (67.6 mg, 0.5 mmol), and 5 equivalents of DIPCDI (77 µl, 0.5 mmol) were mixed with 250 mM DMF solvent (2 ml), activated for 15 minutes, and then placed in a tube containing the resin, and stirred at room temperature for 1 hour. After stirring, the resin was washed with DCM and DMF. The Fmoc group was subsequently deprotected with a process similar to that for the first amino acid.

In this way, condensation and deprotection up to residue 23 were performed. The amino acids used were as follows:
Glu(Bu^{t}) (212.8 mg, 0.5 mmol), Lys(Boc) (234.3 mg, 0.5 mmol), Gly (148.7 mg, 0.5 mmol), Asp(Bu^{t}) (205.8 mg, 0.5 mmol), Leu (176.7 mg, 0.5 mmol), Trp(Boc) (263.3 mg, 0.5 mmol), Asn (177.2 mg, 0.5 mmol), Gln (184.2 mg, 0.5 mmol), His(Trt) (309.9 mg, 0.5 mmol), Leu (176.7 mg, 0.5 mmol), Val (169.7 mg, 0.5 mmol), Thr (But) (198.8 mg, 0.5 mmol), Leu (176.7 mg, 0.5 mmol), Val (169.7 mg, 0.5 mmol), Ser(Bu^{t}) (191.8 mg, 0.5 mmol), Val (169.7 mg, 0.5 mmol), Val (169.7 mg, 0.5 mmol), Arg(Pbf) (324.4 mg, 0.5 mmol), Tyr (229.8 mg, 0.5 mmol), Thr (But) (198.8 mg, 0.5 mmol), and Ser(Bu^{t}) (191.8 mg, 0.5 mmol)

Next, the resin of 3 µmol worth of this 23-residue peptide was transferred to an Eppendorf tube to condense the asialosugar chain Asn. Two equivalents of asialosugar chain Asn (12 mg, 6 µmol) and 3 equivalents of DEPBT (3-(diethoxyphosphoryloxy)-1,2,3-benzotriazin-4(3H)-one) (3 mg, 9 µmol) were dissolved in a DMF:DMSO = 4:1 solution (201 µl, 30 mM), and placed in an Eppendorf tube containing the resin. Then, 2 equivalents of DIPEA (N,N'-diisopropylethyamine) (1.02 µl, 6 µmol) was added, and stirred at room temperature for 22 hours. The resin was transferred to a solid-phase synthesis tube, washed with DMF, and then the Fmoc group was deprotected using 20% piperidine/DMF solution by a process similar to those described above. The structure of the Fmoc-asialosugar chain 7 is shown below.

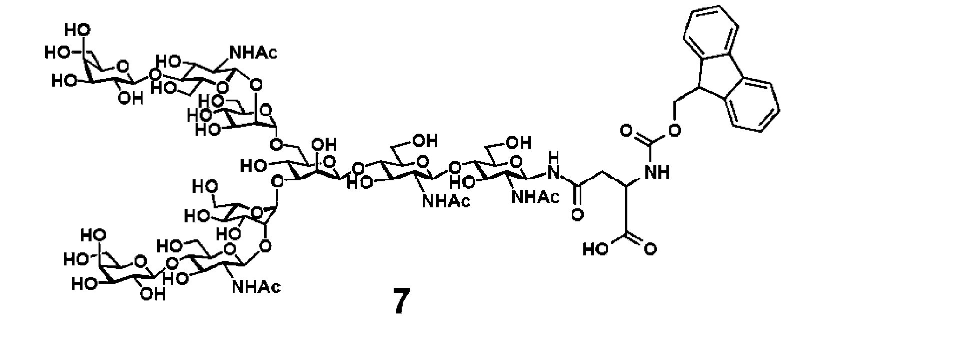

Amino acids were further sequentially condensed with this asialosugar chain peptide. Five equivalents of Fmoc-Tyr(Bu^{t})-OH (6.9 mg, 15 µmol), 5 equivalents of HOBt(2.0 mg, 15 µmol), and 5 equivalents of DIPCI (N,N'-diisopropylcarbodiimide) (2.3 µl, 15 µmol) were dissolved in DMF (375 µl, 40 mM), activated for 15 minutes, and then placed in a solid-phase synthesis tube containing the resin, and stirred at room temperature for 1 hour. After stirring, the resin was washed with DCM and DMF. The Fmoc group was subsequently deprotected with a process similar to that for the first amino acid.

In this way, condensation and deprotection up to residue 28 were performed. The amino acids used were as follows:
Gln (5.5 mg, 15 µmol), Gln (5.5 mg, 15 µmol), and Boc-L-thiazolidine-4-carboxylic acid (3.5 mg, 0.5 mmol)

Next, the resin of 3 µmol worth of glycopeptide with completed condensation was washed well with DMF and DCM, 3 ml of AcOH:TFE = 1:1 solution was added to this resin, stirred for 22 hours, and the protected glycopeptide was cleaved out from the resin. A recovery flask containing hexane was prepared, the solution containing the dissolved protected peptide was added dropwise to this, and the resin was washed with MeOH. This was concentrated under reduced pressure at room temperature, and subjected to azeotropy with acetic acid and benzene. Purification with HPLC yielded the target side chain-protected glycopeptide 8.

### 3-2. Synthesis of fragment C glycopeptide thioester 10

The fragment C protected glycopeptide 8 synthesized as above (3 µmol) was dried well by a vacuum line. This was dissolved in DMF (7.4 mM, 405 µl) under Ar flow, MS4A (10 mg) and 30 equivalents of benzylmercaptan (10.7 µl, 90 µmol) were added, and stirred at -20°C for 1.5 hours. Subsequently, 5 equivalents of PyBOP (7.8 mg, 15 µmol) and 5 equivalents of DIPEA (2.5 µl, 15 µmol) were added, and allowed to react under argon flow for 5.5 hours for thioesterification. After completion of the reaction, diethyl ether was added on ice to allow precipitation of the compound, and after filtration, the precipitate was recovered with DMF. To the solid 9 obtained by concentration under reduced pressure, 95% TFA aqueous solution was added and stirred for 2 hours. After concentration under reduced pressure, HPLC confirmed the purity, and mass spectrometry confirmed the target glycopeptide thioester 10.
HPLC analysis: Cadenza CD-18 (3 µm, 4.6 × 75 mm); developing solvent A: 0.1% TFA aqueous solution; B: 0.1% TFA acetonitrile:water = 90:10; gradient A:B = 95:5 → 25:75, 15 min.; flow rate: 1.0 ml/min
HPLC purification: Vydac C-18 (5 µm, 10 × 250 mm); gradient A:B = 70:30 → 40:60, 30 min.; flow rate: 4.0 ml/min
ESI-MS: m/z calcd for C₂₂₁H₃₃₉N₄₅O₈₉S₂: [M + 3H]³⁺ 1706.1, [M + 4H]⁴⁺ 1279.9, found 1705.8, 1279.6

The synthetic scheme from fragment C protected peptide 8 to fragment C peptide thioester 10 as described above is shown below.

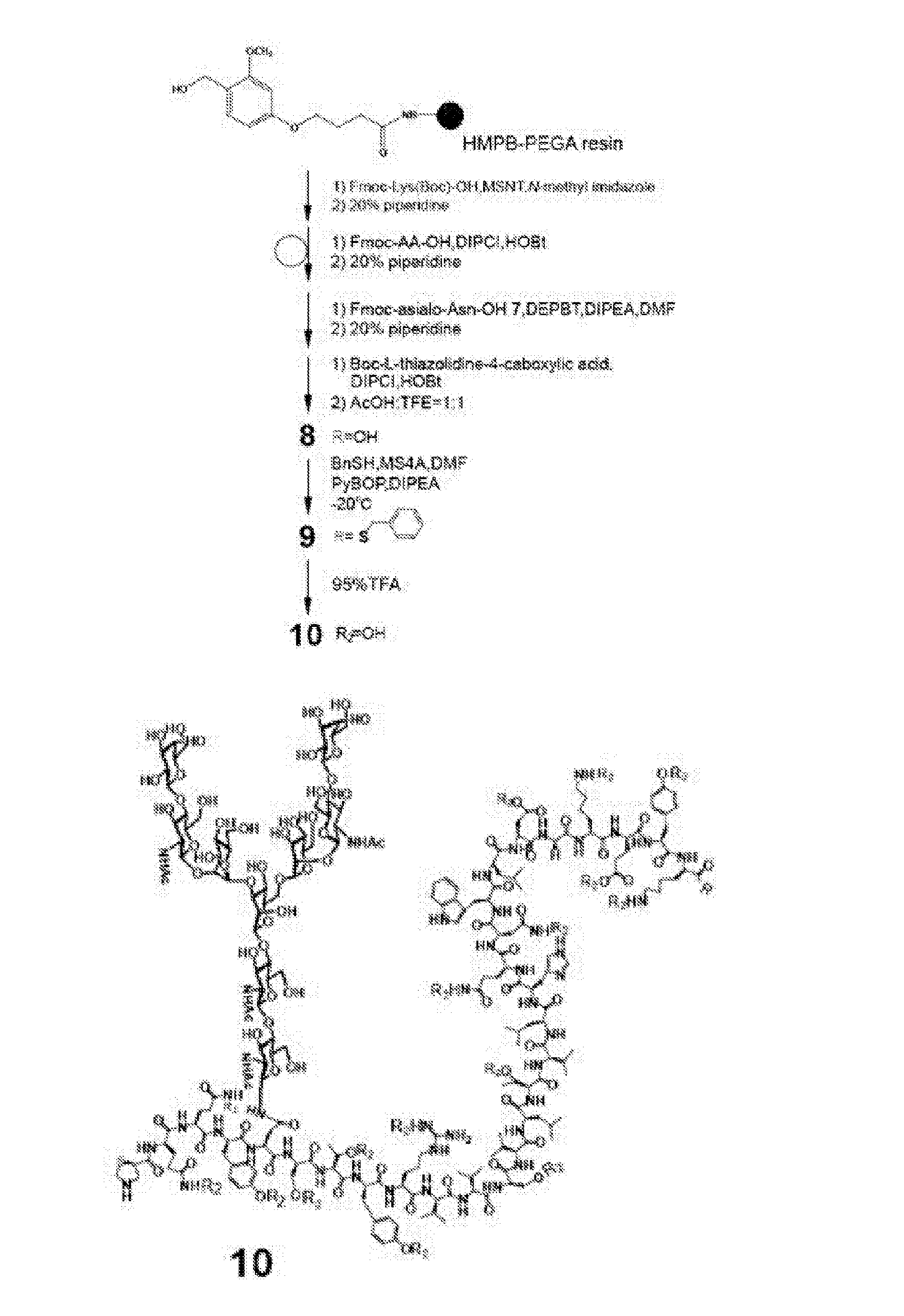

### <Synthesis of peptide fragment using E. coli expression>

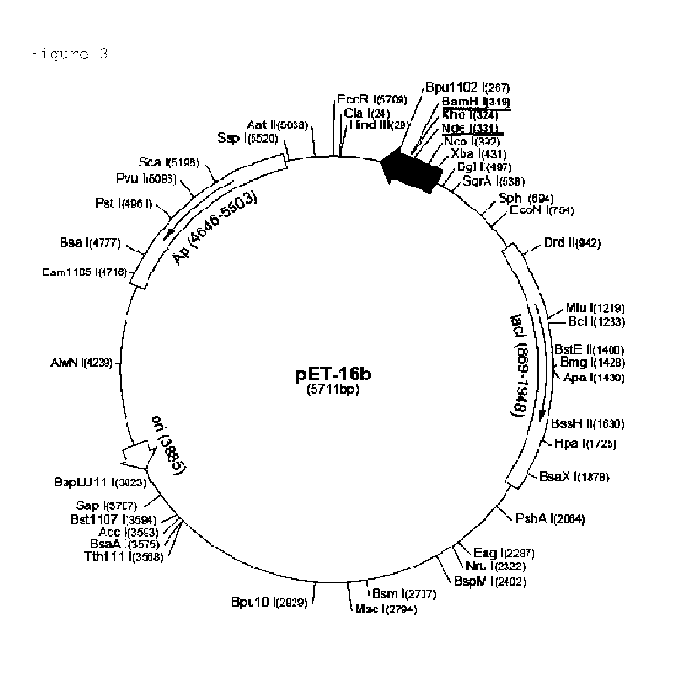

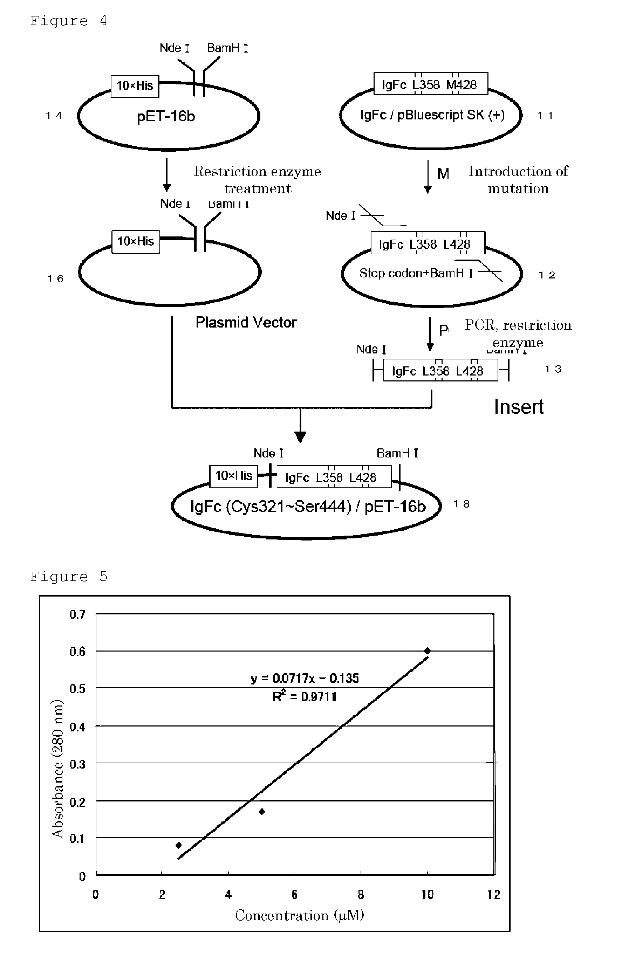

The preparation and purification of fragment D by E. coli expression was performed essentially following the method of Macmillan et al. (Macmillan et al., J. Am. Chem. Soc., 2004, 126, 9530-9531).
Since fragment D was expressed as a fusion protein with a His tag (purification tag), the expression vector used was pET-16b already having a sequence expressing a His tag (14 in Figure 4). The schematic diagram of pET-16b is shown in Figure 3. A brief overview of designing of the expression vector is shown in Figure.

Meanwhile, the structure of the DNA encoding fragment D has already been solved, and IgFc integrated in plasmid vector pBluescript SK(+) (11 in Figure 4) was used (PDB accession no. 1L6X). Where IgFc (1L6X) have Asp356 and Leu358, fragment D is a human antibody variant and have Gln356 and Met358, respectively (each corresponding to positions 128 and 130 in SEQ ID NO: 1). However, Asp356 and Leu358 of IL6X were left as is, and a mutation of Met428 to Leu was further added for convenience of CNBr treatment of fragment D. As a result, mutations which convert Met358 (corresponding to position 130 in SEQ ID NO: 1) and Met428 (corresponding to position 200 in SEQ ID NO: 1) of fragment D to Leu were introduced. Inverse PCR was used for the introduction of mutation. The amino acid sequence consequently obtained is shown in SEQ ID NO: 2.

Fragment D was also expressed by E coli expression as a fusion protein having Met on its N-terminal side, and further having a purification tag peptide called His tag with 10 consecutive His. The fusion protein is shown below.

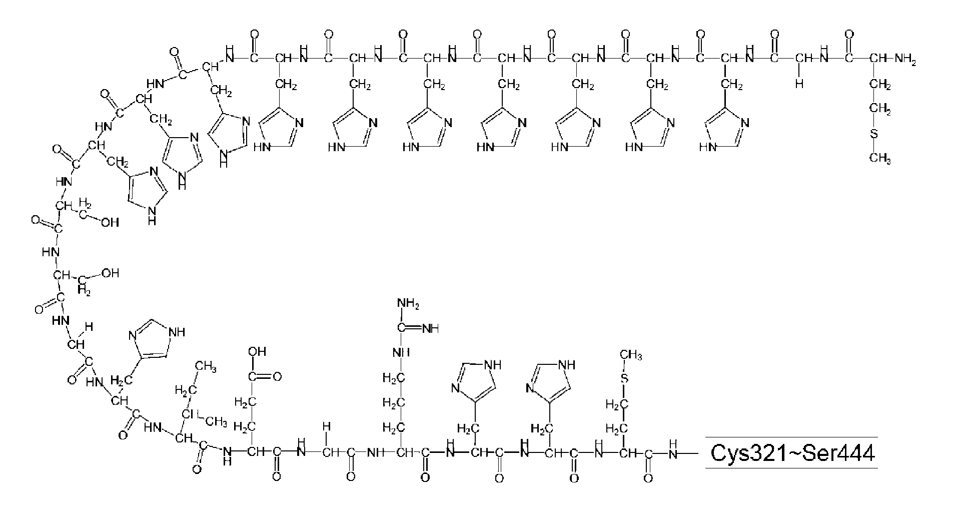

### 1. Introduction of mutation into gene

As described above, two mutations were introduced to render a change from Met to Leu in IgFc.
1-1. Reagents
(1) QuikChange II Site-Directed Mutagenesis Kit (STRATAGENE, Cat# 200523-5)
10 x reaction buffer
Dpn I 10 U/µl
dNTP mix
Pfu Ultra DNA polymerase 2.5 U/µl
XL1-Blue supercompetent cells

(2) 1 M MgCl₂ (per 10 ml)
In a 15 ml FALCON tube, 2.03 g of magnesium chloride hexahydrate (Wako, Cat# 135-00165) was weighed out, dissolved in 10 ml of MilliQ, and filter sterilized.
(3) 1 M MgSO₄ (per 10 ml)
In a 15 ml FALCON tube, 2.46 g of magnesium sulfate heptahydrate (Wako, Cat# 131-00405) was weighed out, dissolved in 10 ml of MilliQ, and filter sterilized.
(4) 20% (w/v) glucose
(5) NZY⁺ broth (per 100 ml)
MilliQ was added to 1.0 g of NZ Amine Type A (Wako, Cat# 541-00241), 0.5 g of yeast extract (DIFCO, Lot# 3346143), and 0.5 g of NaCl (Wako, Cat# 191-01665), filled up to 100 ml, and then adjusted to pH 7.5 with 5 N NaOH (Wako), followed by autoclave.

| | |
|---|---|
| NZ amine Type A | 1.0 g |
| yeast extract | 0.5 g |
| NaCL | 0.5 g |
| MilliQ 1 | Fill up to 100 ml |
| Total | 100 ml |
| ↓ | |
| Adjust to pH = 7.5 | |
| ↓ | |
| Autoclave | |

Filter sterilized 1 M MgCl₂(12.5 µl/ml), 1 M MgSO₄(12.5 µl/ml), and 20% (w/v) glucose (20 µl/ml) were added before use.

**Table 6**

| | |
|---|---|
| NZY broth | 1ml |
| 1 M MgCl₂ | 12.5 µl |
| 1 M MgSO₄ | 12.5 µl |
| 20% glucose | 20 µl |
| Total | 1.05 ml |

(6) Agarose, HT (AMRESCO, Cat#: 9012-36-8)
(7) DNA marker:
- 1kb DNA ladder (BIONEER, Cat# D-1040)
- 100 bp DNA ladder (BIONEER, Cat# D1030)
- 25/100 bp mixed DNA ladder (BIONEER, Cat# D1020)

(8) IPTG (Wako, Cat# 095-02531)
100 mM IPTG stored in a freezer at -30°C
(9) X-gal (Wako, Cat# 023-07851)
12% X-gal stored in a freezer at -30°C
(10) Transformation reagent
See "8. Transformation of IgG Fc (Cys321-Ser444)/pET-16b"
- LB plate
- 2 × YT

(11) Plasmid preparation reagent
See "5. Preparation of pET-16b from E. coli by column method, restriction enzyme treatment"

1-2. Equipments and consumables
   (1) Thermal cycler (GeneAmp PCR System 2700, Applied Biosystems, Part#: 4322620)
   (2) Shaking incubator (THOMAS, AT24S)
   (3) Incubator (SANYO MIR-262)
   (4) Submarine electrophoresis tank Mupid-α (Advance Co., Ltd.)
   (5) 200 µl tube for PCR (ABgene, Cat#: AB-0337)
   (6) 14 ml polypropylene round tube (FALCON, Cat# 352059)
   (7) 100 mm petri dish (FALCON, Cat#: 351001)
   (8) Syringe filter (MILLIPORE, Cat# SLGV 025 LS)

### 1-3. Experiment protocol

### <Primer generation>

(1) The portion to be mutated was specified, and primers complementary to a sequence identical to the template for about 15 bp each on either side of the portion were designed.
(2) Forward primer (SEQ ID NO: 3) and reverse primer (SEQ ID NO: 4) were each prepared to be 100 ng/µl.
(3) Template was prepared to be 5 µg/µl.
(4) PCR was carried out based on the data sheet of QuikChange II Site-Directed Mutagenesis Kit under the condition indicated below.

**Table 7**

| | | |
|---|---|---|
| * PCR Reaction System | | |
| Template | 2 µl | |
| 10 ×Reaction Butter | 5 µl | |
| Forward Primer | 1.25 µl | 125 ng |
| Reverse Primer | 1.25 µl | 125 ng |
| dNTP | 1 µl | |
| Water | 38.5 µl | |
| Total | 50 µl | |

**Table 8**

| * PCR Reaction Condition | | | |
|---|---|---|---|
| Segment | Cycles | Template(°C) | Time |
| 1 | 1 | 95.0 | 30 (sec) |
| | | 5.0 | 30 (sec) |
| 2 | 12-18 | 55.0 | 1 (min) |
| | | | 1 (min/kb of |
| | | 68.0 | plasmid length) |

**Table 9**

| * Number of Cycles | |
|---|---|
| Type of mutation desired | Number of Cycles |
| point mutations | 12 |
| Single amino acid change | 16 |
| Multiple amino acid change | 18 |

(5) After the completion of PCR, 5 µl was electrophorized and bands were confirmed.
(6) 1 µl of Dpn I was added to the PCR product, and reacted at 37°C for 1 hr.
(7) Electrophoresis was performed, and change in band pattern was confirmed.
(8) FALCON 2059 tube was cooled on ice, and 1 µl of PCR product treated with Dpn I was dispensed.
(9) 50 µl of XL-1 Blue was aliquoted on ice, and mixed by tapping.
(10) This was left standing on ice for 30 minutes.
(11) While waiting, 1 M MgCl₂, 1 M Mg SO₄ and 20% (w/v) glucose were added to the NZY broth and heated to 42°C.
(12) This was left standing in a warm bath at 42°C for 45 seconds.
(13) This was left standing on ice for 2 minutes.
(14) 500 µl of the NZY broth warmed in (11) was added.
(15) This was shaken at 37°C for 1 hour.
(16) While waiting, an LB plate was coated with 10 µl of 100 mM IPTG and 100 µl of 12% X-gal, and let dry in an incubator.
(17) The sample that has completed the 1 hr shaking was plated in two plates at 250 µl.
(18) This was static cultured at 37°C for 16 hr.
(19) This was colony-picked to 2.5 ml of 2 × YT.
(20) This was shaking cultured at 37°C for 16 hours.
(21) Mini prep was performed to extract the plasmids (See "5. Preparation of pET-16b from E. coli by column method, restriction enzyme treatment").
Mapping by restriction enzyme was performed (See "7. Insertion of cDNA into pET-16b").
(22) Sequencing was performed.

### 2. Preparation of cDNA fragment by PCR

### 2-1. Reagents

(1) Primer mix: sense and anti-sense primers mixed so that each will be 4 µm
(2) PCR reaction solution
   Ex Taq DNA polymerase (5 U/µl, TAKARA, cat# RR001B)
   10 × Ex Taq buffer (TAKARA, cat# RR001B)
   2.5 mM dNTP mix (TAKARA, cat# RR001B)
(3) 1% agarose gel
(4) TAE
(5) Loading buffer (TAKARA)
(6) EtBr (Nippon Gene, cat# 315-90051)

### 2-2. Equipments

(1) 0.2 ml of Thermo-tube (ABgene, cat# AB-0337)
(2) Thermal cycler
   - Mastercycler gradient (Eppendorf No. 5331 01088)
   - 2720 Thermal cycler (Applied Biosystems, part No. 4359659, serial No. 272S4101291)
(3) Submarine electrophoresis tank (Mupid-2plus ADVANCE)
(4) Transilluminator (ATTA, No. 270022)
(5) ImageMaster VDS: Pharmacia Biotech

### 2-3. Experiment protocol

(1) PCR reaction solution other than DNA polymerase was prepared on ice.
   Template (188 ng/µl) 5.3 µl
   10 × buffer 5 µl
   Primer mix (50 µM) 2 µl
   dNTPs 4 µl
   Water 33.4
   Total 50 µl
(2) PCR reaction solution was dispensed into 0.2 ml tubes for PCR at 50 µl each.
(3) 0.3 µl of DNA polymerase (Ex Tag) was added.
(4) 0.2 ml tubes were set into a thermal cycler, and the program* was started.
   * program: 372 bp
   1. Thermal denaturation at 94°C, 1:00
   2. Thermal denaturation at 94°C, 0:45
   3. Annealing at 55°C, 0:45
   4. Extension reaction at 72°C, 0:30
   5. Final extension reaction at 72°C, 10:00
   2-4 was repeated for 35 cycles.
(5) After completion of the PCR reaction, amplification of the PCR product was confirmed by 1% agarose gel electrophoresis.
   Sample: PCR product 1 µl
   Sterilized MQ 4 µl
   6 × Loading dye 1 µl

### 3. Filter purification of the PCR product

### 3-1. Reagents

(1) Ethanol
(2) Agarose gel
(3) Membrane washing solution
(4) Sterilized MilliQ

### 3-2. Equipments

(1) 1.5 ml tube
(2) SV Minicolumn
(3) Centrifugator
(4) Block incubator
(5) Nano Drop (spectrophotometer)

### 3-3. Experiment protocol

(1) An equal amount of membrane washing solution was added to 150 µl of PCR solution.
(2) The entire amount was applied to the column, left standing for 1 minute, and then centrifuged at 13,000 rpm for 1 minute.
(3) Waste liquid was discarded, 500 µl of membrane wash buffer was added to the column, and centrifuged at 13,000 rpm for 5 minutes.
(4) The column was transferred to a 1.5 ml tube, 50 µl of sterilized MilliQ at 70°C was added, and centrifuged at 13,000 rpm for 1 minute.
(5) OD260 was measured with Nano Drop.

### 4. Restriction enzyme treatment of the PCR product

In order to remove the 3-mers which were added on 5' and 3' sides upon designing for adjustment, treatment by restriction enzymes NdeI and BamHI was carried out. In this way, a DNA having added a 6-mer of the NdeI site on the 5' side and a 6-mer of the BamHI on the 3' side of the 372 bp encoding fragment D can be obtained. After treatment, electrophoresis confirmed that the target product was obtained.

### 4-1. Reagents

(1) Nde I
(2) BamH I
(3) 10 × H
(4) Water

### 4-2. Equipments

(1) Thermostat bath
(2) Submarine electrophoresis tank (Mupid-2plus ADVANCE)
(3) Transilluminator (ATTA, No. 270022)
(4) ImageMaster VDS: Pharmacia Biotech

### 4-3. Experiment protocol

(1) All but the restriction enzyme was prepared on ice.
   DNA (1 µg) 3.74 µl
   10 × H 2.5 µl
   Water 16.76 µl
(2) The restriction enzyme in 1 µl portions on ice.
(3) This was reacted in a thermostat bath at 37°C for 2 hours.
(4) Electrophoresis of 1 µl of reaction mixture was performed.

### 5. Preparation of pET-16b from E. coli by column method, restriction enzyme treatment (14-16 in Figure 4)

The cell wall of E. coli BL21 (DE3) carrying plasmid vector pET-16b was destroyed with sodium dodecylsulfate (SDS), the plasmid vector was extracted, and then restriction enzyme treatment by NdeI and BamHI was performed. The production of target substance was confirmed by electrophoresis after treatment.

### 5-1. Reagents

(1) Wizard® Plus SV Minipreps DNA Purification System; Promega, Cat# A1492X
   - Wizard® Plus SV cell resuspension solution; Part# A711N (pre-mixed with RNase A)
   - Wizard® Plus SV cell lysis solution; Part# A712N
   - Alkaline phosphatase solution; Part# A144B
   - Wizard® Plus SV neutralization solution; Part# A148C
   - Wizard® Plus SV column wash solution; Part# A131C (prepared with solution:100% EtOH = 10:17 for each use)
   - Wizard® SV Mini columns; Part# A129B
   - Collection tubes (2 ml); Part# A130B
   - Nuclease-free water; Part# P119C
(2) Ethanol (99.5); C₂H₅OH (46.07), Wako, Cat# 057-00456

### 5-2. Equipments

(1) High speed micro centrifuge; HITACHI, himac CF.15R, No. 090464
(2) 1.5 ml Eppendorf tube
(3) Nano Drop (spectrophotometer)

### 5-3. Experiment protocol

(1) 1.5 ml of culture solution of E. coli carrying the target plasmid DNA was aliquoted into a microtube.
(2) This was centrifuged at 15,000 rpm at room temperature for 30 seconds.
(3) The supernatant was completely removed.
(4) 250 µl of resuspension buffer was added, and E. coli was suspended first by pipetting, and then by vortexing.
(5) 250 µl of alkaline lysis buffer was added and gently mixed 4-6 times by inversion.
(6) This was left standing on ice for 5 minutes (strict timing) .
(7) 1 minute after the addition of the lysis buffer, 10 µl of alkaline protease solution was added and gently mixed 4 times by inversion.
(8) 5 minutes after the addition of the lysis buffer, 350 µl of neutralization buffer was added and mixed 5 times by inversion.
(9) This was centrifuged at 13,000 rpm at room temperature for 10 minutes centrifugation to obtain the target plasmid DNA in the supernatant.
(10) The necessary amount of column wash solution was prepared during 10 minutes of centrifugation (1 ml/sample).
(11) The column was set on the collection tube.
(12) The supernatant was applied to the column and left standing for 1 minute.
(13) This was centrifuged at 13,000 rpm at room temperature for 1 minute.
(14) The waste liquid of the collection tube was discarded, and 750 µl of column wash solution was added to the column.
(15) This was centrifuged at 13,000 rpm at room temperature for 1 minute.
(16) The waste liquid of the collection tube was discarded, and 250 µl of column wash solution was added to the column.
(17) This was centrifuged at 13,000 rpm at room temperature for 2 minutes.
(18) The column was transferred to a new 1.5 ml microtube.
(19) 100 µl of nuclease-free water was added to the column.
(20) This was left standing for 1 min at room temperature.
(21) This was centrifuged at 13,000 rpm at room temperature for 1 minute, and DNA was eluted.
(22) The concentration and purity of the DNA solution obtained was measured by Nano Drop.
   Sample 1: 54.4 ng/µl, Sample 2: 48.2 ng/µl, Sample 3: 40.6 ng/µl, Sample 4: 29.9 ng/µl
(23) Using Sample 2, agents other than the restriction enzyme were prepared on ice.
   DNA (3 µg) 62.2 µl
   10 × H 8 µl
   Water 3.8 µl
(24) 3 µl each of the restriction enzyme were added on ice.
(25) This was reacted in a thermostat bath at 37°C for 2 hours.
(26) Electrophoresis of 6 µl of reaction mixture was performed.

### 6. Gel purification of the PCR product pET-16b

The DNA insert fragment (13 in Figure 4) and expression vector (16 in Figure 4) were each purified by cutting out from the gel after electrophoresis and recovering. The cut out gel was heated, agarose was removed by centrifugation, then subjected to electrophoresis, and bands for the expression vector and DNA insert fragment were confirmed.

### 6-1. Reagents

(1) Wizard SV gel and PCR Clean-Up System (Promega, cat# A9282)
   - Membrane binding solution (Part# A9303)
   - Wash buffer: Membrane wash solution (Promega, cat# A9282) was diluted 6-fold with EtOH (Wako, cat# 057-00456)
   - Wizard SV mini columns (Promega, part#A129B)
   - Collection tubes (Promega, part#A130B)
(2) 1% agarose gel
(3) EtBr solution (Nippon Gene, cat# 315-90051)
(4) 10 × Loading buffer (TAKARA)

### 6-2. Equipments

(1) Submarine electrophoresis tank (Mupid-2plus, ADVANCE)
(2) Transilluminator (ATTO, No. 270022)
(3) Electronic balance (Development Production Testing, Mettler Toledo, AG64, No. 26210132-6)
(4) Heat block (Dry Thermo Unit, TAITEC, DTU-1B, No. 8032267)
(5) Scalpel (FEATHER, SURGIAL, No. 11 and 14)
(6) Microcentrifugator (HITACHI, himac CF 15R)
(7) Centrifugation evaporator

### 6-3. Experiment protocol

(1) For 9 µl of each of the restriction enzyme-treated cDNA fragment solution and pET-16b solution, 1 µl of 10 × loading buffer was added, applied, and electrophorized.
(2) A 1.5 ml tube was weighed.
(3) After completion of electrophoresis, the gel was placed on Saranwrap (plastic wrap).
(4) The gel was placed on the transilluminator and UV was turned on.
(5) The band for the target PCR product pET-16b was marked with a scalpel.
(6) The gel was moved to a lab bench and there the gel was cut out.
(7) After cutting out the gel, a transilluminator was used to verify that it was accurately cut out.
(8) The gel piece was placed in a 1.5 ml tube and weighed.
(9) Binding buffer in an amount equivalent to the gel piece was added, and heated at 65°C for 15 minutes. Mixing by tapping was performed every 2-3 minutes during this.
(10) The entire amount was applied to the column, and left standing for 1 minute.
(11) After left standing, the tube was centrifuged at 13000 rpm for 1 minute.
(12) Waste liquid was discarded, 700 µl of wash buffer (wash:EtOH = 1:5) was added to the column, and centrifuged at 13000 rpm for 1 minute.
(13) Waste liquid was discarded, 500 µl of wash buffer was added to the column, and centrifuged at 13000 rpm for 5 minutes.
(14) The column was transferred to a 1.5 ml tube, 50 µl of sterilized MilliQ warmed to 70°C was added to this column, and left standing for 5 minutes.
(15) This was centrifuged at 13000 rpm for 1 minute.
(16) This was concentrated with a centrifugation evaporator until the reaction mixture was 15 µl.
(17) 1 µl of concentrated reaction mixture was electrophorized to confirm gel purification.

### 7. Insertion of cDNA into pET-16b (18 in Figure 4)

The DNA insert fragment was inserted into an expression vector.

### 7-1. Reagents

(1) 10 × Loading buffer (TAKARA)
(2) 1 kbp DNA ladder (BioNEER, 130 ng/µl, cat# D-1040)
(3) 100 bp DNA ladder (Bioneer, 135 ng/µl, cat# D-1030)
(4) AGAROSE (AMRESCO, cat# 9012-36-6)
(5) EtBr solution (Nippon Gene, cat# 315-90051)
(6) Phenol/chloroform/isoamyl alcohol (25:24:1) (Nippon Gene, cat# 311-90151)
(7) Chloroform (CH₃Cl (119.38), Wako, cat# 038-02606)
(8) 3 M Sodium acetate pH 5.2 (Nippon Gene, cat# 316-90081)
(9) Ethanol (99.5) (C₂H₅OH (46.05), Wako, cat# 057-00456)
(10) Wizard SV gel and PCR Clean-Up System (Promega, cat# A9282) Ligation high (TOYOBO, cat# LGK-101)
(11) Competent cell (Competent high E. coli DH5α, TOYOBO, cat# DNA-901)
(12) 2 × YTLB plate Bacto tryptone (BD, cat# 211705)
(13) Bacto yeast extract (BD, cat# 212750)
(14) Sodium chloride (NaCl (58.44), Wako, cat# 191-01665)
(15) Bacto Agar (BD, cat# 214010)
(16) Ampicillin (Wako, cat# 010-10371)
(17) rTaq DNA polymerase (5 U/µl, TOYOBO, cat# TAP-201)
(18) 10 × rTaq buffer (TOYOBO, cat# TAP-201)
(19) 2 mM dNTP mix (TOYOBO, cat# TAP-201)
(20) MgCl₂ (TOYOBO, cat# TAP-201)
(21) SV Mini 1000 preps + SV gel & PCR 500 preps (Promega, cat# A1492X)
(22) Glycerol (Wako, cat# 075-00616)
(23) High Purity Plasmid Midiprep System (MARLIGEN, cat# 11451-010)
(24) Isopropyl alcohol (Wako, cat# 166-04836)

### 7-2. Equipments

(1) Warm bath (Iuchi, Model TR-1, No. 1807043)
(2) Program Temp Control System (ASTEC, Model PC-700, No. PN7921205)
(3) Submarine electrophoresis tank (Mupid-2plus ADVANCE)
(4) Transilluminator (ATTO, No. 270022)
(5) Image Master VDS (Pharmacia Biotech)
(6) Thermal cycler
(7) Electronic balance (Development Production Testing, Mettler Toledo, AG64, No. 26210132-6)
(8) Heat block (Dry Thermo Unit, TAITEC, DTU-1B, No. 8032267)
(9) ND-1000 spectrophotometer (Nano Drop Technologies, Inc)
(10) Scalpel (FEATHER, SURGIAL, No. 11 and 14)
(11) Microcentrifugator (HITACHI, himac CF 15R)
(12) Electronic balance (AND, No. 5031522)
(13) Warm bath (TAITEC, No. 91071110)
(14) Petri dish 100 × 15mm style (FALCON, Lot# 3209832)
(15) 14 ml polypropylene round-bottom tube (FALCON, Lot# 4017517)
(16) Thermostatic shaking incubator (THOMAS, No. 007280)
(17) Centrifuge (SAKUMA, No. 30025)
(18) Automatic high speed refrigerated centrifuge; HITACHI, SCR20B, No. 38677
(19) Cell strainer 40 µm; FALCONS Gel and PCR Clean-Up System

### 7-3. Experiment protocol

### 7-3-1. Restriction enzyme reaction (part 1)

(1) The following reaction mixture was prepared on ice in a 1.5 ml tube.

**Table 10**

| | |
|---|---|
| Template | 1µl |
| 10 × buffer | 5µl |
| Nde I | 1µl |
| Sterilized MilliQ | 43µl |
| Total | 50µl |

(2) Restriction enzyme reaction was carried out at 37°C for 1 hour.
(3) After completion of the restriction enzyme reaction, 1 µl was sampled and the band was confirmed by electrophoresis.

### 7-3-2. Phenol/chloroform treatment

(4) 150 µl of TE was added to the restriction enzyme reaction solution.
(5) 200 µl of phenol/chloroform was added, and shaken vigorously for 30 seconds.
(6) This was centrifuged at 15000 rpm for 3 minutes, and the supernatant was aliquoted to a new tube.
(7) 200 µl of phenol/chloroform was added, and shaken vigorously for 30 seconds.
(8) This was centrifuged at 15000 rpm for 3 minutes, and the supernatant was aliquoted to a new tube.
(9) 200 µl of chloroform was added, and shaken vigorously for 30 seconds.
(10) This was centrifuged at 15000 rpm for 3 minutes, and the supernatant was aliquoted to a new tube.
(11) 3 M sodium acetate at 1/10 amount and EtOH at 2.5-fold amount of the supernatant were added, and left standing at - 30°C for 10 minutes.
(12) This was centrifuged at 15000 rpm for 10 minutes centrifugation.
(13) The supernatant was removed, and 1 ml of 70% EtOH was added.
(14) This was centrifuged at 15000 rpm for 10 minutes centrifugation.
(15) The supernatant was removed, and left standing for 3 minutes using a desiccator.
(16) 20 µl of TE was added and dissolved.

### 7-3-3. Restriction enzyme reaction (part 2)

(17) The following reaction mixture was prepared on ice.

**Table 11**

| | |
|---|---|
| Template | 20 µl |
| 10×buffer | 4 µl |
| BamHI I | 1 µl |
| Sterilized MilliQ | 15 µl |
| Total | 40 µl |

(18) This was mixed by tapping, and allowed to react at 37°C for 1 hour.
(19) 4.4 µl of 10 × loading buffer was added, applied to an agarose gel, and electrophorized.

### 7-3-4. Cut-out from gel and purification

(20) Cut-out from the agarose gel was carried out similarly to the above "Gel purification of the PCR product pET-16b." 7-3-5. Ligation reaction
(21) In a 0.5 ml tube, the two solutions were added so that the molar ratio of insert:vector would be 3:1.
(22) Ligation High in an amount equivalent to the two solutions was added.

### 8. Transformation of IgG Fc (Cys321-Ser444)/pET-16b

In order to verify that the DNA insert fragment was correctly inserted into the expression vector, the expression vector was incorporated into E. coli BL21 (DE3) and cultured.

### 8-1. Cell/plasmid

(1) Competent cell (Competent high E. coli DH5α); TOYOBO, code# DNA-903
   - Competent cell
   - SOC medium
(2) Plasmid DNA

### 8-2. Reagents

(1) LB-amp plate
(2) Ethanol (99.5); C₂H₅OH (46.05), Wako, Cat# 057-00456

### 8-3. Equipments

(1) 14 ml polypropylene round-bottom tube; FALCON, Cat# 352059
(2) Warm bath (42°C)
(3) Conradi stick
(4) Thermostatic shaking incubator; THOMAS, No. 007280

### 8-4. Experiment protocol

(1) The warm bath was started to warm up to 42°C.
(2) Competent cells and SOC medium stored in a deep freezer were thawed on ice.
(3) 0.1 pg - 10 ng of plasmid DNA was dispensed in 14 ml polypropylene round tubes cooled on ice.
(4) 10-50 µl of competent cells were added to 14 ml tubes.
(5) This was left standing on ice for 20 minutes.
(6) After soaking in a warm bath at 42°C for 45 seconds, this was placed back on ice and cooled for 2 minutes.
(7) 90-450 µl of SOC medium was added, and shaken at 37°C at 140 rpm for 1 hour.
(8) LB-amp plate was warmed in an incubator at 37°C during shaking, and dried on bench for about 10 minutes.
(9) E. coli culture solution was applied onto the selection media plate at 2-3 concentrations.
(10) Plates were inverted and cultured overnight in an incubator at 37°C.
(11) The number of colonies was counted on the next day, several single colonies were picked, and cultured with shaking in 1.5 ml of 2 × YT media.

### 9. Alkali Miniprep

In order to subject to the following restriction enzyme mapping, the cultured E. coli was treated with SDS and the expression vector was extracted.

### 9-1. Reagents

(1) RNase
(2) Solution (I, II, III)
(3) Phenol/chloroform/isoamyl alcohol (25:24:1)
(4) 3 M NaOAc
(5) 100% ethanol
(6) 70% ethanol
(7) Sterilized MilliQ

### 9-2. Equipments

(1) 1.5 ml Eppendorf tube
(2) Centrifugator
(3) Desiccator
(4) Eppendorf shaker

### 9-3. Experiment protocol

(1) Culture solution incubated overnight was transferred to a 1.5 ml Eppendorf tube.
(2) This was centrifuged at 15,000rpm for 30 seconds at 4°C.
(3) The supernatant (sup) was removed.
(4) 4 µl of RNase was added to solution I (Sol I:RNase = 1.7:3.4).
(5) 100 µl of 4) was added to the precipitate (pellet) of 3) on ice.
(6) 200 µl of solution II (5 × NaOH:20% SDS:MilliQ = 0.136:0.17:3.094) was added, gently mixed by inversion, and then left standing on ice for 5 minutes.
(7) 150 µl of solution III (5 M KoAc:AcOH:MilliQ = 60:11.5:28.5) was added, gently mixed by inversion, and then left standing on ice for 5 minutes.
(8) This was centrifuged at 15,000rpm for 10 minutes at 4°C. During this time, an Eppendorf was prepared, and 450 µl of phenol/chloroform/isoamyl alcohol (25:24:1) per Eppendorf tube was collected.
(9) Supernatant was placed in Eppendorf prepared in (8), and shaken for 30 seconds.
(10) This was centrifuged at 15,000rpm for 3 minutes at 4°C.
(11) The supernatant was taken in new Eppendorfs, and 40 µl each of 3 M NaOAc were added.
(12) 1 ml each of 100% ethanol was added, gently mixed by inversion, and then left standing at -30°C for 10 minutes.
(13) This was centrifuged at 15,000rpm for 10 minutes at 4°C.
(14) 700 µl each of 70% ethanol was added and vortexed.
(15) This was centrifuged at 15,000rpm for 10 minutes at 4°C.
(16) The supernatant was removed and dried for 3 minutes in a desiccator.
(17) 100 µl of sterilized MilliQ was added, and subjected to 3 minutes on an Eppendorf shaker.

### 10. Enzyme treatment of IgG Fc (Cys321-Ser444)/pET-16b

Two kinds of restriction enzymes were used on expression vector extracted from E. coli to perform restriction enzyme mapping. Electrophoresis was carried out after restriction enzyme treatment, and the DNA insert fragment was confirmed to be correctly inserted into the expression vector.

### 10-1. Reagents

(1) Nde I
(2) BamH I
(3) EcoR V
(4) 10 × H
(5) Water

### 10-2. Equipments

Similar to the above "Restriction enzyme treatment of the PCR product."

### 10-3. Experiment protocol

(1) Agents other than the restriction enzyme were prepared on ice.
   2 µl of DNA
   1 µl of 10 × H
   6.5 µl of water (6.75 µl in case of EcoR V)
(2) 0.25 µl each of restriction enzyme was added on ice.
(3) This was reacted in a thermostat bath at 37°C for 45 minutes.
(4) Electrophoresis of 1 µl of reaction mixture was performed.

### 11. Transformation of IgG Fc (Cys321-Ser444)/pET-16b for BL21 (DE3)

The expression vector was incorporated into E. coli BL21 (DE3), and a fusion protein of His tag and fragment D was expressed.

### 11-1. Generation of competent cells

### Experiment protocol

(1) 3 ml each of LB media was taken into the fraction.
(2) The fraction of E. coli cultured overnight was vortexed.
(3) 30 µl each of the cultured E. coli was subcultured into the fraction with media, and incubated at 37°C at 160 min⁻¹ (overnight) .
(4) 5 ml each of LB media was taken into the fraction.
(5) The fraction of E. coli cultured overnight was vortexed.
(6) 50 µl each of the cultured E. coli was subcultured into the fraction with media, and incubated at 37°C at 160min⁻¹ until OD₅₅₀ was 0.4.
   The 15 ml centrifugation tubes were cooled during this time.
(7) OD measurement after 1 hour 20 minutes was OD₅₅₀ = 0.349.
(8) The culture solution was transferred to a 15 ml centrifugation tube cooled on ice.
(9) This was centrifuged at 2,000 rpm (0°C) for 20 minutes.
(10) The supernatant was discarded, 3 ml of 0.1 M cold MgCl₂ was added and washed.
(11) This was centrifuged at 2,000 rpm (0°C) for 20 minutes.
(12) The supernatant was discarded, 3 ml 0.1 M cold CaCl₂ was added and washed.
(13) This was stored on ice for 20 minutes.
(14) This was centrifuged at 2,000 rpm (0°C) for 20 minutes.
(15) The supernatant was discarded, 0.5 ml of 0.1 M cold CaCl₂ was added and vortexed.
   2057 tubes were cooled.

### 11-2. Transformation

(1) The competent cells were transferred to 2057 tubes.
(2) 1 µl of plasmid DNA solution was gently transferred to a tube containing the competent cells, and stored on ice for 1 hour.
   (1) This was soaked in a thermostat bath at 42°C for 3 minutes.
   (2) The sample was quickly transferred onto ice, and left for 1 minute.
   (3) 5 ml of LB media was added to the sample, and shaken at 37°C for 45 minutes.
   (4) This was centrifuged at 3,000 rpm (rt) for 10 minutes.
   (5) The supernatant was removed, and 1 ml of LB media was added to the pellet and vortexed.
   (6) Each sample was divided into 100 µl (*1) and 900 µl.
   (7) The 900 µl sample was centrifuged at 7,000 rpm (rt) for
   4 minutes.
   (8) The supernatant was removed by decantation, and 100 µl of LB media was added to the pellet and vortexed (*2).
   (9) 100 µl each of (*1) and (*2) were plated.
   (10) This was cultured overnight at 37°C.

### 12. Protein induction and purification

Isopropyl-β-D-thiogalactopyranoside (IPTG) was added to the E. coli BL21 (DE3) media to induce expression. After cultivation, E. coli was collected, the cell wall was homogenized with ultrasound, and then the E. coli homogenate was run in a Ni-NTA resin column to adsorb the His tag-fragment D fusion protein. Next, washing buffer containing imidazole was run to elute the fusion protein. The eluted fusion protein was confirmed to be the target substance by HPLC analysis and mass spectrometry.
Subsequently, the eluate was lyophilized to yield a white solid. This solid was subjected to CNBr treatment to remove the His tag from fragment D. CNBr treatment was carried out in the presence of 40% acetonitrile and 2% TFA.

### 12-1. Experiment protocol (500 ml culture)

(1) 5 ml each of LB-amp media was taken into the fraction.
(2) 1 colony was picked up from plates with grown bacteria.
(3) The picked up bacteria was suspended well in LB-amp.
(4) This was incubated at 37°C at 160min⁻¹ (overnight).
(5) The culture from 4) was added to 500 ml of 2 × YT-amp, and incubated at 37°C 160min⁻¹ until OD₆₀₀ reached 0.6.
(6) OD₆₀₀ after 4 hours = 0.963.
(7) The media was brought back to room temperature, 5 ml of 100 mM IPTG was added (final concentration 1 mM), and incubated at 16°C.
(8) After 24 hours, induction was terminated, the culture solution was transferred to a 50 ml centrifugation tube, and centrifuged at 3,500 rpm (rt) for 10 minutes.
(9) The supernatant was removed, PBS(-) was added and suspended, and aliquoted into 50 ml centrifugation tubes.
(10) This was centrifuged at 3,500 rpm (rt) for 10 minutes.
(11) The supernatant was removed, and stored at -20°C.
(12) After thawing the sample in a thermostat bath at 37°C, 50 ml of lysis buffer (100 mM NaH₂PO₄, 10 mM This_{˙}HCl, 8 M urea, 5 mM imidazole, pH 8.0) was added, and subjected to ultrasound.
(13) After homogenation by ultrasound, the sample was put on a shaker. During this time, 1.5 ml of Ni-NTA resin was equilibrated with lysis buffer.
(14) The sample was loaded onto the Ni-NTA resin column, and adsorbed 10 times.
(15) The resin was washed well with wash buffer (100 mM NaH₂PO₄, 10 mM Tris_{˙}HCl, 8 M urea, 50 mM imidazole, pH 8.0).
   The extent of wash was measured by CBBG test (800 µl of CBB was added to 5 µl of wash solution and OD₅₉₅ was measured) . (16) This was eluted with elution buffer (100 mM NaH₂PO₄, 10 mM Tris_{˙}HCl, 8 M urea, 400 mM imidazole, pH 8.0). The CBBG test was performed as above to determine the fraction to be recovered.

### 12-2-1. Confirmation electrophoresis

(17) To 10 µl of eluate in the fraction, 300 µl of acetone:MeOH = 1:1 solution was added (left standing on ice for 0.5h) .
(18) This was centrifuged at 13,000 rpm (4°C) for 10 minutes. (19) The supernatant was removed, and dried with a desiccator.
(20) 25 µl each of 1 × sample buffer was added, and boiled at 95°C for 5 minutes.
(21) SDS-PAGE was performed.

### 12-2-2. Confirmation by HPLC and mass spectrometry

(22) 100 µl of 10 mM DTT was added to 100 µl of eluate, and soaked in a thermostat bath at 37°C for 30 minutes.
   Cadenza CD-18 (3 µm, 4.6 × 75 mm), developing solvent A: 0.1% TFA aqueous solution; B: 0.1% TFA CH₃CN: H₂O = 90:10, gradient A:B = 95:5 → 25:75, 15 min., flow rate: 1.0 ml/min ESI-MS: m/z calcd for C₇₂₄H₁₁₁₀N₂₁₂O₂₁₆S₅: [M + 23H]²³⁺ 714.2, [M + 22H] ²²⁺ 746.6, [M + 21H]²¹⁺ 782.1, [M + 20H]²⁰⁺ 821.2, [M + 19H]¹⁹⁺ 864.4, [M + 18H]¹⁸⁺ 912.3, [M + 17H]¹⁷⁺ 965.9, [M + 16H]¹⁶⁺ 1026.3, [M + 15H]¹⁵⁺ 1094.6, [M + 14H]¹⁴⁺ 1172.7, [M + 13H]¹³⁺ 1262.9, found 714.4, 746.8, 782.3, 821.4, 864.5, 912.6, 966.2, 1026.4, 1094.7, 1172.8, 1263.0

### 12-2-3. Dialysis before CNBr treatment

(23) The eluate was transferred to a dialysis tube (Spectra/Por® Membrane MW: 3,500, Flat Width: 54mm, Diameter: 34mm, Vol/Length: 9.3 ml/cm, Length: 15m/50 ft), and using 5 L of distilled water as the external solution, stirred at 4°C (overnight) .
(24) The sample in the dialysis tube was recovered and lyophilized.
   Yield: 40 mg/L culture

### 12-2-4. CNBr treatment

(25) 10 mg of white solid obtained in 24) was dissolved in 5 ml of 40% CH₃CN aqueous solution and 2% TFA aqueous solution, 0.1 mg of CNBr was added under Ar flow/in shade and left standing.
(26) This was concentrated under reduced pressure, 100 µ of buffer (100 mM NaCl, 50 mM Tris_{˙}HCl, 6 M urea, 1 mM DTT, pH 8.0) was added, and soaked in a thermostat bath at 37°C for 1 hour.
(27) After desalting with HPLC, the target fragment D was purified with gel filtration chromatography.
   Gel filtration on Superdex 75™ 10/300 GL. The column (10 × 300mm) was equilibrated and operated as follows: 10% (v/v) formic acid, flow rate of 0.4 mL min⁻¹.
   ESI-MS: m/z calcd for C₆₁₉H₉₆₆N₁₆₆O₁₉₁S₃: [M + 18H]¹⁸⁺ 772.4, [M + 17H]¹⁷⁺ 817.8, [M + 16H]¹⁶⁺ 868.9, [M + 15H]¹⁵⁺ 926.7, [M + 14H]¹⁴⁺ 992.8, [M + 13H]¹³⁺ 1069.1, [M + 12H]¹²⁺ 1158.1, [M + 11H]¹¹⁺ 1263.3, [M + 10H]¹⁰⁺ 1389.6, [M + 9H]⁹⁺ 1543.9, [M + 8H]⁸⁺ 1736.7, [M + 7H]⁷⁺ 1984.7, found 772.7, 821.3, 869.0, 927.0, 993.1, 1069.4, 1158.4, 1263.7, 1389.8, 1544.2, 1737.2, 1985.0

### 13. Agarose gel electrophoresis

The reaction mixture was concentrated under reduced pressure, desalted, and purified by gel filtration chromatography to yield the target fragment D. Analysis by ODS column, mass spectrometry and SDS-PAGE confirmed that the product was fragment D.

### 13-1. Reagents

(1) 50 × TAE (Tris-acetate-EDTA)
Tris base (Wako), acetic acid (Wako Cat#: 017-00256), and 0.5 M EDTA (pH = 8.0) were weighed out as below, and filled up to 1 L with MilliQ.

**Table 12**

| | | (Final Concentration) |
|---|---|---|
| Tris base | 242 g | 2M |
| Acetic acid | 57. 1ml | 1M |
| 0.5M EDTA | 100ml | 50mM |
| MilliQ | | Fill up to 1 L |
| Total | 1L | |

(2) 1 × TAE
50 × TAE (2 M Tris base, 1 M CH₃COOH, 50 mM EDTA) was diluted 50-fold with MilliQ.
(3) 0.5 g/ml of EtBr (ethidium bromide)
10 g/ml of EtBr (Wako, Code#315-90051; stored at -4°C) was diluted 20-fold with 1 × TAE.
(4) Agarose, HT (AMRESCO, Cat# 9012-36-8)
(5) 1kb DNA ladder (BIONEER, Cat# d-1040)
(6) 100 bp DNA ladder (BIONEER, Cat# D1030)

### 13-2. Equipments and consumables

(1) Submarine electrophoresis tank Mupid-α (Advance Co., Ltd.)
(2) Gel Maker Stand (Advance Co., Ltd.)
(3) Gel tray (Advance Co., Ltd.)
(4) Comb (Advance Co., Ltd.)
(5) Gel photograph device (Pharmacia)

### 13-3. Experiment protocol

### 13-3-1. Preparation of agarose gel (preparation of one sheet in a Gel Maker Stand is described.)

(1) 1.2 g of agarose was weighed out into a conical flask, and 117.6 ml of MilliQ was added.
(2) Saranwrap (plastic wrap) was wrapped around the opening of the conical flask, and warmed in a microwave to dissolve the agarose.
(3) This was left at room temperature and waited until it cooled to about 60°C.
(4) While letting the gel cool, Gel Maker Stand and gel tray were prepared.
(5) When the gel was about 60°C, 2.4 ml of 50 × TAE and 6 µl of 10 mg/ml EtBr were added and mixed.
(6) The gel was poured into a Gel Maker Stand prepared beforehand.
(7) Bubbles at the gel surface were removed with Blue Tip etc. before the gel solidified.
(8) When the removal of bubbles was complete, the comb was inserted.
(9) This was left for about 30 minutes at room temperature to let the gel solidify.

### 13-3-2. Preparation of electrophoresis buffer (preparation for 350 ml is described.)

(1) 7 ml of 50 × TAE was weighed out into a 500 ml graduated cylinder.
(2) This was filled up to 350 ml with MilliQ.
(3) The opening of the graduated cylinder was covered with Parafilm, and mixed by inversion.
(4) This was poured into the electrophoresis tank.
(5) 17.5 µl of 10 mg/ml EtBr was added.
(6) The electrophoresis buffer was homogenously stirred with e.g. Blue Tip or tweezers.

### 13-3-3. Electrophoresis method

(1) The electrophoresis buffer was mixed with tweezers or Blue Tip to homogenously stir the solution.
(2) The sample to be electrophorized and the loading dye were mixed well by pipetting.
(3) The sample was applied to the well.
(4) The DNA ladder (marker) suited for the objective was applied.
(5) The power was turned on, the voltage was selected, and electrophoresis was started.
(6) The power was turned off at the end of electrophoresis, the gel tray was picked up with tweezers, and placed on Saranwrap.
(7) The gel was taken out of the gel tray onto the Saranwrap.
(8) A photograph was taken with a gel photograph device.

### 14. Other reagents

(1) LB; 10 g of Bacto-tryptone, 5 g of yeast extract, 10 g of NaCl, 15 g/1 L H₂O of agar
(2) Petri dish containing LB; 10 g of Bacto tryptone, 5 g of yeast extract, 5 g of NaCl, 5 g/1 L H₂O of glucose were mixed, 15 g/L of 1.5% agar was added, autoclaved for 20 minutes, let cooled to 50-60°C, 100 mg of ampicillin was added, and plated at 30 ml each on petri dish.
(3) LB-amp; LB:10% ampicillin = 1000:1.
(4) Alkaline SDS solution; 0.2 N NaOH and 1% SDS
(5) 4% polyacrylamide gel; 1.995 ml of 30% acrylic solution, 1.5 ml of 10 × TBE ^{(Note 1)}, 75 µl of 10% APS ^{(Note 2)}, and 15 ml of 10 µl/H₂O TEMED
   Note 1 10 × TBE; 108 g of trisma base, 55 g of broic acid, 80 ml/1 L H₂O of 0.25 M EDTA
   Note 2 10% APS; 0.1 g/H₂O 1 ml of ammonium persulfate
(6) 2 × YT; Bacto-tryptone 16 g, Bacto yeast extract 10 g, NaCl 5 g, ampicillin 100 mg, aH₂O 1 L
(7) 2 × YT-amp; 2 × YT: 10% ampicillin = 1000:1
(8) 1 × SB; 1 ml of 0.5 M Tris-HCl (pH 6.8), 2 ml of 10% SDS, 0.6 ml of β-mercaptoethanol, 1 ml of glycerol, 5.4 ml of H₂O, and a few drops of 1% BTB added until the solution turns dark blue.
(9) 12.5% acrylamide gel;
   * Upper gel; 5 ml of H₂O, 3.75 ml of lower gel buffer, 6.25 ml of 30% acrylamide, 75 µl of 10% APS, and 10 µl of TEMED
   * Lower gel; 3 ml of H₂O, 1.25 ml of upper gel buffer, 0.75 ml of 30% acrylamide, 17.5 µl of 10% APS, and 5 µl of TEMED
(10) 1 × SDS-PAGE buffer; 1.51 g of Tris(hydroxymethyl) aminomethane, 7.2 g of glycine, 500 ml of 500 mg/H₂O sodium lauryl sulfate (SDS)
(11) Stripping solution; MeOH 25%, AcOH 10%, H₂O 65%

### <Kinetically controlled ligation between fragments A and B>

KCL is a method where the thioester at the C-terminus of fragment A is prepared as a thioester having higher leaving ability than the thioester of fragment B, and the difference in reaction rates of the two fragments is utilized for linking by NCL. By using this method, it will be possible to link the peptide chain while keeping the thioester at the C-terminus of fragment B.

Fragment A peptide thioester (2 mg, 0.56 µmol) was transferred to an Eppendorf tube, 6 M GnHCl, 0.2 M phosphate buffer, and 40 mM TCEP adjusted to pH 6.8 were added and dissolved. Fragment B peptide thioester (2 mg, 0.53 µmol) dissolved in the buffer solution (300 µl) beforehand was added to the Eppendorf tube containing fragment A peptide thioester, and reacted for 2 hours. Purification by HPLC yielded fragment A + B. Moreover, tracing of reaction was performed with HPLC.
HPLC: Cadenza CD-18 (3 µm, 4.6 × 75 mm); developing solvent A: 0.1% TFA aqueous solution; B: 0.1% TFA acetonitrile:water = 90:10; gradient A:B = 95:5 → 25:75, 15 min.; flow rate: 1.0 ml/min
HPLC purification: Vydac C-18 (5 µm, 4.6 × 250 mm); gradient A:B = 70:30 → 40:60, 20 min.; flow rate: 1.0 ml/min
ESI-MS: m/z calcd for C₃₂₇H₅₁₀N₈₄O₉₀S₄: [M + 4H]⁴⁺ 1797.6, [M + 5H]⁵⁺ 1438.3, [M + 6H]⁶⁺ 1198.7, [M + 7H]⁷⁺ 1027.6, [M + 8H]⁸⁺ 899.2, [M + 9H]⁹⁺ 799.5, found 1797.6, 1438.3, 1198.7, 1027.7, 899.5, 799.6

### <Native chemical ligation of glycopeptide fragments C and D>

First, in order to examine the rough concentration of fragment D in the ligation buffer, a calibration curve was created employing lysozyme (MW: 14,000) as the model protein. The absorbance at 280 nm for lysozyme concentrations of 10 µl, 5 µl, and 2.5 µl were measured. Each concentration was measured 3 times, and the average of the measurements was set to be the absorbance at that concentration. The result is shown in the table below and in Figure 5.

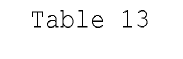

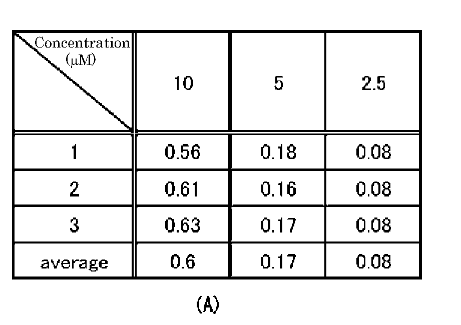

Moreover, the solution for dissolving the lysozyme is the ligation buffer (8 M GnHCl, 0.2 M phosphate buffer, 40 mM TCEP, and 25 mM MPAA adjusted to pH 7.5) used for NCL of fragments C and D, but without MPAA.
Next, since the solvent employed in the gel filtration of "13. Agarose gel electrophoresis" was 10% formic acid aqueous solution, the solvent was concentrated with Ultracel Amicon® YM-10, and at the same time the 10% formic acid aqueous solution was filtered. Subsequently, the buffer was exchanged to 8 M GnHCl, 0.2 M phosphate buffer, and 40 mM TCEP adjusted to pH 7.5. Absorbance at 280nm was measured when the total amount was about 500 µl. The resulting measurement was 0.14. From this result and the calibration curve in Figure 5, the concentration of fragment D in this concentrated solution was determined to be about 1 mM.

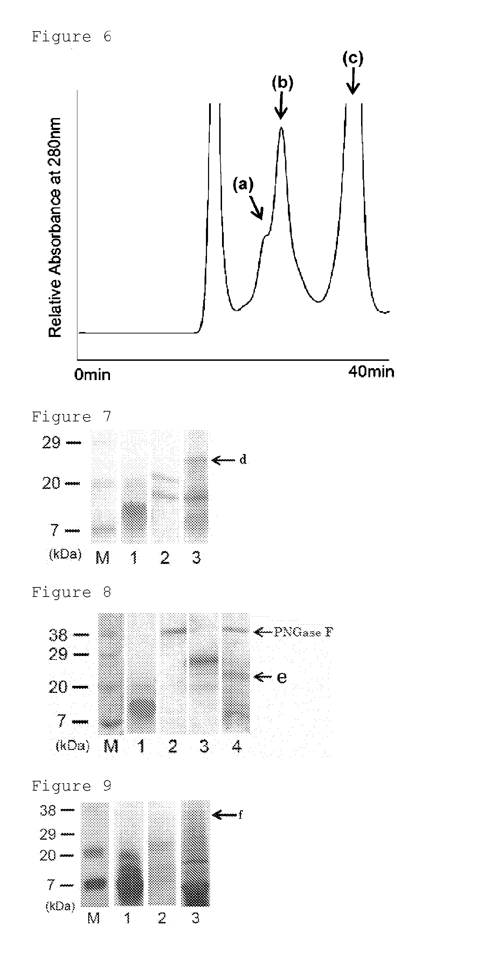

Concentration by centrifugation was continued to increase the concentration of fragment D, and recovered when the solution volume was about 125 µl. Fifteen µl of this solution containing dissolved fragment D was added to a siliconized tube containing fragment C dissolved in ligation buffer (8 M GnHCl, 0.2 M phosphate buffer, 40 mM TCEP, and 25 mM MPAA adjusted to pH 7.5), and reacted at room temperature. The final concentrations of fragments C and D in the reaction solution were 2 mM and 1 mM for fragments C and D, respectively. After 15 hours, DTT was added to the reaction solution so that the final concentration would be 50 mM, and reduced at 37°C for 30 minutes. This was then subjected to gel filtration chromatography. For gel filtration chromatography, Superdex 75™ 10/300 GL was used, and 10% (v/v) formic acid at a flow rate of 0.4 mL/min was run to equilibrate the column for operation. The result is shown in Figure 6. Each peak in Figure 6 was aliquoted, and subjected to mass spectrometry.

As a result, it was confirmed that (b) is the peak of raw material fragment D and (c) is the peak of raw material fragment C. Moreover, the peak (c) had a mass number that is thought to be compound P or Q below in which the side chain of intramolecular Lys of fragment C attacked the thioester, detaching the thioester and allowing cyclization.

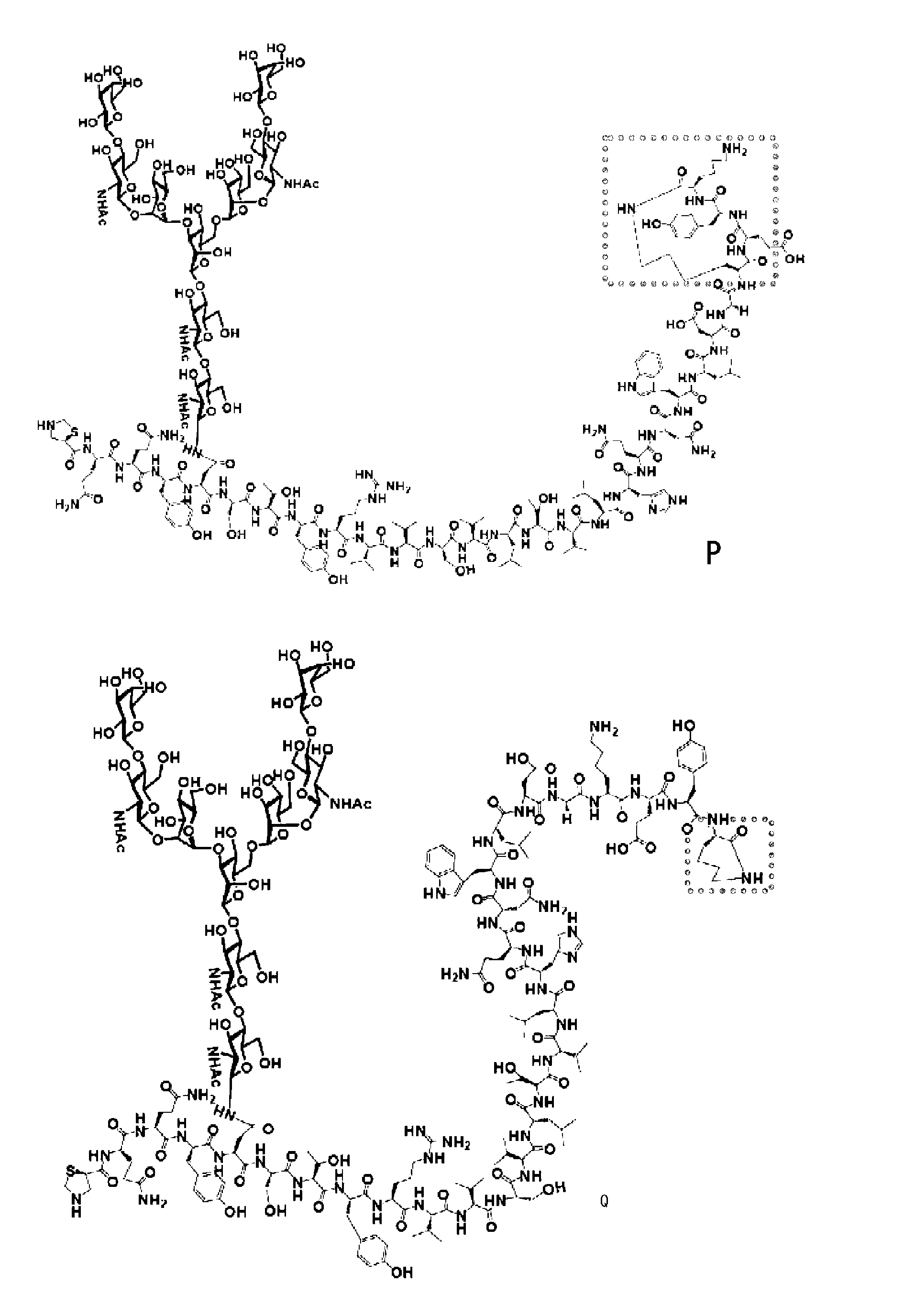

In peak (a), a product having a mass number different from the raw material fragment D was confirmed at a slight intensity, but it could not be confirmed as the target fragment C + D in mass spectrometry.
The result of SDS-PAGE analysis of the progress of this reaction is shown in Figure 7. Lane 1 shows fragment C, lane 2 shows His tag + fragment D (16 kDa), and lane 3 shows reaction solution. M is the molecular weight marker. The band with the highest molecular weight in lane 3 (product (d)) was thought to be band of the target. The reason the peak could not be confirmed in mass spectrometry was thought to be that because the peptide chain became a mixture forming a diverse 3-dimensional structure, the concentration of each of these compounds in solution is low, and thereby ion intensity in ESI mass became low.

If fragments C and D were accurately linked by NCL, the mass number of the product should be about 19 kDa, however, peak by electrophoresis was about 25 kDa. This is thought to be due to the phenomenon in which a sugar chain is added to the peptide chain, but in order to more certainly confirm the production of the target product, enzyme treatment by PNGase F was performed. When an N-type sugar chain is bound to the target, by treating with PNGase F, the binding between sugar chain and peptide is specifically cleaved at the Asn position where the sugar chain is bound. Accordingly, when this is verified with electrophoresis, it is expected that a band of a peptide having a molecular weight in which the weight amounting to the sugar chain is smaller can be confirmed.

Specifically, the fraction comprising gel filtration chromatography peak (a) was concentrated by centrifugation, and at the same time the solution was exchanged to 0.5 M phosphate buffer adjusted to pH 7.5. During this time, 10 × glycoprotein denaturing buffer (2.5 mg of SDS, 30.8 mg/500 µl of DTT) and 10 × G7 reaction buffer (0.5 M phosphate buffer adjusted to pH 7.5) were prepared. Next, 3 µl of 10 × glycoprotein denaturing buffer was added to 27 µl of solution comprising concentrated peak (a), and heated at 110°C. After heating for 25 minutes, 3.3 µl of 10 × G7 reaction buffer and 3.3 µl of 10% NP-40 and 5 µl of PNGase F were added, and heated at 37°C for 2 hours. Similarly, a reaction without adding PNGase was performed as a control experiment. Confirmation was by SDS-PAGE.
The result is shown in Figure 8. Lane 1 shows the ligation product, lane 2 shows PNGaseF, lane 3 shows fragment C, lane 4 shows the reaction solution, and M shows the molecular weight marker. A new product (e) with a smaller molecular weight position than product (d) was confirmed in lane 4. Since the position of bands changed by a treatment using an enzyme that causes specific sugar chain hydrolysis, it was suggested that product (d) and an N-type sugar chain attached. From the above results, it was determined that fragments C and D can be linked by NCL, and that product (d) was the target fragment C + D.

### <Native chemical ligation of fragment A + B and fragment C + D aiming for synthesis of entire IgG1-Fc fragment>

The peak shown in Figure 6 (a) was aliquoted, thiazoline at the N-terminal of product (d) was converted to cystein. Since the eluting solution for purifying product (d) using gel filtration column was 10% formic acid solution, first, using Ultracel Amicon® YM-10, 10% formic acid solution in the fraction was exchanged to 6 M GnHCl and 0.2 M phosphate buffer adjusted to pH 7.5 with centrifugation filtration. Next, 0.2 M methoxyamine hydrochloride was added to this system until pH was 4.0. After stirring at room temperature for 5.5 hours, 6 M GnHCl, 0.2 M phosphate buffer, and 0.5 M NaOH were carefully added until the reaction solution was pH 7.0, and the reaction solution was concentrated while centrifugation filtration using Ultracel Amicon® YM-10 as above. When the reaction solution volume was about 20 µl, 20 µl of 6 M GnHCl, 0.2 M phosphate buffer, 80 mM TCEP, and 0.1 M MPAA adjusted to pH 7.0 was prepared, fragment A + B was dissolved in this, and added to fragment C + D solution treated with methoxyamine. NCL was started when the two peptides which are the raw materials dissolved in the solution. After 15 hours from starting the reaction, the progress of this ligation reaction was examined by SDS-PAGE.

The result is shown in Figure 9. In addition to the bands shown in lane 1 (fragment A + B) and lane 2 (fragment C + D), product (f) was confirmed, and it was determined to the target IgG1-Fc fragment. When fragment A + B and fragment C + D are linked by NCL, the molecular weight is about 25 kDa. The band for product f was seen around 35 kDa, and this is thought to be a phenomenon caused by sugar chain addition.

### Sequence Listing Free Text

SEQ ID NO1 shows the amino acid sequence of human IgG1-Fc fragment.
SEQ ID NO2 shows the amino acid sequence of human IgG1-Fc fragment produced by the production process according to the present invention.
SEQ ID NO3 shows the base sequence of the sense primer for IgFc cloning.
SEQ ID NO4 shows the base sequence of the antisense primer for IgFc cloning.

## Claims

1. An IgG-Fc fragment having a sugar chain added thereto,
wherein the sugar chain is added at the same position as that in a naturally occurring IgG-Fc fragment,
wherein among the amino acids at positions 1-30 from the sugar chain-added amino acid on the N-terminal side of the sugar chain-added amino acid,
(i) any one is substituted by Cys;
(ii) any one amino acid that is not Ser in the naturally occurring form is substituted by Ser;
(iii) any one amino acid that is not Thr in the naturally occurring form is substituted by Thr; or
(iv) any one amino acid that is not Ala in the naturally occurring form is substituted by Ala, and
wherein at least one Met is substituted by an amino acid other than Met.

2. The IgG-Fc fragment according to claim 1,
wherein said IgG-Fc fragment is an IgG1-Fc fragment,
wherein a sugar chain is added to Asn at position 69 of the amino acid sequence shown in SEQ ID NO: 1, and
wherein among the amino acids at positions 59-68,
(i) any one is substituted by Cys;
(ii) any one is substituted by Ser;
(iii) any one amino acid that is not Thr in the naturally occurring form is substituted by Thr; or
(iv) any one amino acid that is not Ala in the naturally occurring form is substituted by Ala.

3. The IgG-Fc fragment according to claim 2, wherein Glu at position 65 of the amino acid sequence shown in SEQ ID NO: 1 is substituted by Cys.

4. The IgG-Fc fragment according to claims 2 or 3, wherein Met at positions 130 and 200 of the amino acid sequence shown in SEQ ID NO: 1 are substituted by Leu.

5. The IgG-Fc fragment according to any one of claims 1 to 4, wherein said sugar chain is a sugar chain represented by: 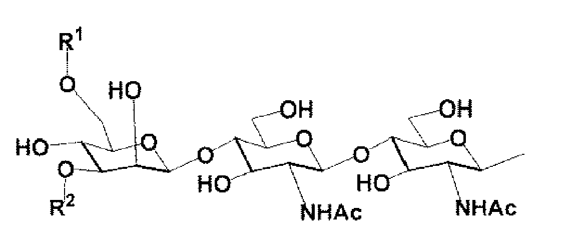 [wherein R¹ and R², which are identical or different, are 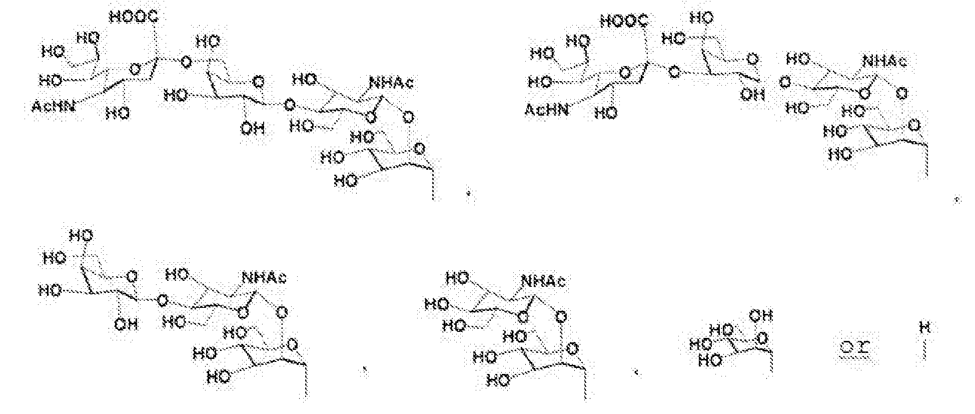 , and Ac represents an acetyl group].

6. A process for producing an IgG-Fc fragment having a substantially homogeneous sugar chain added thereto, comprising:
a step of synthesizing by solid-phase synthesis using Asn having a substantially homogenous sugar chain added thereto, a partial peptide of said IgG-Fc fragment which is a peptide having a sugar chain added thereto comprising a sugar chain-added amino acid having 3 to 50 amino acid residues;
a step of expressing at least one of a partial peptide on the N-terminal side of said peptide having a sugar chain added thereto within said IgG-Fc fragment and a partial peptide on the C-terminal side of said peptide having a sugar chain added thereto within said IgG-Fc fragment by an expression system, and synthesizing the remainder by solid-phase synthesis; and
a step of linking said peptide having a sugar chain added thereto, said N-terminal partial peptide, and said C-terminal partial peptide by ligation;
wherein the step of expressing said partial peptide by an expression system comprises:
a step of expressing said partial peptide as a fusion protein with a purification tag via Met, and purifying the fusion protein by utilizing the purification tag; and
a step of cleaving said partial peptide and said purification tag by degrading Met in the presence of a strong acid and a water-miscible solvent.

7. The process according to claim 6, wherein said strong acid is selected from the group consisting of trifluoroacetic acid, hydrogen fluoride and methanesulfonic acid.

8. The process according to claims 6 or 7, wherein the N-terminal amino acid of said peptide having a sugar chain added thereto is substituted with Cys or a threonine derivative shown in the following formula (1) for synthesis.

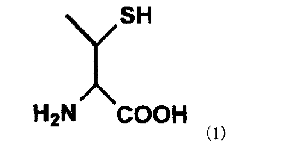

9. The process according to any one of claims 6 to 8, wherein, in the step of expressing said partial peptide by an expression system, said partial peptide is expressed in such a way that the Met contained in the partial peptide is substituted with an amino acid other than Met.

10. The process according to any one of claims 6 to 9,
wherein said IgG-Fc fragment is an IgG1-Fc fragment, and
wherein when said N-terminal partial peptide is synthesized by solid-phase synthesis, the step of synthesizing the N-terminal partial peptide comprises:
a step of synthesizing each peptide of said N-terminal partial peptide divided between Thr at position 32 and Cys at position 33 of the amino acid sequence shown in SEQ ID NO: 1 by solid-phase synthesis; and
a step of linking the synthesized peptides by ligation.

11. The process according to claim 10, wherein when said C-terminal partial peptide is synthesized by solid-phase synthesis, the step of synthesizing the C-terminal partial peptide comprises:
a step of synthesizing each peptide of said C-terminal partial peptide divided between Thr at position 138 and Cys at position 139 and/or between Ser at position 196 and Cys at position 197 of the amino acid sequence shown in SEQ ID NO: 1 by solid-phase synthesis; and
a step of linking the synthesized peptides by ligation.

12. The process according to any one of claims 10 or 11,
wherein said peptide having a sugar chain added thereto is positions 65-92 of the amino acid sequence shown in SEQ ID NO: 1, said N-terminal partial peptide is positions 1-64, and said C-terminal partial peptide is positions 93-216,
wherein said peptide having a sugar chain added thereto is synthesized with solid-phase synthesis by substituting Glu at position 65 with Cys, wherein said N-terminal partial peptide is divided into positions 1-32 and 33-64 and each synthesized with solid-phase synthesis, and
wherein said C-terminal partial peptide is expressed by an expression system in such a way that Met at positions 130 and 200 are substituted with Leu.

13. The process according to any one of claims 6 to 12, further comprising a step of folding the IgG-Fc fragment after the linking step by said ligation.

14. The process according to any one of claims 6 to 13, wherein said expression system is an E. coli expression system.

15. A chimeric antibody comprising an IgG-Fc fragment according to any one of claims 1 to 5, or an IgG-Fc fragment produced by the process for producing the IgG-Fc fragment according to any one of claims 6 to 14.

16. A method for designing a process for producing an IgG-Fc fragment having a homogenous sugar chain added thereto,
wherein said IgG-Fc fragment is produced by dividing it into a peptide having a sugar chain added thereto which comprises a sugar chain-added amino acid and one or more other peptides,
wherein said peptide having a sugar chain added thereto has any amino acid at positions 1-30 from the sugar chain-added amino acid on the N-terminal side of said sugar chain-added amino acid as the N-terminus, and has an amino acid that flanks on the N-terminal side of the closest Cys, Ser, Thr or Ala of the C-terminal side of the sugar chain-added amino acid as the C-terminus,
wherein the peptide having a sugar chain added thereto is synthesized by solid-phase synthesis employing an amino acid having a homogenous sugar chain added thereto,
wherein the other peptides are either expressed by an expression system or synthesized by solid-phase synthesis, and
wherein said peptide having a sugar chain added thereto and said peptides are linked by ligation.

17. The process according to claim 16, wherein in the solid-phase synthesis of said peptide having a sugar chain added thereto, the N-terminal amino acid is substituted with Cys or a Thr derivative shown in the following formula (1) for synthesis:

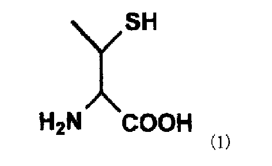

18. The process according to claims 16 or 17, wherein the other peptides are divided into two or more partial peptides at the N-terminal side of at least one amino acid selected from the group consisting of Cys, Ser, Thr and Ala contained in the peptide, and each of the partial peptides are either expressed by an expression system or synthesized by solid-phase synthesis, and
wherein said peptide having a sugar chain added thereto and said partial peptides are linked by ligation.

19. The process according to any one of claims 16 to 18,
wherein when the other peptides are expressed by an expression system, the peptide is expressed as a fusion protein with a purification tag via Met, purified by binding the purification tag to the particular substance,
wherein said peptide and said purification tag is cleaved by degrading Met in the presence of a strong acid and a water-miscible solvent, and
wherein Met contained in said peptide is altered to an amino acid other than Met for expression.


SEQUENCE LISTING

<110>  Otsuka Chemical Co., Ltd.

<120>  IgG-Fc Fragment and manufacturing method thereof

<130>  OCKP0901F

<150>  JP 2009-029180
<151>  2009-02-10

<160>  4

<170>  PatentIn version 3.1

<210>  1
<211>  216
<212>  PRT
<213>  Homo sapiens

<220>
<223>  amino acid sequence of Human IgG-Fc fragment

<220>
<221>  CARBOHYD
<222>  (69)..(69)
<223>  N-linked


<400>  1

Cys Pro Ala Pro Glu Leu Leu Gly Gly Pro Ser Val Phe Leu Phe Pro
1               5                   10                  15


Pro Lys Pro Lys Asp Thr Leu Met Ile Ser Arg Thr Pro Glu Val Thr
            20                  25                  30


Cys Val Val Val Asp Val Ser His Glu Asp Pro Gln Val Lys Phe Asn
        35                  40                  45


Trp Tyr Val Asp Gly Val Gln Val His Asn Ala Lys Thr Lys Pro Arg
    50                  55                  60


Glu Gln Gln Tyr Asn Ser Thr Tyr Arg Val Val Ser Val Leu Thr Val
65                  70                  75                  80


Leu His Gln Asn Trp Leu Asp Gly Lys Glu Tyr Lys Cys Lys Val Ser
                85                  90                  95


Asn Lys Ala Leu Pro Ala Pro Ile Glu Lys Thr Ile Ser Lys Ala Lys
            100                 105                 110


Gly Gln Pro Arg Glu Pro Gln Val Tyr Thr Leu Pro Pro Ser Arg Gln
        115                 120                 125


Glu Met Thr Lys Asn Gln Val Ser Leu Thr Cys Leu Val Lys Gly Phe
    130                 135                 140

Tyr Pro Ser Asp Ile Ala Val Glu Trp Glu Ser Asn Gly Gln Pro Glu
145                 150                 155                 160

Asn Asn Tyr Lys Thr Thr Pro Pro Val Leu Asp Ser Asp Gly Ser Phe
                165                 170                 175

Phe Leu Tyr Ser Lys Leu Thr Val Asp Lys Ser Arg Trp Gln Gln Gly
            180                 185                 190

Asn Val Phe Ser Cys Ser Val Met His Glu Ala Leu His Asn His Tyr
        195                 200                 205

Thr Gln Lys Ser Leu Ser Leu Ser
    210                 215

<210>  2
<211>  216
<212>  PRT
<213>  Artificial

<220>
<223>  Amino acid sequence of Human IgG1-Fc fragment made according
       to the manufacturing method of the present invention.

<220>
<221>  CARBOHYD
<222>  (69)..(69)
<223>  N-linked

<220>
<221>  MISC_FEATURE
<222>  (65)..(65)
<223>  amino acid substitution Glu -> Cys

<220>
<221>  MOD_RES
<222>  (1)..(1)
<223>  Cys having blocking group Thz

<220>
<221>  MUTAGEN
<222>  (200)..(200)
<223>  mutagen Met -> Leu

<220>
<221>  MISC_FEATURE
<222>  (128)..(128)
<223>  Asp derived from IL6X

<220>
<221>  MISC_FEATURE
<222>  (130)..(130)
<223>  Leu derived from IL6X

<400>  2

Cys Pro Ala Pro Glu Leu Leu Gly Gly Pro Ser Val Phe Leu Phe Pro
1               5                   10                  15

Pro Lys Pro Lys Asp Thr Leu Met Ile Ser Arg Thr Pro Glu Val Thr
            20                  25                  30

Cys Val Val Val Asp Val Ser His Glu Asp Pro Gln Val Lys Phe Asn
        35                  40                  45

Trp Tyr Val Asp Gly Val Gln Val His Asn Ala Lys Thr Lys Pro Arg
    50                  55                  60

Cys Gln Gln Tyr Asn Ser Thr Tyr Arg Val Val Ser Val Leu Thr Val
65                  70                  75                  80

Leu His Gln Asn Trp Leu Asp Gly Lys Glu Tyr Lys Cys Lys Val Ser
                85                  90                  95

Asn Lys Ala Leu Pro Ala Pro Ile Glu Lys Thr Ile Ser Lys Ala Lys
            100                 105                 110

Gly Gln Pro Arg Glu Pro Gln Val Tyr Thr Leu Pro Pro Ser Arg Asp
        115                 120                 125

Glu Leu Thr Lys Asn Gln Val Ser Leu Thr Cys Leu Val Lys Gly Phe
    130                 135                 140

Tyr Pro Ser Asp Ile Ala Val Glu Trp Glu Ser Asn Gly Gln Pro Glu
145                 150                 155                 160

Asn Asn Tyr Lys Thr Thr Pro Pro Val Leu Asp Ser Asp Gly Ser Phe
                165                 170                 175

Phe Leu Tyr Ser Lys Leu Thr Val Asp Lys Ser Arg Trp Gln Gln Gly
            180                 185                 190

Asn Val Phe Ser Cys Ser Val Leu His Glu Ala Leu His Asn His Tyr
        195                 200                 205

Thr Gln Lys Ser Leu Ser Leu Ser
    210                 215


<210>  3
<211>  33
<212>  DNA
<213>  Artificial

<220>
<223>  sense primer designed for IgFc cloning

<400>  3
tttcatatgt gcaaggtctc caacaaagcc ctc                                    33


<210>  4
<211>  32
<212>  DNA
<213>  Artificial

<220>
<223>  anti sense primer designed for IgFc cloning

<400>  4
aaaggatcct caagacaggg agaggctctt ct                                     32